# EUROPEAN PATENT APPLICATION

(11) **EP 4 825 841 A2**
(43) Date of publication of application: **21.10.2026**
(21) Application number: 26190187.0
(22) Date of filing: 10.11.2022
(51) Int. Cl.: C07D 213/65

(54) **NOVEL MODULATORS OF THE ARYL HYDROCARBON RECEPTOR AND METHODS OF USE THEREOF**

(30) Priority: 10.11.2021 WO PCT/CN2021/129722; 23.11.2021 US 202163282491 P
(62) Document number(s) of the earlier application(s) in accordance with Art. 76 EPC: 22826261.4 / 4 430 028
(71) Applicant: Allianthera (Suzhou) Biopharmaceutical Co., Ltd., Suzhou, Jiangsu 215000 (CN); Allianthera Boston Inc., Natick, MA 01760 (US)
(72) Inventor: CHEN, Jinshan, Jiangsu 215000 (CN); CHOPRA, Rajiv, Natick, 01760 (US); GU, Jiamin, Jiangsu 215000 (CN)
(74) Representative: Zacco Sweden AB

(57) **Abstract**

Disclosed herein are compounds which can act modulators of the aryl hydrocarbon receptor (AHR). Further disclosed herein are methods for treating diseases and disorders, such as inflammatory and autoimmune diseases and disorders, using the compounds disclosed herein.

## Description

### BACKGROUND

This disclosure relates to compounds that act as aryl hydrocarbon receptor (AHR) activity modulators, pharmaceutical formulations thereof, and methods of using the compounds to treat diseases and disorders, such as inflammatory and autoimmune diseases and disorders.

The aryl hydrocarbon receptor (AHR) is a ligand-dependent transcription factor that regulates gene expression in a variety of cells, such as epithelial and immune cells. Activation of the AHR can lead to changes of the inflammation cytokine profile and antioxidant response in the cellular environment, and can facilitate the healing process of a diseased tissue. AHR signaling can play a key role in maintaining skin and ocular homeostasis by regulating the skin immune network, keratinocyte differentiation, skin barrier function and pigmentation, retinal epithelial cell, rod photoreceptors, choroidal endothelial cells, choroidal neovascular formation, and response to oxidative stress. Cells found in the skin and eye that express AHR, includes keratinocytes, sebocytes, fibroblasts, melanocytes, endothelial cells, Langerhans cells, choroidal endothelial cells, retinal pigment epithelial cells, rod photoreceptors and immune cells present in the skin and eye. Thus, modulation of the AHR signal transduction pathway is implicated in the pathology of various diseases and disorders of the skin and eye.

AHR signaling also is known to regulate the composition and function of different cell types in the gastrointestinal tract, and therefore, has a prominent role in maintaining the balance between health and disease. AHR is involved in several physiological processes, including regulation of homeostasis and immunity at epithelial barriers such as the one formed by intestinal epithelial cells (IECs). Potent immune responses in the body occur in the gut and, as such, there is considerable interest in elucidating the molecular mechanism(s) underlying the role of AHR in cells in the intestinal mucosa - including IECs and various immune cells, such as B cells, T cell receptor γδ T cells (TCRγδ), T helper 17 cells (Th17), regulatory T cells (Treg), type 1 regulatory T cells (Tr1), innate lymphoid cells (ILC), macrophages (MQ), intraepithelial lymphocytes (IEL), dendritic cells (DC), and neutrophils. Accordingly, aberrant AHR activity has been implicated in several intestinal pathologies, such as intestinal inflammation, infection and cancer.

AHR signaling also is known to play a primary role in bone remodeling by altering the interplay between bone-forming osteoblasts and bone-resorbing osteoclasts. The overall effect of AHR activation in osteoblasts is suppressed cell differentiation, and AHR agonism has dose-dependent effects on osteoblasts in which hyperactivation and hypoactivation, respectively, inhibit and promote bone formation. The effect of AHR modulation in osteoclasts is less well-understood, but the AHR pathway has been implicated in both stimulation and impairment of osteoclast differentiation. Accordingly, the AHR pathway is an attractive target for the treatment of various human diseases in which osteoblasts and osteoclasts are implicated in pathogenesis, including bone destructive diseases such as osteoporosis and cancer.

### SUMMARY

One aspect of the disclosure provides a compound having a structure of Formula (I): 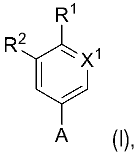 or a pharmaceutically acceptable salt thereof, wherein: R¹ is C₁₋₆alkyl, C₁₋₆haloalkyl, C₁₋₆hydroxyalkyl, OC₁₋₆alkyl, C(=O)C₁₋₆alkyl, or C(=CH)C₁₋₆alkyl; R² is OH, OC₁₋₆alkyl, N(R^{N1})₂, or halo; each R^{N1} independently is H or C₁₋₆alkyl; X¹ is N, C-OH, or C-OC₁₋₆alkyl; A is an optionally substituted fused bicyclic heteroaryl group having 8-10 total ring atoms and 1, 2, or 3 heteroatoms selected from N, O, or S; or 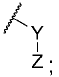 Y is 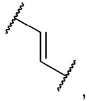 O, S, S=O, or SO₂; and Z is an optionally substituted phenyl group or an optionally substituted heteroaryl group having 5 or 6 total ring atoms and 1, 2, or 3 heteroatoms selected from N, O, and S; with the proviso that when R² is OH or OC₁₋₆alkyl and X¹ is C-OH or C-OC₁₋₆alkyl; then Y is not

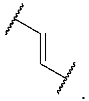

In some cases, R¹ is C₁₋₃alkyl. In some cases, R¹ is 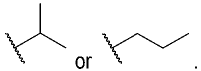 In some cases, R¹ is 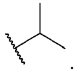 In some cases, R¹ is 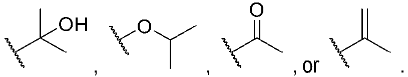 In some cases, R² is OH, OCH₃, N(R^{N1})₂, or F; and each R^{N1} independently is H or CH₃. In some cases, R² is OH. In some cases, R² is OCH₃. In some cases, R² is NH₂. In some cases, R² is F. In some cases, X¹ is N. In some case, X¹ is C-OH. In some cases, X¹ is C-OCH₃. In some cases, R² is OH, OCH₃, NH₂, or F and X¹ is N, C-OH, or C-OCH₃. In some cases, R² is OH and X¹ is N. In some cases, R² is OH and X¹ is C-OH. In some cases, R² is NH₂ and X¹ is C-OH. In some cases, R² is NH₂ and X¹ is N. In some cases, R² is F and X¹ is C-OH. In some cases, R² is OH and X₁ is C-OCH₃. In some cases, R¹ is 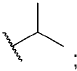 R² is OH, OCH₃, or NH₂; and X¹ is N, C-OH, or C-OCH₃.

In some cases, A is an optionally substituted fused bicyclic heteroaryl group having 8-10 total ring atoms and 1, 2, or 3 heteroatoms selected from N, O, or S. In some cases, A is benzooxazolyl, benzoimidazolyl, indolyl, isoindolyl, indolizinyl, purinyl, benzofuranyl, benzimidazole, quinolinyl, isoquinolinyl, naphthiridinyl, or pyridopyrazinyl, wherein any of the foregoing is unsubstituted or substituted. In some cases, A is benzooxazolyl, benzoimidazolyl, quinolinyl, isoquinolinyl, or naphthiridinyl, wherein any of the foregoing is unsubstituted or substituted. In some cases, A is 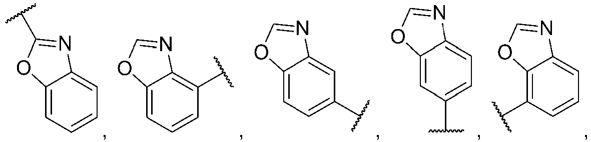 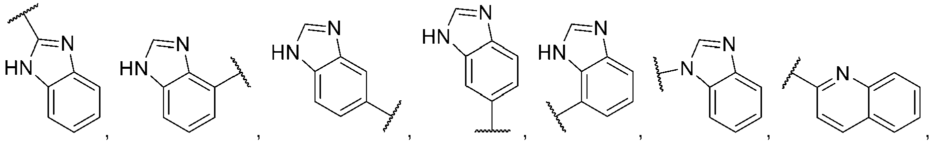 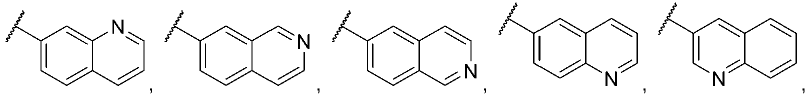 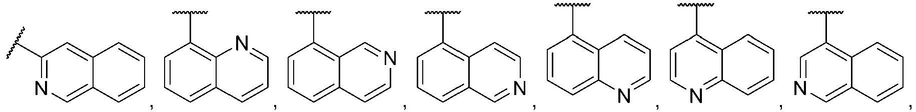 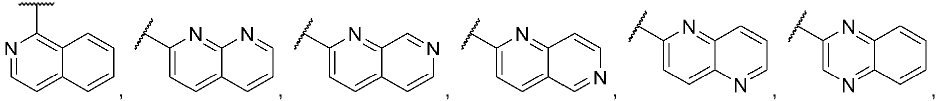 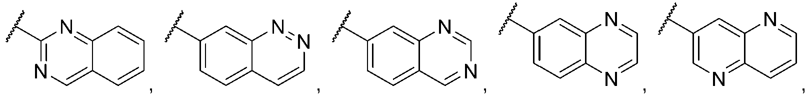 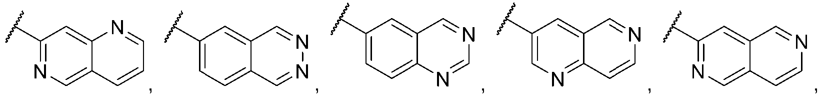 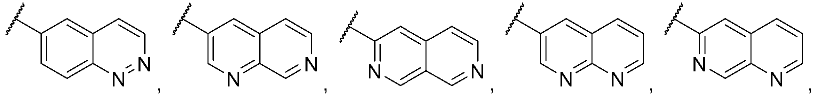 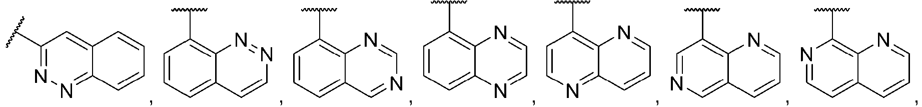 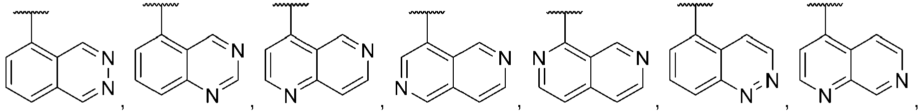 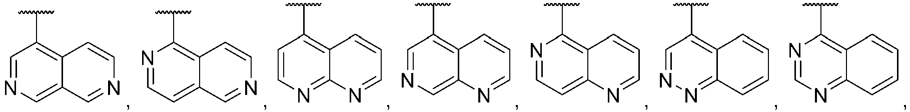 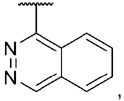 wherein any of the foregoing is unsubstituted or substituted.

In some cases, A is unsubstituted. In some cases, A is 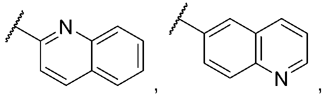 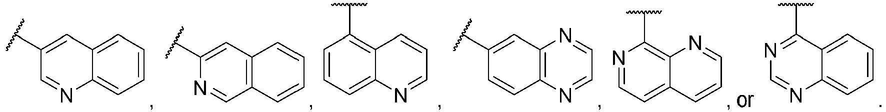 In some cases, A is 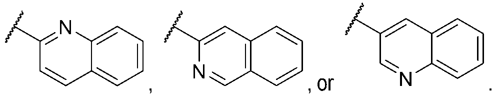 In some cases, A is 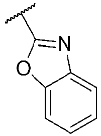 or

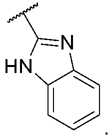

In some cases, A is substituted. In some cases, A is substituted with 1, 2, or 3 substituents, wherein each substituent independently is halo, OH, CN, COOH, COOC₁₋₆alkyl, C₁₋₆alkyl, C₁₋₆haloalkyl, OC₁₋₆alkyl, OC₁₋₆haloalkyl, C=OC₁₋₃alkyl, or N(R^{N1})₂; wherein each R^{N1} independently is H or C₁₋₆alkyl. In some cases, A is substituted with 1, 2, or 3 substituents, wherein each substituent independently is F, Cl, OH, CN, COOH, CH₃, OCH₃, CF₃, OCF₃, NH₂, NHCH₃, or NH(CH₃)₂. In some cases, A is 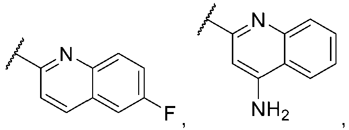 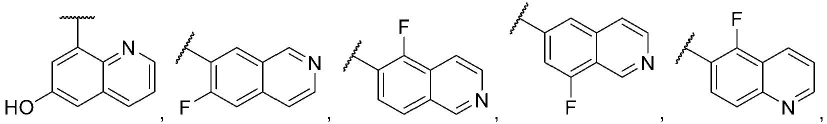 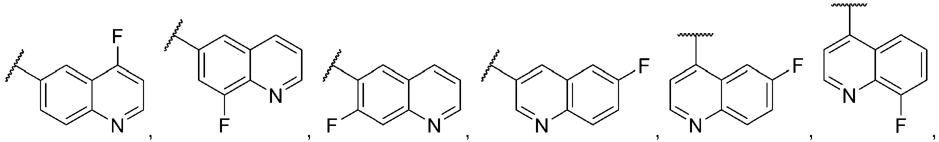 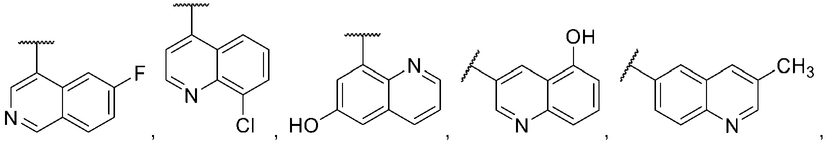 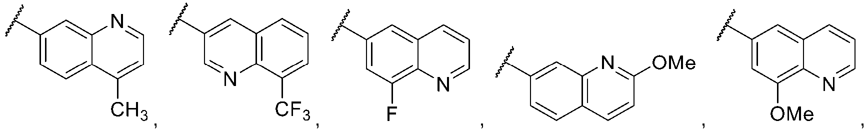 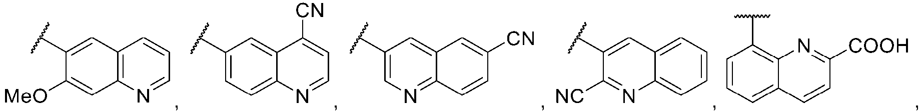 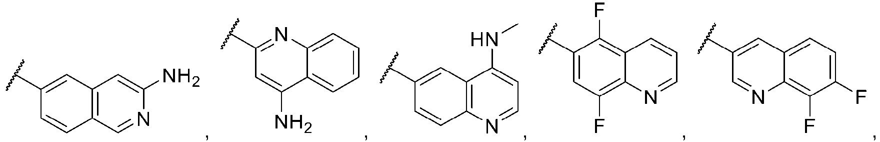 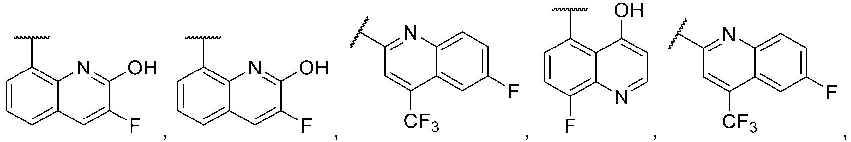 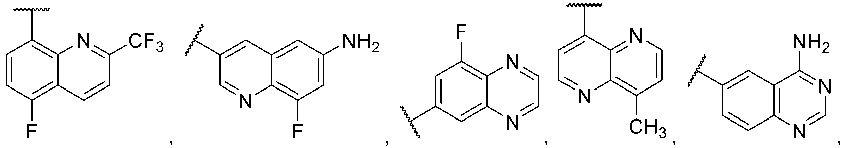 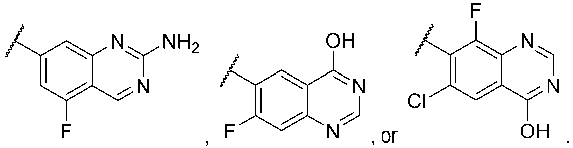 In some cases, A is 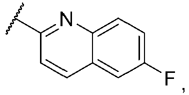 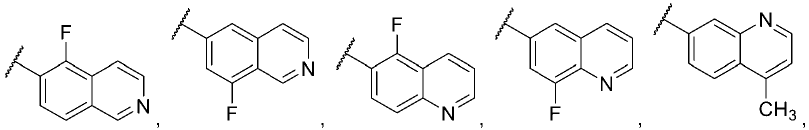 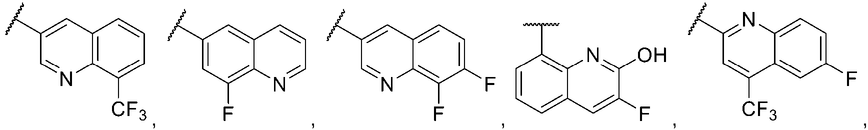 or

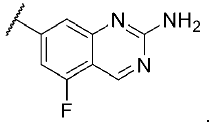

In some cases, A is 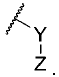 In some cases, Y is 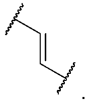 In some cases, Y is O, S, S=O, or SO₂. In some cases, Z is an unsubstituted phenyl group or an unsubstituted pyridyl. In some cases, A is 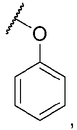 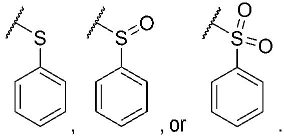 In some cases, A is 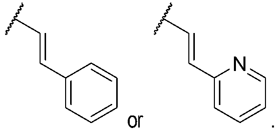 In some cases, Z is a substituted phenyl group or a substituted pyridyl group. In some cases, the phenyl group or heteroaryl group is substituted with 1, 2, or 3 substituents, wherein each substituent is selected from halo, OH, CN, COOH, COOC₁₋₆alkyl, C₁₋₆alkyl, C₁₋₆haloalkyl, OC₁₋₆alkyl, OC₁₋₆haloalkyl, C=OC₁₋₆alkyl, and N(R^{N1})₂; wherein each R^{N1} independently is H or C₁₋₆alkyl. In some cases, the phenyl group or pyridyl group is substituted with 1, 2, or 3 substituents, wherein each substituent independently is F, Cl, OH, CN, COOH, CH₃, OCH₃, CF₃, OCF₃, NH₂, NHCH₃, or NH(CH₃)₂. In some cases, A is

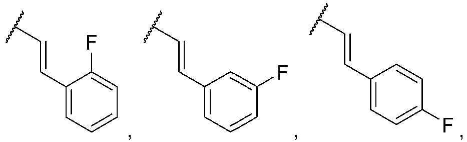

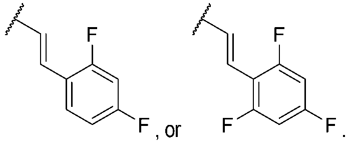

In some cases, R¹ is 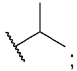 R² is OH, OCH₃, or NH₂; X¹ is N, C-OH, or C-OCH₃, and A is unsubstituted quinolinyl, unsubstituted isoquinolinyl, unsubstituted benzooxazolyl, or

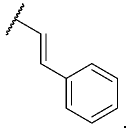

Another aspect of the disclosure provides a compound having a structure listed in Table A, Table B, Table C, or Table D, or a pharmaceutically acceptable salt thereof. In some cases, the compounds is selected from 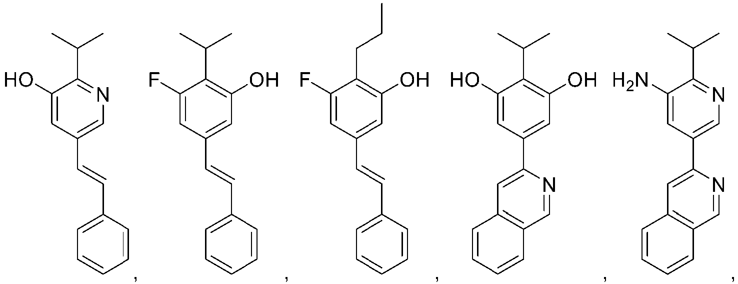 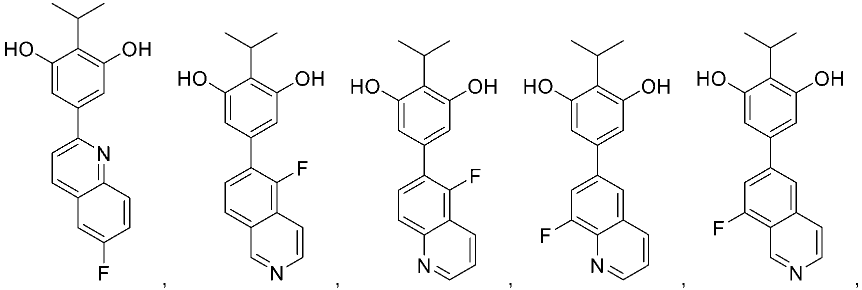 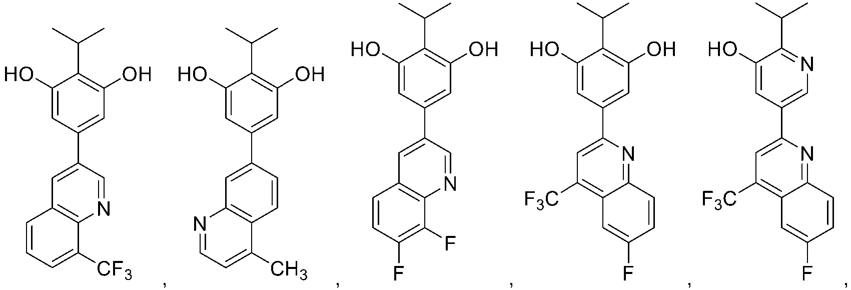 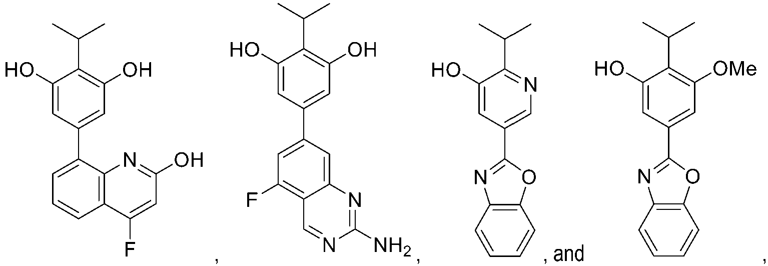 or a pharmaceutically acceptable salt thereof.

In some cases, the compound or salt of the disclosure comprises one or more deuterium atoms, one or more tritium atoms, one or more C¹¹ atoms, one or more C¹³ atoms, one or more C¹⁴ atoms, or any combination of the foregoing.

Another aspect of the disclosure provides a pharmaceutical formulation comprising the compound or salt of the disclosure and a pharmaceutically acceptable excipient.

Yet another aspect of the disclosure provides a method of modulating the aryl hydrocarbon receptor (AHR) activity in a subject, comprising contacting the AHR with the compound or salt of the disclosure, or a formulation of the disclosure, in an amount effective to modulate the AHR activity.

Still another aspect of the disclosure provides a method of treating or preventing a disease or disorder in a subject, comprising administering to the subject a therapeutically effective amount of the compound or salt of the disclosure or a formulation of the disclosure. In some cases, the disease or disorder is an inflammatory disease or disorder. In some cases, the disease or disorder is a disease or disorder of the gastrointestinal tract, skin, lung, central nervous system, pancreas, eye, bones, or bone joints, neuroinflammatory diseases and disorders, or neurodegenerative diseases and disorders. In some cases, the disease or disorder of the gastrointestinal tract is selected from the group consisting of colitis, inflammatory bowel disease, Crohn's disease, celiac disease, necrotizing enterocolitis, irritable bowel syndrome, chronic idiopathic constipation, traveler's diarrhea, and colorectal cancer. In some cases, the disease or disorder of the skin is selected from the group consisting of atopic dermatitis, acne, psoriasis, and vitiligo. In some cases, the disease or disorder of the eye is age-related macular degeneration. In some cases, the disease or disorder of the lung is lung fibrosis or chronic obstructive pulmonary disease. In some cases, the disease or disorder of the bone or bone joints is osteoporosis, rheumatoid arthritis, or bone cancer. In some cases, the disease or disorder is a disease or disorder of the central nervous system. In some cases, the disease or disorder of the central nervous system is Alzheimer's Disease and other memory disorders, Amyotrophic Lateral Sclerosis (ALS, Lou Gehrig's disease, or motor neuron disease). In some cases, the disease or disorder is a disease or disorder of the pancreas. In some cases, the disease or disorder of the pancreas is type 1 diabetes. In some cases, the disease or disorder is an autoimmune disease or disorder. In some cases, the autoimmune disease or disorder is Hidradenitis Suppurativa, Systemic Sclerosis, Lupus Nephritis, Ankylosing Spondylitis, Non-Radiographic Axial Spondyloarthritis (NR-Ax-SpA), Rheumatoid Arthritis, Juvenile Idiopathic arthritis (JIA), Crohn's Disease, Ulcerative Colitis, Systemic Lupus Erythematosus, Atopic Dermatitis, Chronic Obstructive Pulmonary Disease, Severe Asthma, Psoriasis, Sjogren's Disease, Chronic Spontaneous Urticaria, Cutaneous Lupus Erythematosus, Still's Disease, Idiopathic Pulmonary Fibrosis, Dermatomyositis, Sarcoidosis, Psoriatic Arthritis, Pustular Psoriasis, Osteoarthritis, Alopecia Areata, Giant Cell Arteritis (GCA), ANCA Vasculitis, Vasculitis, Eosinophilic Esophagitis, Acute Respiratory Distress Syndrome, Severe Acne or Lichen Planus. In some cases, the disease or disorder is a neuroinflammatory disease or disorder. In some cases, the neuroinflammatory disease or disorder is Multiple Sclerosis, Rasmussen's encephalitis, acute disseminated encephalomyelitis (ADEM), Autoimmune encephalitis, transverse myelitis (TM), or anti-NMDA receptor encephalitis. In some cases, the disease or disorder is a neurodegenerative disease or disorder. In some cases, the neurodegenerative disease or disorder is Uveitis, optic neuritis, neuromyelitis optica spectrum disorder (NMOSD), or Age-related Macular Degeneration (AMD).

In some cases, the disease or disorder is diabetes, cancer, a viral infection, or a bacterial infection. In some cases, the viral infection is flavivirus infection or coronavirus infection. In some cases, the disease or disorder is a bacterial infection. In some cases, the bacterial infection is a pulmonary infection, gastrointestinal infection, a skin infection, an ear infection or a septicemia.

In some cases, the methods of the disclosure further comprising the administration of a therapeutic agent. In some cases, the therapeutic agent is an anti-inflammatory agent. In some cases, the anti-inflammatory agent is selected from mesalazine, naproxen, ibuprofen, diclofenac, celecoxib, sulindac, oxaprozin, piroxicam, indomethacin, meloxicam, fenoprofen, difunisal, etodolac, ketorolac tromethamine, meclofenamate, nabumetone, salsalate, or any combination of the foregoing.

The disclosure further provides a compound or salt of the disclosure, or a formulation of the disclosure for use in the treatment or prevention of a disease or disorder in a subject. The disclosure also provides a compound or salt of the disclosure or the pharmaceutical composition of the disclosure in a method for treating or preventing a disease or disorder in a subject.

Further aspects and advantages will be apparent to those of ordinary skill in the art from a review of the following detailed description, taken in conjunction with the drawings. While the compounds and methods disclosed herein are susceptible of cases in various forms, the description hereafter includes specific cases with the understanding that the disclosure is illustrative, and is not intended to limit the invention to the specific cases described herein.

### DETAILED DESCRIPTION

There is a need for novel compounds that modulate aryl hydrocarbon receptor (AHR) activity to provide new and effective therapies for diseases and disorders, such as inflammatory diseases and disorders. Accordingly, provided herein are compounds that can act as potent modulators of AHR activity, such as compounds of Formula (I): 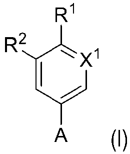 wherein R¹, R², X¹, and A are as described herein.

### Compounds of the Disclosure

In one aspect, disclosed herein are compounds having a structure of Formula (I): 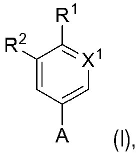 or a pharmaceutically acceptable salt thereof, wherein:
R¹ is C₁₋₆alkyl, C₁₋₆haloalkyl, C₁₋₆hydroxyalkyl, OC₁₋₆alkyl, C(=O)C₁₋₆alkyl, or C(=CH)C₁₋₆alkyl;
R² is OH, OC₁₋₆alkyl, N(R^{N1})₂, or halo;
each R^{N1} independently is H or C₁₋₆alkyl;
X¹ is N, C-OH, or C-OC₁₋₆alkyl;
A is an optionally substituted fused bicyclic heteroaryl group having 8-10 total ring atoms and 1, 2, or 3 heteroatoms selected from N, O, or S; or 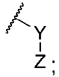
Y is 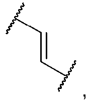 O, S, S=O, or SO₂; and
Z is an optionally substituted phenyl group or an optionally substituted heteroaryl group having 5 or 6 total ring atoms and 1, 2, or 3 heteroatoms selected from N, O, and S;
with the proviso that when R² is OH or OC₁₋₆alkyl and X¹ is C-OH or C-OC₁₋₆alkyl; then Y is not

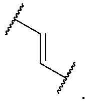

In compounds of Formula (I), R¹ can be C₁₋₆alkyl, C₁₋₆haloalkyl, C₁₋₆hydroxyalkyl, OC₁₋₆alkyl, C(=O)C₁₋₆alkyl, or C(=CH)C₁₋₆alkyl. In some cases, R¹ is C₁₋₆alkyl. In some cases, R¹ is C₁₋₃alkyl. In some cases, R¹ is n-propyl, isopropyl, ethyl, or methyl. In some cases, R¹ is C₃alkyl. In some cases, R¹ is 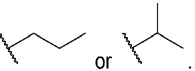 In some cases, R¹ is 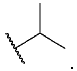 In some cases, R¹ is 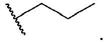 In some cases, R¹ is C₁₋₆haloalkyl, C₁₋₆hydroxyalkyl, OC₁₋₆alkyl, C(=O)C₁₋₆alkyl, or C(=CH)C₁₋₆alkyl. In some cases, R¹ is C₁₋₆haloalkyl (e.g., C₁haloalkyl, C₂haloalkyl, C₃haloalkyl, C₄haloalkyl, C₅haloalkyl, or C₆haloalkyl). In some cases, R¹ is C₁₋₆fluoroalkyl. In some cases, R¹ is CF₃, CHF₂, or CH₂F. In some cases, R¹ is C₁₋₆hydroxyalkyl (e.g., C₁hydroxyalkyl, C₂hydroxyalkyl, C₃hydroxyalkyl, C₄hydroxyalkyl, C₅hydroxyalkyl, or C₆hydroxyalkyl). In some cases, R¹ is 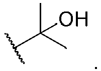 In some cases, R¹ is OC₁₋₆alkyl (e.g., OC₁alkyl, OC₂alkyl, OC₃alkyl, OC₄alkyl, OC₅alkyl, or OC₆alkyl). In some cases, R¹ is 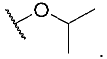 In some cases, R¹ is C(=O)C₁₋₄alkyl (e.g., C(=O)C₁alkyl, C(=O)C₂alkyl, C(=O)C₃alkyl, C(=O)C₄alkyl, C(=O)C₅alkyl, or C(=O)C₆alkyl). In some cases, R¹ is 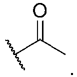 In some cases, R¹ is C(=CH)C₁₋₆alkyl (e.g., C(=CH)C₁alkyl, C(=CH)C₂alkyl, C(=CH)C₃alkyl, C(=CH)C₄alkyl, C(=CH)C₅alkyl, or C(=CH)C₆alkyl). In some cases, R¹ is 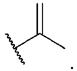 In some cases, R¹ is

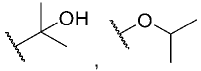

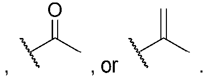

In compounds of Formula (I), R² can be OH, OC₁₋₆alkyl, N(R^{N1})₂, or halo. In some cases, R² is OH or OC₁₋₆alkyl. In some cases, R² is OH. In some cases, R² is OC₁₋₆alkyl (e.g., OC₁alkyl, OC₂alkyl, OC₃alkyl, OC₄alkyl, OC₅alkyl, or OC₆alkyl). In some cases, R² is OCH₃. In some cases, R² is OH or OCH₃. In some cases, R² is N(R^{N1})₂, wherein each R^{N1} independently is H or C₁₋₆alkyl (e.g., CH₃, CH₂CH₃, CH₂CH₂CH₃, or CH(CH₃)₂). In some cases, each R^{N1} independently is H or CH₃. In some cases, each R^{N1} is H. In some cases, each R^{N1} is CH₃. In some cases, one R^{N1} is H and one R^{N1} is CH₃. In some cases, R² is NH₂. In some cases, R² is halo (e.g., F, Cl, Br, or I). In some case, R² is Cl or F. In some cases, R² is F. In some cases, R² is OH, OCH₃, N(R^{N1})₂, or F; and each R^{N1} independently is H or CH₃.

In compounds of Formula (I), X¹ can be N, C-OH, or C-OC₁₋₆alkyl. In some cases, X¹ is N. In some cases, X¹ is C-OH. In some cases, X¹ is C-OC₁₋₆alkyl (e.g., C-OC₁alkyl, C-OC₂alkyl, C-OC₃alkyl, C-OC₄alkyl, C-OC₅alkyl, or C-OC₆alkyl). In some cases, X¹ is C-OCH₃.

In some cases, R² is OH and X¹ is N. In some cases, R² is OH and X¹ is C-OH. In some cases, R² is NH₂ and X¹ is C-OH. In some cases, R² is NH₂ and X¹ is N. In some cases, R² is F and X¹ is C-OH. In some cases, R² is OH and X₁ is C-OCH₃. In some cases, R² is OCH₃ and X₁ is C-OH. In some cases, R² is OCH₃ and X₁ is C-OCH₃.

In some cases, R¹ is 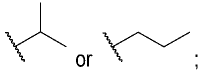 R² is OH, OCH₃, F, or NH₂; and X¹ is N, C-OH, or C-OCH₃. In some cases, R¹ is 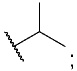 R² is OH, OCH₃, or NH₂; and X¹ is N, C-OH, or C-OCH₃.

In compounds of Formula (I), A can be an optionally substituted fused bicyclic heteroaryl group having 8-10 total ring atoms and 1, 2, or 3 heteroatoms selected from N, O, or S. In some cases, A is an optionally substituted fused bicyclic heteroaryl group having 8 total ring atoms and 1 or 2 heteroatoms selected from N. In some cases, A is an optionally substituted fused bicyclic heteroaryl group having 9 total ring atoms and 1 or 2 heteroatoms selected from N or O. In some cases, A is an optionally substituted fused bicyclic heteroaryl group having 10 total ring atoms and 1 or 2 heteroatoms selected from N. In some cases, A is benzooxazolyl, benzoimidazolyl, indolyl, isoindolyl, indolizinyl, purinyl, benzofuranyl, benzimidazole, quinolinyl, isoquinolinyl, naphthiridinyl, or pyridopyrazinyl, wherein any of the foregoing is unsubstituted or substituted. In some cases, A is benzooxazolyl, benzoimidazolyl, quinolinyl, isoquinolinyl, or naphthiridinyl, wherein any of the foregoing is unsubstituted or substituted. Substitutions are discussed in detail below. In some cases, A is 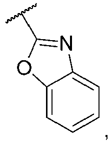 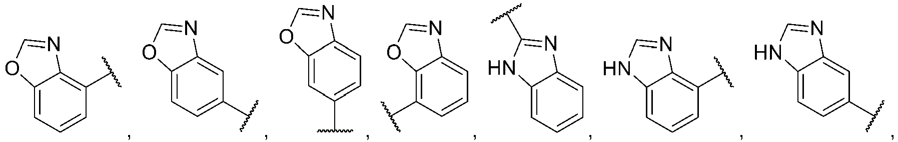 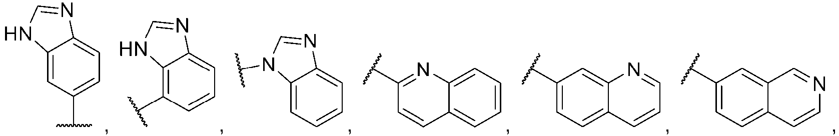 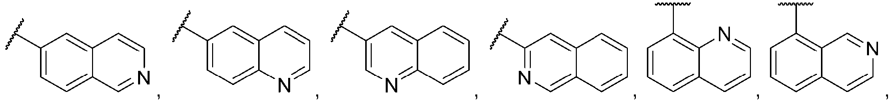 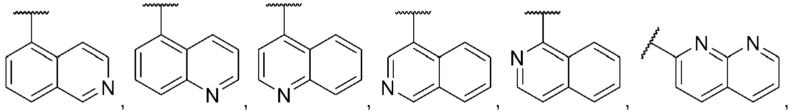 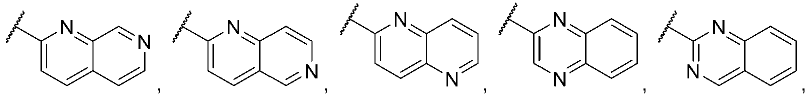 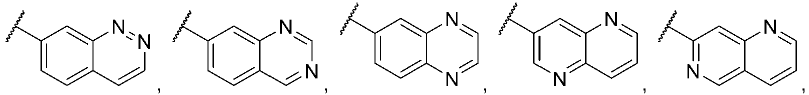 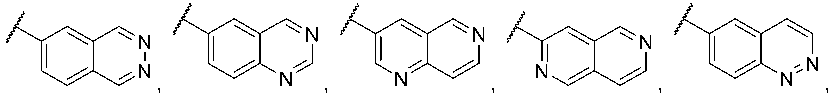 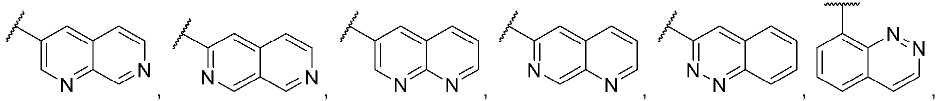 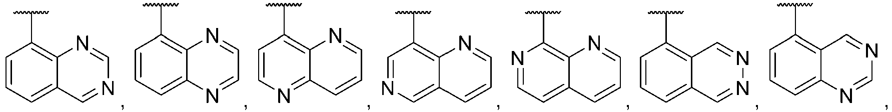 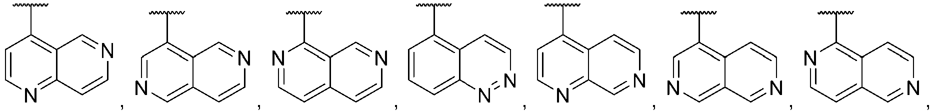 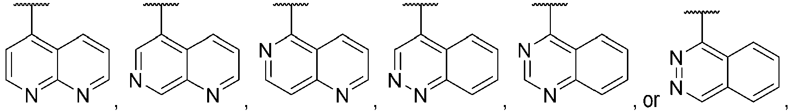 wherein any of the foregoing is unsubstituted or substituted.

In some cases, A is unsubstituted. In some cases, A is 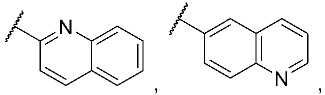 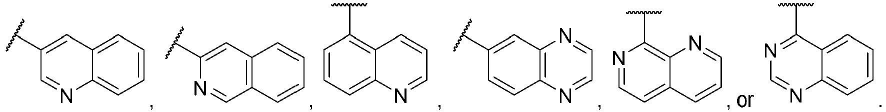 In some cases, A is unsubstituted and selected from the group consisting of 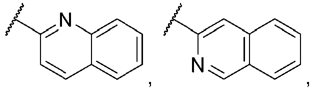 an 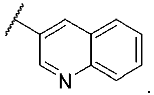 In some cases, the unsubstituted A is 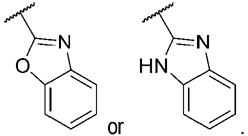 In some cases, unsubstituted A is

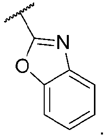

In some cases, A is a fused bicyclic 8-10 membered heteroaryl and is substituted. In some cases, A is substituted with 1, 2, or 3 substituents, wherein each substituent independently is halo, OH, CN, COOH, C=OOC₁₋₆alkyl, C₁₋₆alkyl, C₁₋₆haloalkyl, OC₁₋₆alkyl, OC₁₋₆haloalkyl, C=OC₁₋₆alkyl, or N(R^{N1})₂; wherein each R^{N1} independently is H or C₁₋₆alkyl. In some cases, A is substituted with one substituent, wherein the substituent is halo, OH, CN, COOH, C=OOC₁₋₆alkyl, C₁₋₆alkyl, C₁₋₆haloalkyl, OC₁₋₆alkyl, OC₁₋₆haloalkyl, C=OC₁₋₆alkyl, or N(R^{N1})₂; wherein each R^{N1} independently is H or C₁₋₆alkyl. In some cases, A is substituted with two substituents, wherein each substituent independently is halo, OH, CN, COOH, C=OOC₁₋₆alkyl, C₁₋₆alkyl, C₁₋₆haloalkyl, OC₁₋₆alkyl, OC₁₋₆haloalkyl, C=OC₁₋₆alkyl, or N(R^{N1})₂; wherein each R^{N1} independently is H or C₁₋₆alkyl. In some cases, A is substituted with three substituents, wherein each substituent independently is halo, OH, CN, COOH, C=OOC₁₋₆alkyl, C₁₋₆alkyl, C₁₋₆haloalkyl, OC₁₋₆alkyl, OC₁₋₆haloalkyl, C=OC₁₋₆alkyl, or N(R^{N1})₂; wherein each R^{N1} independently is H or C₁₋₆alkyl. In some cases, A is substituted and at least one substituent is halo. In some cases, A is substituted and at least one substituent is F. In some cases, A is substituted and at least one substituent is Cl. In some cases, A is substituted and at least one substituent is OH. In some cases, A is substituted and at least one substituent is CN. In some cases, A is substituted and at least one substituent is COOH. In some cases, A is substituted and at least one substituent is C=OOC₁₋₆alkyl (e.g., C=OOC₁alkyl, C=OOC₂alkyl, C=OOC₃alkyl, C=OOC₄alkyl, C=OOC₅alkyl, or C=OOC₆alkyl). In some cases, A is substituted and at least one substituent is C=OOCH₃. In some cases, A is substituted and at least one substituent is C₁₋₆alkyl (e.g., CH₃, CH₂CH₃, CH₂CH₂CH₃, or CH(CH₃)₂). In some cases, A is substituted and at least one substituent is CH₃. In some cases, A is substituted and at least one substituent is C₁₋₆haloalkyl (e.g., C₁haloalkyl, C₂haloalkyl, C₃haloalkyl, C₄haloalkyl, C₅haloalkyl, or C₆haloalkyl). In some cases, A is substituted and at least one substituent is C₁₋₆fluoroalkyl. In some cases, A is substituted and at least one substituent is CF₃, CHF₂, or CH₂F. In some cases, A is substituted and at least one substituent is OC₁₋₆alkyl (e.g., OC₁alkyl, OC₂alkyl, OC₃alkyl, OC₄alkyl, OC₅alkyl, or OC₆alkyl). In some cases, A is substituted and at least one substituent is OCH₃. In some cases, A is substituted and at least one substituent is OC₁₋₆haloalkyl (e.g., OC₁haloalkyl, OC₂haloalkyl, OC₃haloalkyl, OC₄haloalkyl, OC₅haloalkyl,or OC₆haloalkyl). In some cases, A is substituted and at least one substituent is OC₁₋₆fluoroalkyl. In some cases, A is substituted and at least one substituent is OCF₃, OCHF₂, or OCH₂F. In some cases, A is substituted and at least one substituent is C=OC₁₋₆alkyl (e.g., C=OC₁alkyl, C=OC₂alkyl, C=OC₃alkyl, C=OC₄alkyl, C=OC₅alkyl, or C=OC₆alkyl. In some cases, A is substituted and at least one substituent is C=OCH₃. In some cases, A is substituted and at least one substituent is N(R^{N1})₂, wherein each R^{N1} independently is H or C₁₋₆alkyl (e.g., CH₃, CH₂CH₃, CH₂CH₂CH₃, or CH(CH₃)₂). In some cases, each R^{N1} independently is H or CH₃. In some cases, each R^{N1} is H. In some cases, each R^{N1} is CH₃. In some cases, one R^{N1} is H and one R^{N1} is CH₃. In some cases, A is substituted and at least one substituent is N(CH₃)₂, NHCH₃, or NH₂. In some cases, A is substituted and at least one substituent is NH₂. In some cases, A is substituted with 1, 2, or 3 substituents, wherein each substituent independently is F, Cl, OH, CN, COOH, CH₃, OCH₃, CF₃, OCF₃, NH₂, NHCH₃, or NH(CH₃)₂. In some cases, A is 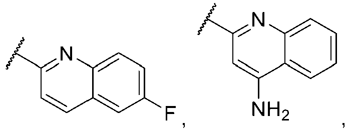 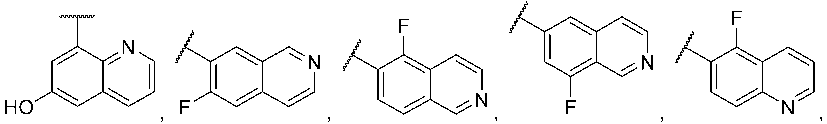 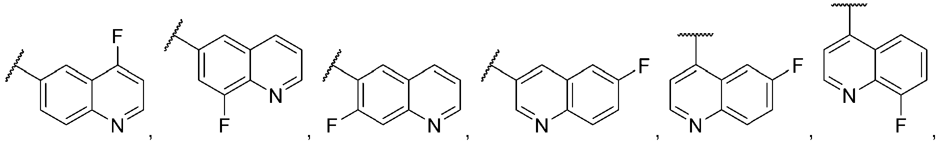 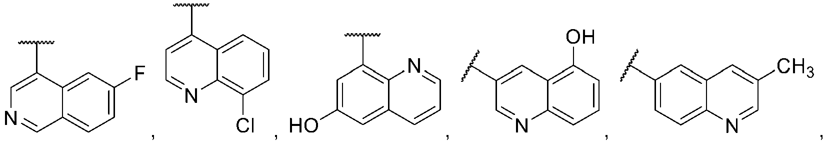 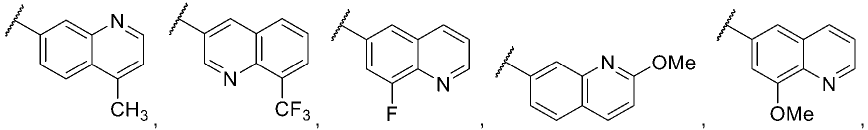 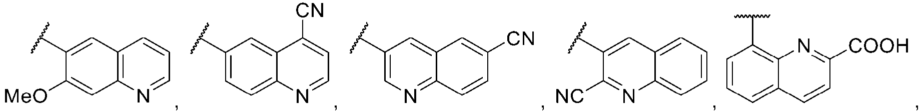 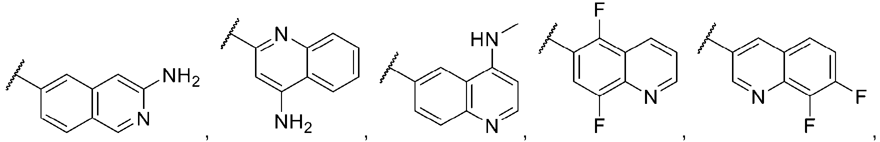 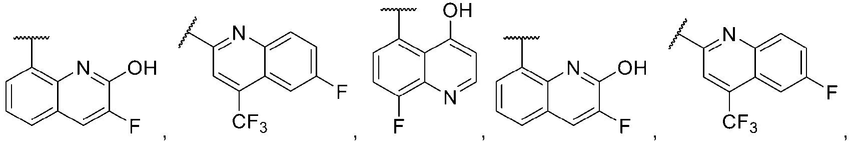 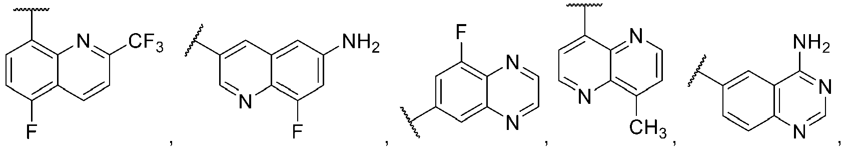 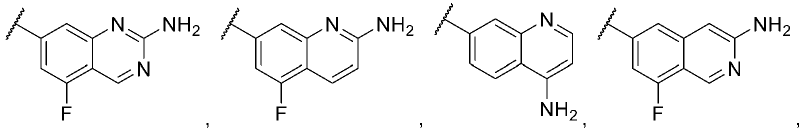 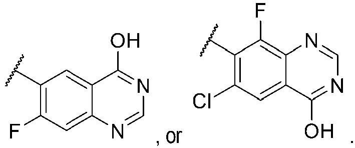 In some cases, A is 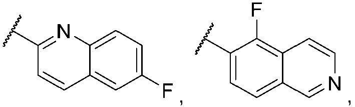 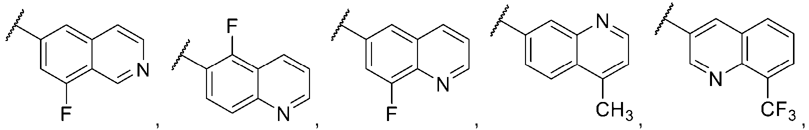 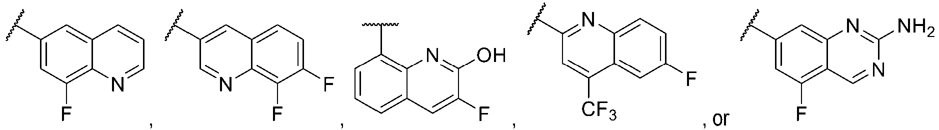 . In some cases, A is

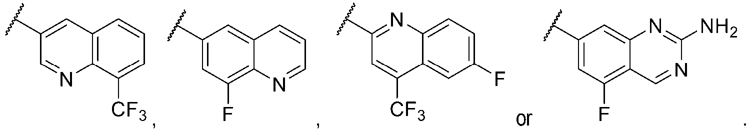

In some cases, A is 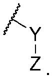 In some cases, Y is 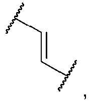 O, S, S=O, or SO₂. In some cases, Y is 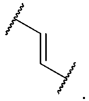 In some cases, Y is O, S, S=O, or SO₂. In some cases, Y is O or S. In some cases, Y is S=O, or SO₂. In some cases, Z is an optionally substituted phenyl group or an optionally substituted heteroaryl group having 5 or 6 total ring atoms and 1, 2, or 3 heteroatoms selected from N, O, and S. In some cases, Z is an optionally substituted phenyl group. In some cases, Z is an unsubstituted phenyl group. In some cases, Z is an unsubstituted heteroaryl group having 6 total ring atoms and 1 or 2 heteroatoms selected from N, O, S. In some cases, Z is unsubstituted pyridyl. In some cases, A is 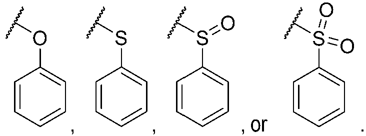 In some cases, Y is O, S, S=O, or SO₂, and Z is pyridyl. In some cases Y is 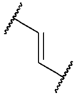 and Z is phenyl or pyridyl. In some cases, A is 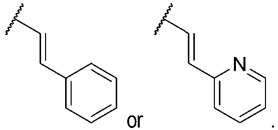 In some cases, Z is substituted. In some cases, Z is a substituted phenyl group. In some cases, Z is a substituted heteroaryl group having 6 total ring atoms and 1 or 2 heteroatoms selected from N, O, S. In some cases, Z is a substituted pyridyl group. In some cases, Z is a substituted phenyl group or a substituted pyridyl group. In some cases, Z is substituted with 1, 2, or 3 substituents, wherein each substituent is selected from halo, OH, CN, COOH, COOC₁₋₆alkyl, C₁₋₆alkyl, C₁₋₆haloalkyl, OC₁₋₆alkyl, OC₁₋₆haloalkyl, C=OC₁₋₆alkyl, and N(R^{N1})₂; wherein each R^{N1} independently is H or C₁₋₆alkyl. In some cases, Z is substituted with OH. In some cases, Z is substituted with CN. In some cases, Z is substituted with COOH. In some cases, Z is substituted with C=OOC₁₋₆alkyl (e.g., C=OOC₁alkyl, C=OOC₂alkyl, C=OOC₃alkyl, C=OOC₄alkyl, C=OOCsalkyl,or C=OOC₆alkyl). In some cases, Z is substituted with C=OOCH₃. In some cases, Z is substituted with C₁₋₆alkyl (e.g., CH₃, CH₂CH₃, CH₂CH₂CH₃, or CH(CH₃)₂). In some cases, Z is substituted with CH₃. In some cases, Z is substituted with C₁₋₆haloalkyl (e.g., C₁haloalkyl, C₂haloalkyl, C₃haloalkyl, C₄haloalkyl, C₅haloalkyl, or C₆haloalkyl). In some cases, Z is substituted with C₁₋₆fluoroalkyl. In some cases, Z is substituted with CF₃, CHF₂, or CH₂F. In some cases, Z is substituted with OC₁₋₆alkyl (e.g., OC₁alkyl, OC₂alkyl, OC₃alkyl, OC₄alkyl, OC₅alkyl, or OC₆alkyl). In some cases, Z is substituted with OCH₃. In some cases, Z is substituted with OC₁₋₆haloalkyl (e.g., OC₁haloalkyl, OC₂haloalkyl, OC₃haloalkyl, OC₄haloalkyl, OC₅haloalkyl, or OC₆haloalkyl). In some cases, Z is substituted with OC₁₋₆fluoroalkyl. In some cases, Z is substituted with OCF₃, OCHF₂, or OCH₂F. In some cases, Z is substituted with C=OC₁₋₆alkyl (e.g., C=OC₁alkyl, C=OC₂alkyl, C=OC₃alkyl, C=OC₄alkyl, C=OC₅alkyl, or C=OC₆alkyl). In some cases, Z is substituted with C=OCH₃. In some cases, Z is substituted with N(R^{N1})₂, wherein each R^{N1} independently is H or C₁₋₆alkyl (e.g., CH₃, CH₂CH₃, CH₂CH₂CH₃, or CH(CH₃)₂). In some cases, each R^{N1} independently is H or CH₃. In some cases, each R^{N1} is H. In some cases, each R^{N1} is CH₃. In some cases, one R^{N1} is H and one R^{N1} is CH₃. In some cases, Z is substituted with N(CH₃)₂, NHCH₃, or NH₂. In some cases, Z is substituted with NH₂. In some cases, Z is substituted with 1, 2, or 3 substituents, wherein each substituent independently is F, Cl, OH, CN, COOH, CH₃, OCH₃, CF₃, OCF₃, NH₂, NHCH₃, or NH(CH₃)₂. In some cases, A is

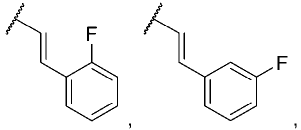

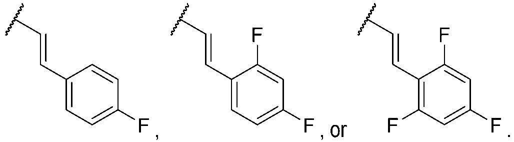

In some cases, R¹ is 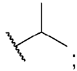 R² is OH, OCH₃, or NH₂; X1 is N, C-OH, or C-OCH₃, and A is unsubstituted quinolinyl, unsubstituted isoquinolinyl, unsubstituted benzooxazolyl, or

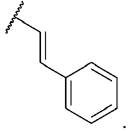

Contemplated compounds of the disclosure include, but are not limited to the compounds listed in Table A, Table B, Table C, and Table D, below.

**Table A**

| Cmpd No. | Structure |
|---|---|
| 1 | 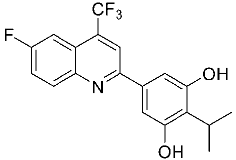 |
| 2 | 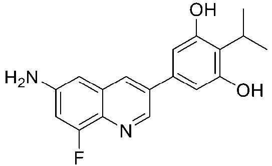 |
| 3 | 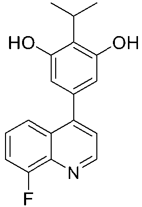 |
| 4 | 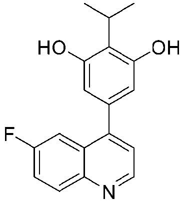 |
| 5 | 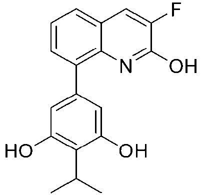 |
| 6 | 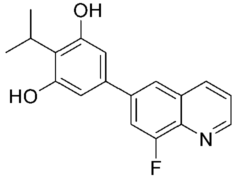 |
| 7 | 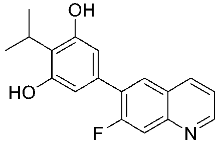 |
| 8 | 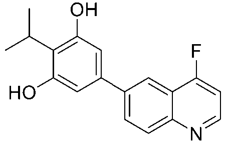 |
| 9 | 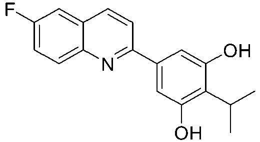 |
| 10 | 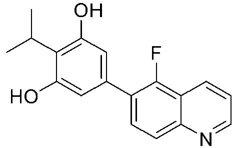 |
| 11 | 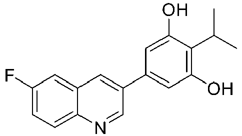 |
| 12 | 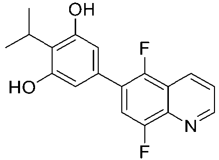 |
| 13 | 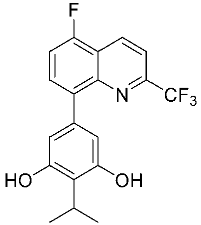 |
| 15 | 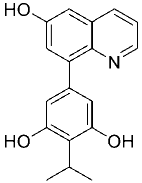 |
| 16 | 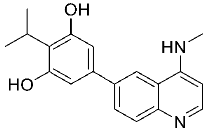 |
| 17 | 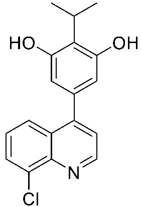 |
| 18 | 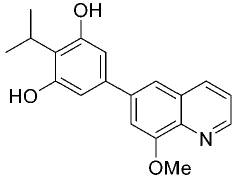 |
| 19 | 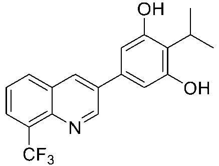 |
| 20 | 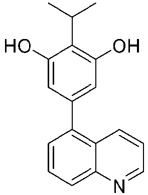 |
| 21 | 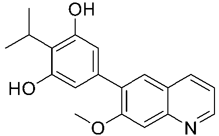 |
| 22 | 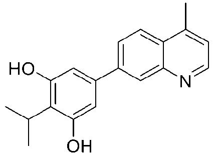 |
| 23 | 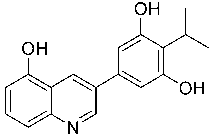 |
| 24 | 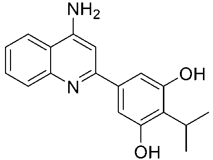 |
| 25 | 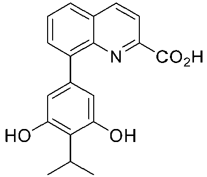 |
| 26 | 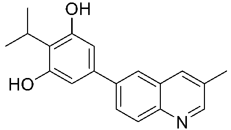 |
| 27 | 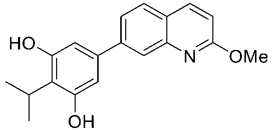 |
| 28 | 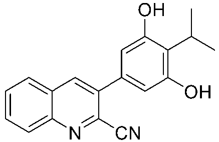 |
| 29 | 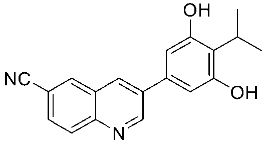 |
| 30 | 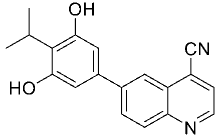 |
| 31 | 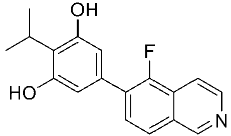 |
| 32 | 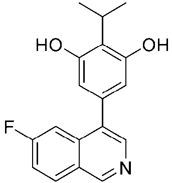 |
| 33 | 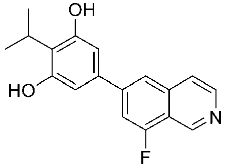 |
| 34 | 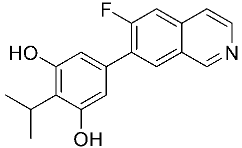 |
| 35 | 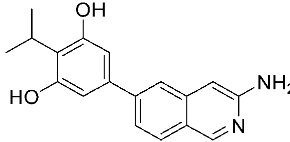 |
| 36 | 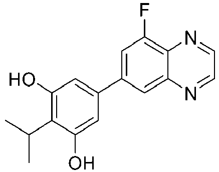 |
| 37 | 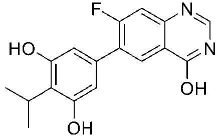 |
| 38 | 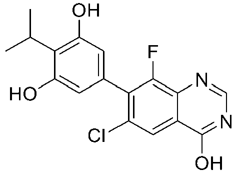 |
| 39 | 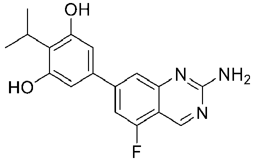 |
| 40 | 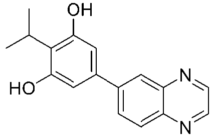 |
| 41 | 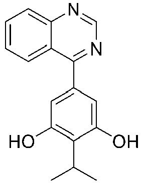 |
| 42 | 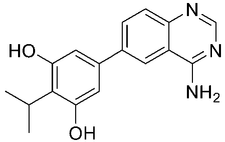 |
| 43 | 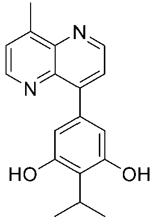 |
| 44 | 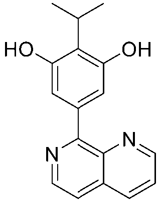 |
| 45 | 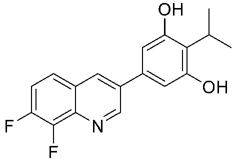 |
| 46 | 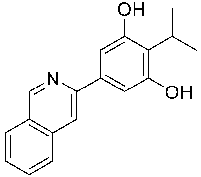 |
| 47 | 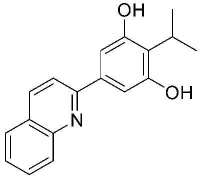 |
| 48 | 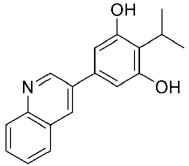 |
| 49 | 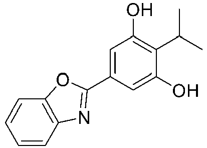 |
| 50 | 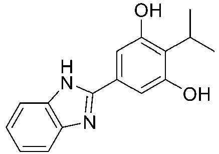 |
| 51 | 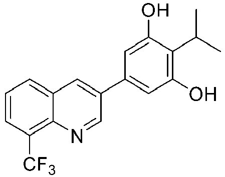 |
| 52 | 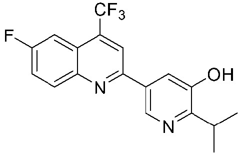 |
| 53 | 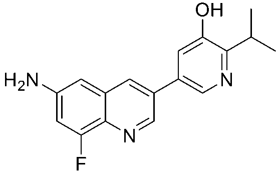 |
| 54 | 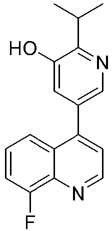 |
| 55 | 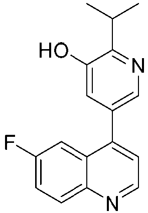 |
| 56 | 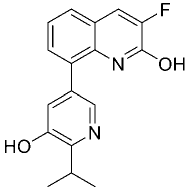 |
| 57 | 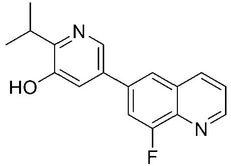 |
| 58 | 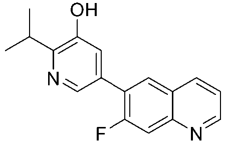 |
| 59 | 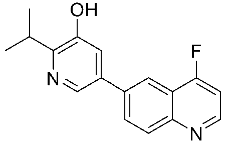 |
| 60 | 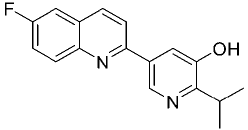 |
| 61 | 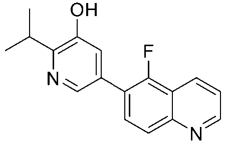 |
| 62 | 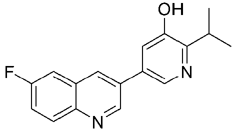 |
| 63 | 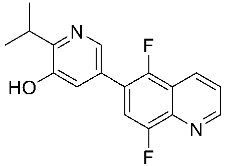 |
| 64 | 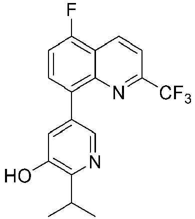 |
| 65 | 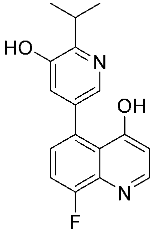 |
| 66 | 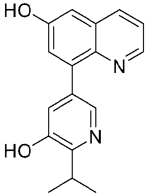 |
| 67 | 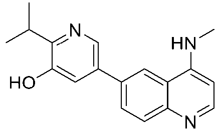 |
| 68 | 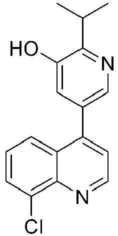 |
| 69 | 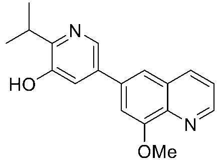 |
| 70 | 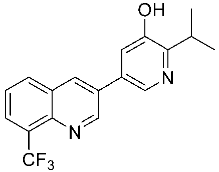 |
| 71 | 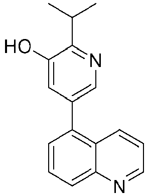 |
| 72 | 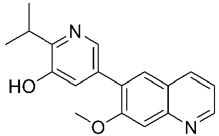 |
| 73 | 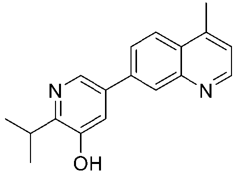 |
| 74 | 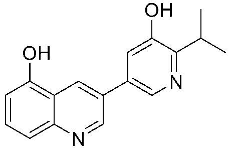 |
| 75 | 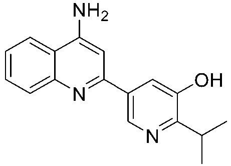 |
| 76 | 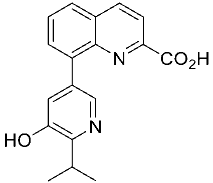 |
| 77 | 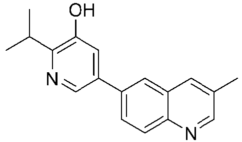 |
| 78 | 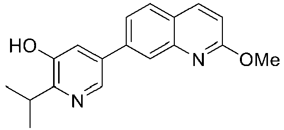 |
| 79 | 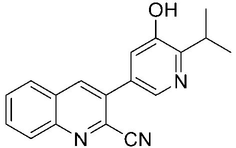 |
| 80 | 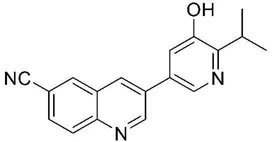 |
| 81 | 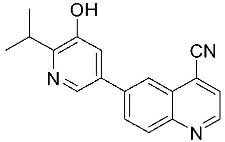 |
| 82 | 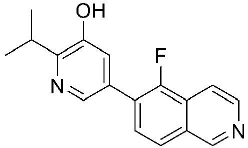 |
| 83 | 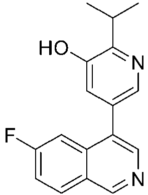 |
| 84 | 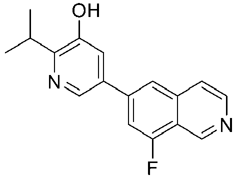 |
| 85 | 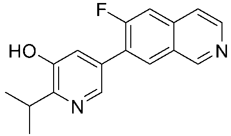 |
| 86 | 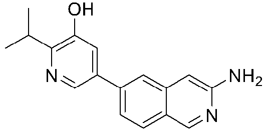 |
| 87 | 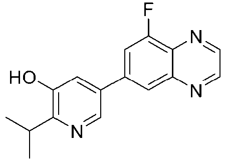 |
| 88 | 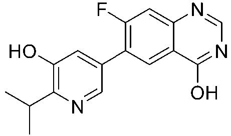 |
| 89 | 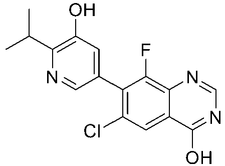 |
| 90 | 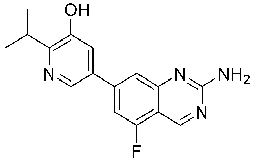 |
| 91 | 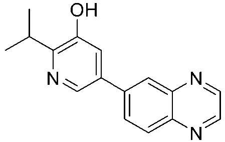 |
| 92 | 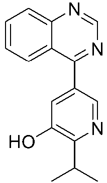 |
| 93 | 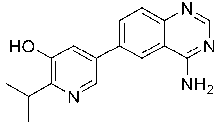 |
| 94 | 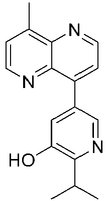 |
| 95 | 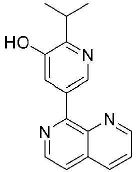 |
| 96 | 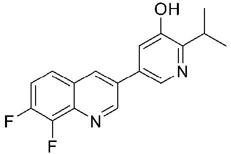 |
| 97 | 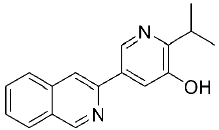 |
| 98 | 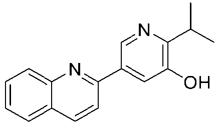 |
| 99 | 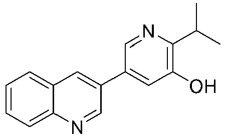 |
| 100 | 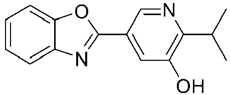 |
| 101 | 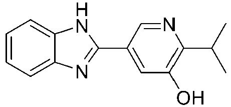 |
| 102 | 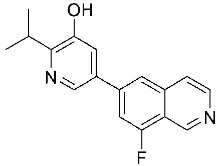 |
| 118 | 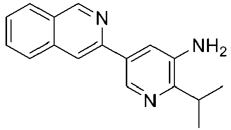 |
| 119 | 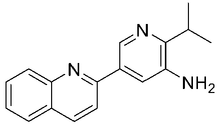 |
| 120 | 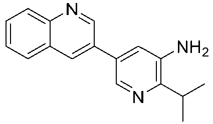 |
| 121 | 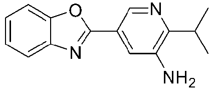 |
| 122 | 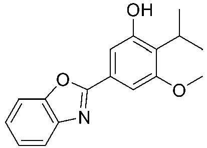 |
| 134 | 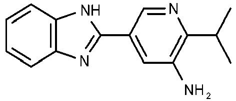 |
| 139 | 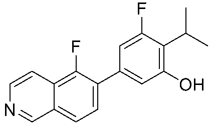 |
| 140 | 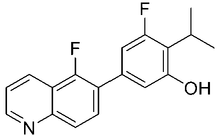 |
| 141 | 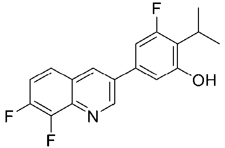 |
| 142 | 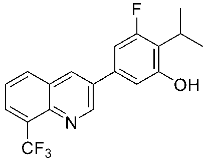 |
| 143 | 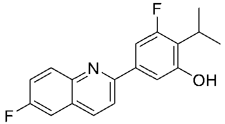 |
| 144 | 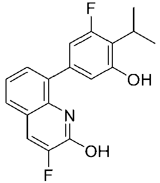 |
| 145 | 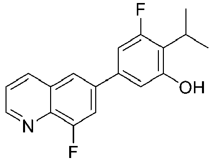 |
| 146 | 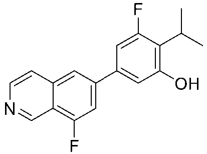 |
| 147 | 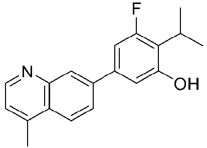 |
| 148 | 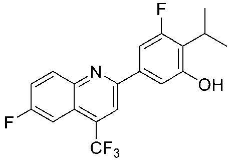 |
| 149 | 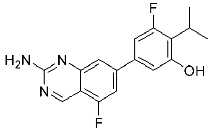 |
| 150 | 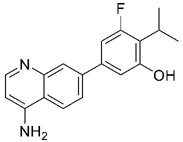 |
| 151 | 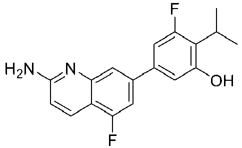 |
| 152 | 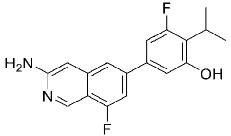 |
| 153 | 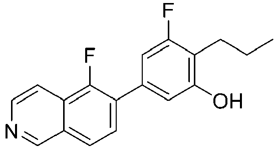 |
| 154 | 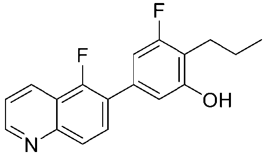 |
| 155 | 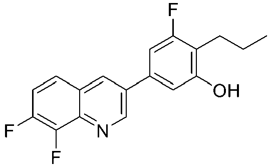 |
| 156 | 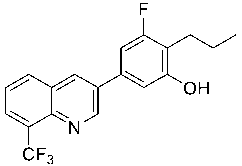 |
| 157 | 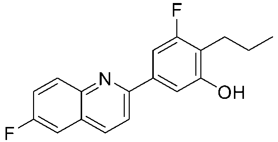 |
| 158 | 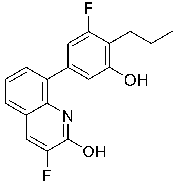 |
| 159 | 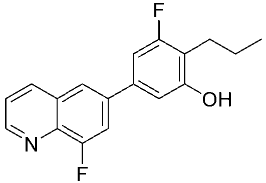 |
| 160 | 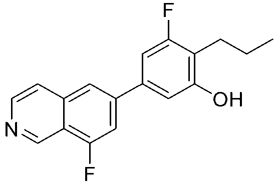 |
| 161 | 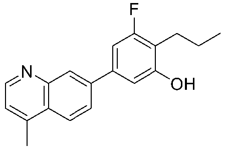 |
| 162 | 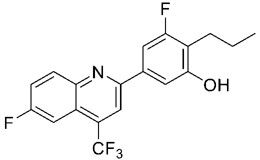 |
| 163 | 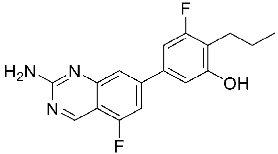 |
| 164 | 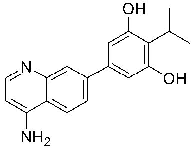 |
| 165 | 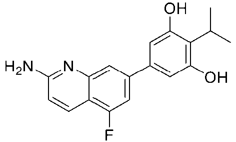 |
| 166 | 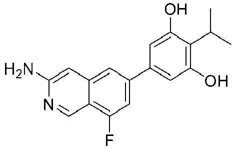 |

**Table B**

| Cmpd No. | Structure |
|---|---|
| 103 | 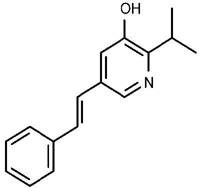 |
| 107 | 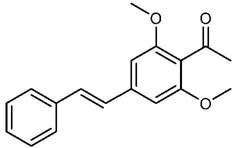 |
| 108 | 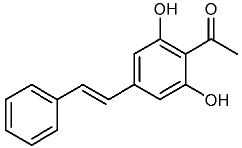 |
| 110 | 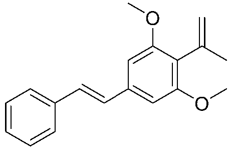 |
| 111 | 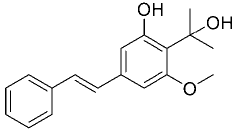 |
| 112 | 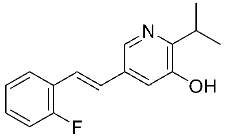 |
| 113 | 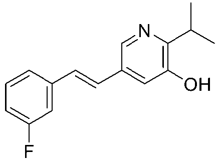 |
| 114 | 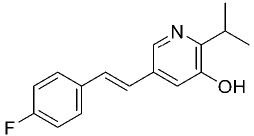 |
| 115 | 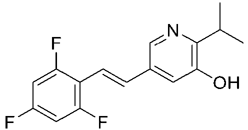 |
| 116 | 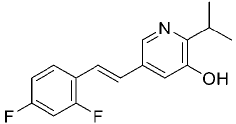 |
| 117 | 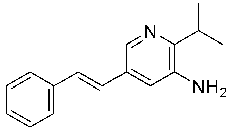 |
| 125 | 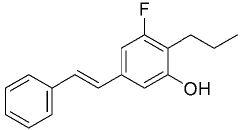 |
| 135 | 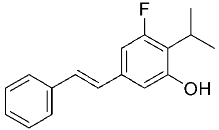 |
| 136 | 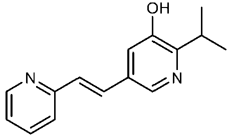 |
| 137 | 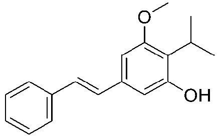 |

**Table C**

| Cmd No. | Structure |
|---|---|
| 129 | 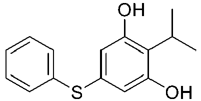 |
| 130 | 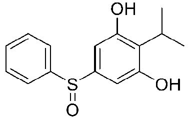 |
| 131 | 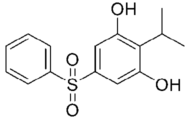 |
| 132 | 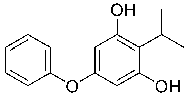 |
| 133 | 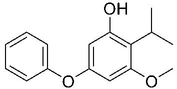 |

**Table D**

| Cmpd No. | Structure |
|---|---|
| 104 | 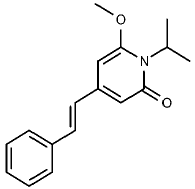 |
| 105 | 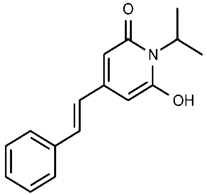 |
| 106 | 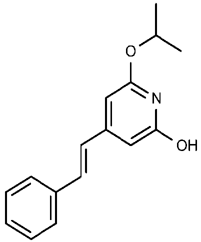 |
| 109 | 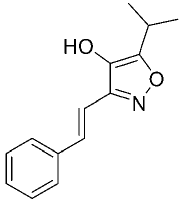 |
| 123 | 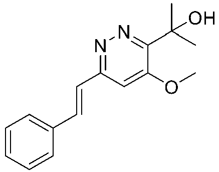 |
| 124 | 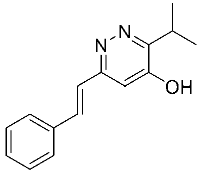 |
| 127 | 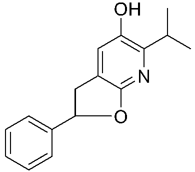 |
| 128 | 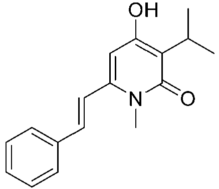 |
| 138 | 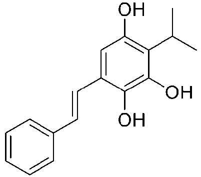 |

In some cases, A is an optionally substituted fused bicyclic heteroaryl group having 8-10 total ring atoms and 1, 2, or 3 heteroatoms selected from N, O, or S, and the compound of Formula (I) is selected from the compounds listed in Table A, or a pharmaceutically acceptable salt of any of the foregoing. In some cases, the compound of Formula (I) is 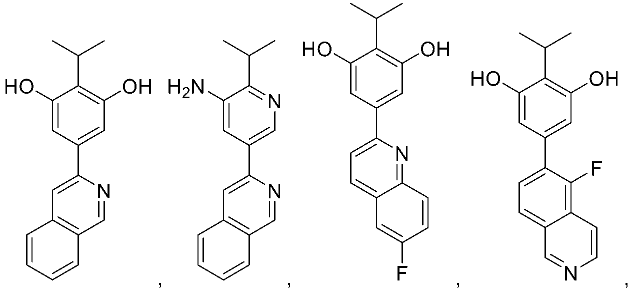 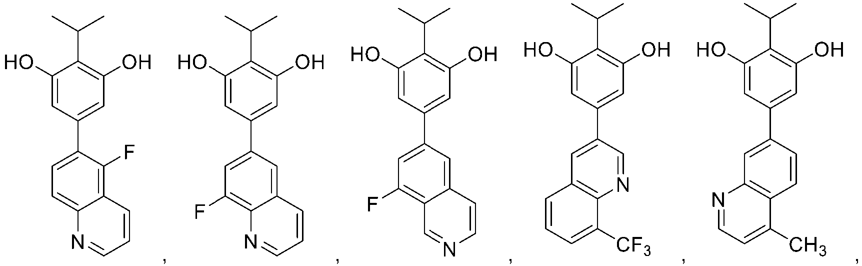 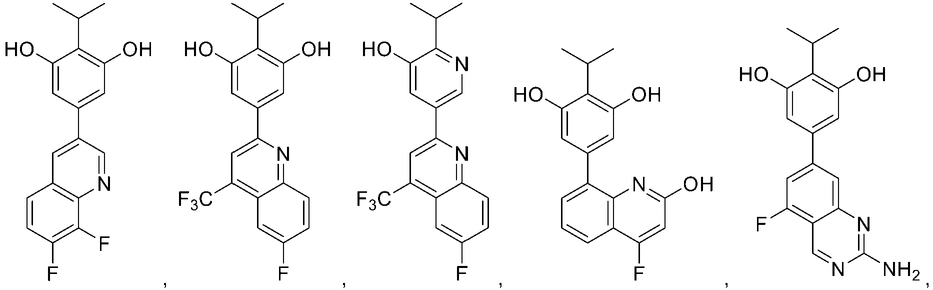 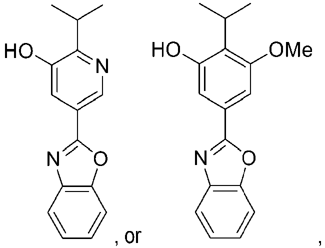 or a pharmaceutically acceptable salt of any of the foregoing. In some cases, the compound of Formula (I) is selected from the compounds listed in Table B, or a pharmaceutically acceptable salt thereof. In some cases, A is 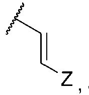 and the compound of Formula (I) is compound 103, 112-117, 125, 135, 136, or a pharmaceutically acceptable salt of any of the foregoing. In some cases, A is 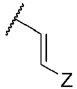 and the compound of Formula (I) is 103, 112, 113, 114, 115, 116, 117, 136, or a pharmaceutically acceptable salt of any of the foregoing. In some cases, A is 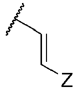 and the compound of Formula (I) is 125, 135, or a pharmaceutically acceptable salt of any of the foregoing. In some cases, the compound of the disclosure is 107, 108, 110, 111, 137, or a pharmaceutically acceptable salt of any of the foregoing. In some cases, the compound of Formula (I) is 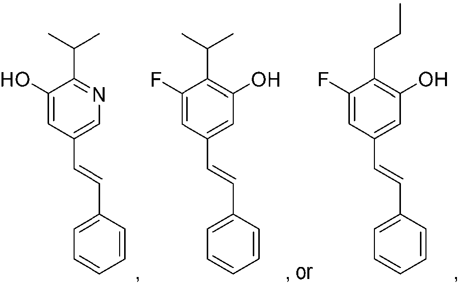 or a pharmaceutically acceptable salt of any of the foregoing. In some cases, the compound of Formula (I) is selected from a compound listed in Table C, or a pharmaceutically acceptable salt thereof. In some cases, the compound of the disclosure is selected from a compound listed in Table D, or a pharmaceutically acceptable salt thereof. In some cases, the compound of Formula (I) is 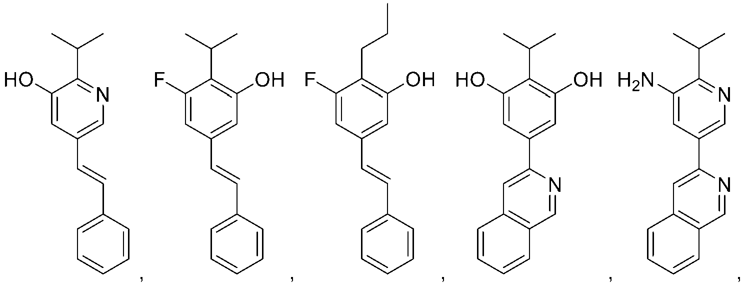 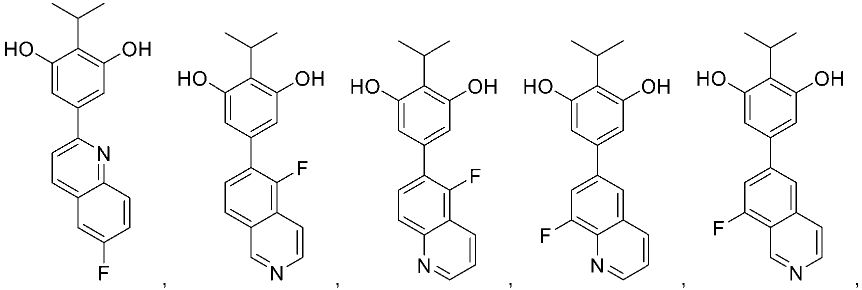 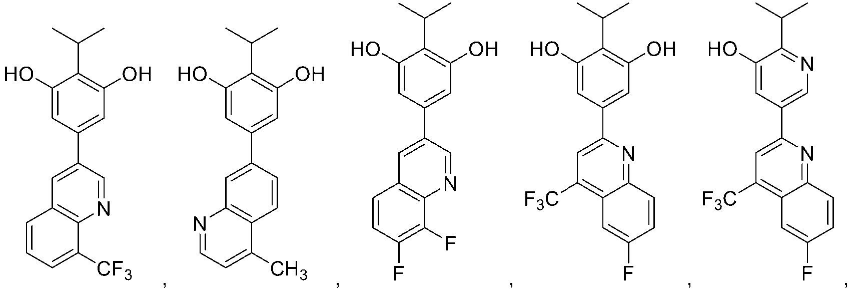 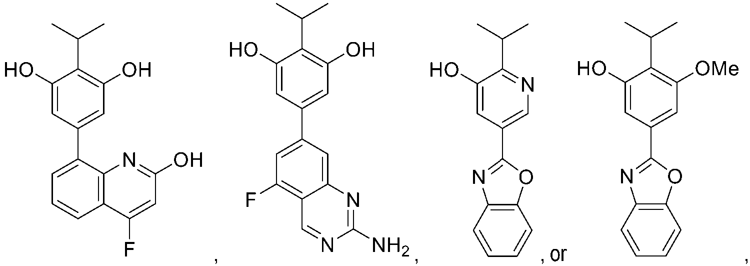 or a pharmaceutically acceptable salt of any of the foregoing.

Unless otherwise indicated, structures depicted herein are also meant to include all isomeric (e.g., enantiomeric, diastereomeric, cis-trans, conformational, and rotational) forms of the structure. For example, the R and S configurations for each asymmetric center, (Z) and (E) double bond isomers, and (Z) and (E) conformational isomers are included in this disclosure, unless only one of the isomers is specifically indicated. Therefore, single stereochemical isomers as well as enantiomeric, diastereomeric, cis/trans, conformational, and rotational mixtures of the present compounds are within the scope of the disclosure. In some cases, the compounds disclosed herein are stereoisomers. "Stereoisomers" refer to compounds that differ in the chirality of one or more stereocenters. Stereoisomers include enantiomers and diastereomers. The compounds disclosed herein can exist as a single stereoisomer, or as a mixture of stereoisomers. Stereochemistry of the compounds shown herein indicate a relative stereochemistry, not absolute, unless discussed otherwise. As indicated herein, a single stereoisomer, diastereomer, or enantiomer refers to a compound that is at least more than 50% of the indicated stereoisomer, diastereomer, or enantiomer, and in some cases, at least 90% or 95% of the indicated stereoisomer, diastereomer, or enantiomer.

The compounds disclosed herein that have a double bond can exhibit E or Z (not shown) stereochemistry at the double bond. In some cases, the compounds of Formula (I) exhibit E stereochemistry at the double bond. In various cases, the compounds of Formula (I) exhibit Z stereochemistry at the double bond. The compounds of Formula (I) can have any stereochemical configuration at the sp³ carbon atoms. In some cases, the compounds of the disclosure are optically pure. As used herein, "optically pure" refers to the presence of only one enantiomer of a compound if multiple stereochemical configurations can exist.

Unless otherwise indicated, all tautomeric forms of the compounds of the disclosure are within the scope of the disclosure.

Additionally, unless otherwise indicated, structures depicted herein are also meant to include compounds that differ only in the presence of one or more isotopically enriched atoms. For example, compounds having the present structures except for the replacement of hydrogen by deuterium or tritium, or the replacement of a carbon by a ¹¹C- ¹³C- or ¹⁴C-enriched carbon are within the scope of this disclosure. Such compounds are useful, for example, as analytical tools or probes in biological assays. Such compounds, especially deuterium analogs, can also be therapeutically useful. Thus, further disclosed herein are deuterated compounds or salts of Formula (I), in which one or more isotopes of hydrogen have been replaced with deuterium. Further disclosed herein are compounds of Formula (I) having one or more tritium atoms. Also disclosed herein are compounds of Formula (I) in which one or more carbon atoms have been replaced by one or more carbon isotopes, such as one or more C¹¹ atoms, one or more C¹³ atoms, one or more C¹⁴ atoms, or any combination of the foregoing.

The compounds of the disclosure are defined herein by their chemical structures and/or chemical names. Where a compound is referred to by both a chemical structure and a chemical name, and the chemical structure and chemical name conflict, the chemical structure is determinative of the compound's identity.

As used herein, "alkyl" refers to straight chained and branched saturated hydrocarbon groups containing one to thirty carbon atoms, for example, one to four carbon atoms (e.g., 1, 2, 3, 4, 5, or 6). The term Cₙ means the alkyl group has "n" carbon atoms. For example, C₃ alkyl refers to an alkyl group that has 3 carbon atoms. C₁₋₆alkyl refers to an alkyl group having a number of carbon atoms encompassing the entire range (i.e., 1 to 6 carbon atoms), as well as all subgroups (e.g., 1-2, 1-3, 1-4, 1-5, 2-3, 2-4, 2-5, 2-6, 3-4, 3-5, 3-6, 4-5, 4-6, 1, 2, 3, 4, 5, and 6 carbon atoms). Nonlimiting examples of alkyl groups include, methyl, ethyl, n-propyl, isopropyl, n-butyl, sec-butyl (2-methylpropyl), and t-butyl (1,1-dimethylethyl). Unless otherwise indicated, an alkyl group can be an unsubstituted alkyl group or a substituted alkyl group.

As used herein, "alkylene" refers to a bivalent saturated aliphatic radical. The term Cₙ means the alkylene group has "n" carbon atoms. For example, C₁₋₆alkylene refers to an alkylene group having a number of carbon atoms encompassing the entire range, as well as all subgroups, as previously described for "alkyl" groups.

As used herein, the term "haloalkyl" refers to an alkyl group in which one or more of the hydrogen atoms are replaced by halogen. Such groups include but are not limited to, chloromethyl, fluoromethyl, difluoromethyl, trifluoromethyl, 1,1-difluoroethyl, 2-fluoroethyl, 1-chloro-2-fluoromethyl and 2-fluoroisobutyl. A haloalkyl may be further substituted or unsubstituted, and some cases relate to a haloalkyl having e.g., 1 to 6 carbon atoms, such as C₁₋₆ haloalkyl.

As used herein, the term "hydroxyalkyl" refers to an alkyl group in which one or more of the hydrogen atoms are replaced by a hydroxyl group (OH). Such groups include but are not limited to, hydroxymethyl, hydroxyethyl, and the like. A hydroxyalkyl may be further substituted or unsubstituted, and some cases relate to a hydroxyalkyl having e.g., 1 to 6 carbon atoms, such as C₁₋₆ hydroxyalkyl.

As used herein, the term "aryl" refers to a monocyclic or bicyclic aromatic group, having 6 to 14 total ring atoms. Unless otherwise indicated, an aryl group can be unsubstituted or substituted. Aryl groups can be isolated (e.g., phenyl) or fused to another aryl group (e.g., naphthyl, or anthracenyl).

As used herein, the term "heteroaryl" refers to a cyclic aromatic ring having five to twelve total ring atoms (e.g., a monocyclic aromatic ring with 5-6 total ring atoms), and containing one to three heteroatoms selected from nitrogen, oxygen, and sulfur atom in the aromatic ring. Unless otherwise indicated, a heteroaryl group can be unsubstituted or substituted. Heteroaryl groups can be isolated (e.g., pyridyl) or fused to another heteroaryl group (e.g., purinyl), a cycloalkyl group (e.g., tetrahydroquinolinyl), a heterocycloalkyl group (e.g., dihydronaphthyridinyl), and/or an aryl group (e.g., benzothiazolyl and quinolyl). Examples of heteroaryl groups include, but are not limited to, pyrazolyl, thienyl, furyl, pyridyl, pyrrolyl, oxazolyl, quinolyl, thiophenyl, isoquinolyl, indolyl, triazinyl, triazolyl, isothiazolyl, isoxazolyl, imidazolyl, benzothiazolyl, pyrazinyl, pyrimidinyl, thiazolyl, and thiadiazolyl. In some cases, a heteroaryl group is fused to another heteroaryl group and each ring can contain five or six total ring atoms and one to three heteroatoms in its aromatic ring. Examples of fused heteroaryl groups include, but are not limited to, benzooxazolyl, benzoimidazolyl, indolyl, isoindolyl, indolizinyl, purinyl, benzofuranyl, benzimidazole, quinolinyl, isoquinolinyl, naphthiridinyl, and pyridopyrazinyl.

As used herein, the term "halo" refers to a fluoro (F), chloro (Cl), bromo (Br), or iodo (I) group.

A "substituted" functional group (e.g., a substituted alkyl, alkyleneyl, cycloalkyl, aryl, or heteroaryl refers to an alkyl, alkyleneyl, cycloalkyl, aryl, or heteroaryl) is a functional, group having at least one hydrogen radical that is substituted with a non-hydrogen radical (i.e., a substitutent). Examples of non-hydrogen radicals (or substituents) include, but are not limited to, alkyl, cycloalkyl, alkenyl, cycloalkenyl, alkynyl, ether, aryl, heteroaryl, heterocycloalkyl, hydroxyl, oxy (or oxo), alkoxyl, ester, thioester, acyl, carboxyl, cyano, nitro, amino, sulfhydryl, and halo. When a substituted alkyl group includes more than one non-hydrogen radical, the substituents can be bound to the same carbon or two or more different carbon atoms.

### Pharmaceutically Acceptable Salts

The compounds described herein can exist in free form, or where appropriate, as salts. Those salts that are pharmaceutically acceptable are of particular interest since they are useful in administering the compounds described herein for medical purposes. Salts that are not pharmaceutically acceptable are useful in manufacturing processes, for isolation and purification purposes, and in some cases, for use in separating stereoisomeric forms of the compounds of the disclosure or intermediates thereof.

As used herein, the term "pharmaceutically acceptable salt" refers to salts of a compound which are, within the scope of sound medical judgment, suitable for use in contact with the tissues of humans and lower animals without undue side effects, such as, toxicity, irritation, allergic response and the like, and are commensurate with a reasonable benefit/risk ratio.

Pharmaceutically acceptable salts are well known in the art. For example, S. M. Berge et al., describe pharmaceutically acceptable salts in detail in J. Pharmaceutical Sciences, 1977, 66, 1-19, which is incorporated herein by reference. Pharmaceutically acceptable salts of the compounds described herein include those derived from suitable inorganic and organic acids and bases. These salts can be prepared *in situ* during the final isolation and purification of the compounds.

Where the compound described herein contains a basic group, or a sufficiently basic bioisostere, acid addition salts can be prepared by 1) reacting the purified compound in its free-base form with a suitable organic or inorganic acid and 2) isolating the salt thus formed. In practice, acid addition salts might be a more convenient form for use and use of the salt amounts to use of the free basic form.

Examples of pharmaceutically acceptable, non-toxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, glycolate, gluconate, glycolate, hemisulfate, heptanoate, hexanoate, hydrochloride, hydrobromide, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, palmoate, pectinate, persulfate, 3-phenylpropionate, phosphate, picrate, pivalate, propionate, salicylate, stearate, succinate, sulfate, tartrate, thiocyanate, p-toluenesulfonate, undecanoate, valerate salts, and the like.

Where the compound described herein contains a carboxyl group or a sufficiently acidic bioisostere, base addition salts can be prepared by 1) reacting the purified compound in its acid form with a suitable organic or inorganic base and 2) isolating the salt thus formed. In practice, use of the base addition salt might be more convenient and use of the salt form inherently amounts to use of the free acid form. Salts derived from appropriate bases include alkali metal (e.g., sodium, lithium, and potassium), alkaline earth metal (e.g., magnesium and calcium), ammonium and N⁺(C₁-₄alkyl)₄ salts. This disclosure also envisions the quaternization of any basic nitrogen-containing groups of the compounds disclosed herein. Water or oil-soluble or dispersible products may be obtained by such quaternization.

Basic addition salts include pharmaceutically acceptable metal and amine salts. Suitable metal salts include the sodium, potassium, calcium, barium, zinc, magnesium, and aluminum. The sodium and potassium salts are usually preferred. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate. Suitable inorganic base addition salts are prepared from metal bases which include sodium hydride, sodium hydroxide, potassium hydroxide, calcium hydroxide, aluminum hydroxide, lithium hydroxide, magnesium hydroxide, zinc hydroxide and the like. Suitable amine base addition salts are prepared from amines which are frequently used in medicinal chemistry because of their low toxicity and acceptability for medical use. Ammonia, ethylenediamine, N-methyl-glucamine, lysine, arginine, ornithine, choline, N,N'-dibenzylethylenediamine, chloroprocaine, dietanolamine, procaine, N-benzylphenethylamine, diethylamine, piperazine, tris(hydroxymethyl)-aminomethane, tetramethylammonium hydroxide, triethylamine, dibenzylamine, ephenamine, dehydroabietylamine, N-ethylpiperidine, benzylamine, tetramethylammonium, tetraethylammonium, methylamine, dimethylamine, trimethylamine, ethylamine, basic amino acids, dicyclohexylamine and the like.

Other acids and bases, although not in themselves pharmaceutically acceptable, may be employed in the preparation of salts useful as intermediates in obtaining the compounds described herein and their pharmaceutically acceptable acid or base addition salts.

It should be understood that a compound disclosed herein can be present as a mixture/combination of different pharmaceutically acceptable salts. Also contemplated are mixtures/combinations of compounds in free form and pharmaceutically acceptable salts.

### Pharmaceutical Formulations

Also provided herein are pharmaceutical formulations that include an effective amount of compounds of the disclosure and one or more pharmaceutically acceptable excipients. As used herein, the term "formulation" is used interchangeable with "composition."

An "effective amount" includes a "therapeutically effective amount" and a "prophylactically effective amount." The term "therapeutically effective amount" refers to an amount effective in treating and/or ameliorating a disease or condition in a subject. The term "prophylactically effective amount" refers to an amount effective in preventing and/or substantially lessening the chances of a disease or condition in a subject. As used herein, the terms "patient" and "subject" may be used interchangeably and mean animals, such as dogs, cats, cows, horses, and sheep (i.e., non-human animals) and humans. Particular patients or subjects are mammals (e.g., humans). The terms "patient" and "subject" include males and females.

As used herein, the term "excipient" means any pharmaceutically acceptable additive, carrier, diluent, adjuvant, or other ingredient, other than the active pharmaceutical ingredient (API), suitably selected with respect to the intended form of administration, and consistent with conventional pharmaceutical practices.

The compounds of the disclosure can be administered alone or as part of a pharmaceutically acceptable composition or formulation. In addition, the compounds can be administered all at once, as for example, by a bolus injection, multiple times, e.g. by a series of tablets, or delivered substantially uniformly over a period of time, as for example, using transdermal delivery. It is also noted that the dose of the compound can be varied over time.

The compounds disclosed herein and other pharmaceutically active compounds, if desired, can be administered to a subject or patient by any suitable route, e.g. orally, topically, rectally, parenterally, (for example, subcutaneous injections, intravenous, intramuscular, intrasternal, and intrathecal injection or infusion techniques), or as a buccal, inhalation, or nasal spray. The administration can be to provide a systemic effect (e.g. eneteral or parenteral). All methods that can be used by those skilled in the art to administer a pharmaceutically active agent are contemplated. In some cases, the disclosed formulations can be administered orally or topically.

Suitable oral compositions or formulations in accordance with the disclosure include without limitation tablets, troches, lozenges, aqueous or oily suspensions, dispersible powders or granules, emulsion, hard or soft capsules, syrups or elixirs. Compositions or formulations suitable for oral use may be prepared according to any method known to the art for the manufacture of pharmaceutical compositions.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

The active compounds can also be in microencapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

The pharmaceutical compositions and formulations described herein may also be administered topically or transdermally, especially when the target of treatment includes areas or organs readily accessible by topical application, including diseases of the eye, the skin, or the lower intestinal tract. Suitable topical formulations are readily prepared for each of these areas or organs. Topical application for the lower intestinal tract, e.g., can be effected in a rectal suppository formulation or in a suitable enema formulation. Dosage forms for topical or transdermal administration of a compound described herein include ointments, pastes, creams, lotions, gels, powders, solutions, sprays, inhalants, suppositories, or patches.

For topical applications, the pharmaceutical compositions may be formulated in a suitable ointment, cream, lotion, or gel, containing the active component suspended or dissolved in one or more carriers, and any needed preservatives or buffers as may be required. Carriers for topical administration of the compounds of this disclosure include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2 octyldodecanol, benzyl alcohol and water.

Ophthalmic formulation, eardrops, and eye drops are also contemplated as being within the scope of this disclosure. Additionally, the present disclosure contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound described herein, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are specifically suppositories which can be prepared by mixing the compounds described herein with suitable non-irritating excipients or carriers such as cocoa butter, polyethylene glycol or a suppository wax which are solid at ambient temperature but liquid at body temperature and therefore melt in the rectum or vaginal cavity and release the active compound.

Sterile injectable forms of the compositions described herein may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutical compositions may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The compounds for use in the methods of the disclosure can be formulated in unit dosage form. The term "unit dosage form" refers to physically discrete units suitable as unitary dosage for subjects undergoing treatment, with each unit containing a predetermined quantity of active material calculated to produce the desired therapeutic effect, optionally in association with a suitable pharmaceutical carrier. The unit dosage form can be for a single daily dose or one of multiple daily doses (e.g., about 1 to 4 or more times per day). When multiple daily doses are used, the unit dosage form can be the same or different for each dose.

The compounds of the disclosure can be administered to a subject or patient at dosage levels in the range of about 0.1 to about 3,000 mg per day. For a normal adult human having a body weight of about 70 kg, a dosage in the range of about 0.01 to about 100 mg per kilogram body weight is typically sufficient. The specific dosage and dosage range that will be used can potentially depend on a number of factors, including the requirements of the subject or patient, the severity of the condition or disease being treated, and the pharmacological activity of the compound being administered. The determination of dosage ranges and optimal dosages for a particular subject or patient is within the ordinary skill in the art.

### Methods of Treatment

The compounds disclosed herein (e.g., the compounds of Formula (I), the compounds listed in Tables A, B, C, and D), and pharmaceutically acceptable salts thereof, can modulate the aryl hydrocarbon receptor (AHR) pathway, e.g., by modulating AHR in a cell. In some cases, the compounds of the disclosure act as agonists of the AHR. In some cases, the compounds of the disclosure act as antagonists of the AHR. AHR is a ligand-activated transcription factor that has been implicated in a variety of conditions, including by modulating the immune system during steady state and during infection and inflammation. The AHR pathway has been recognized for its role in the pathogenesis of inflammatory skin diseases such as atopic dermatitis, acne, psoriasis, and vitiligo; intestinal pathologies, such as inflammatory bowel disorder, necrotizing enterocolitis and other autoimmune diseases, and for colorectal cancer; in diseases and disorders of the bones and joints, including rheumatoid arthritis; diseases of the eye, such as age-related macular degeneration; and diseases of the lung, such as lung fibrosis, chronic obstructive pulmonary disease. The AHR pathway also has been recognized for its role in the pathogenesis of diseases and disorders including neuroinflammatory diseases and disorders, neurodegenerative diseases and disorders, cancer, diabetes, viral, and bacterial infections.

Thus, the disclosure provides a method of modulating the aryl hydrocarbon receptor (AHR) in a cell comprising contacting the cell with a therapeutically effective amount of a compound or salt disclosed herein (e.g., the compounds of Formula (I), the compounds listed in Tables A, B, C, and D, and pharmaceutically acceptable salts of any of the foregoing) or a formulation thereof, in an amount effective to modulate the AHR. In some cases, the contacting occurs *in vitro.* In some cases, the contacting occurs *in vivo.* In some cases, the contacting comprises administering to a subject in need thereof. As used herein, the terms "patient" and "subject" may be used interchangeably and mean animals, such as dogs, cats, cows, horses, and sheep (i.e., non-human animals) and humans. Particular patients are mammals (e.g., humans). In some cases, the subject suffers from an inflammatory disease or disorder. In some cases, the subject suffers from a disease or disorder of the gastrointestinal tract, skin, lung, eye, or bone joints. In some cases, the subject suffers from cancer or a viral infection.

Another aspect of the disclosure provides a method of treating a disease or disorder in a subject, comprising administering to the subject a therapeutically effective amount of a compound or salt disclosed herein (such as a compound of Formula (I), or a compound listed in Table A, B, C, or D), or a formulation thereof. In some cases, the terms "treating", "treat" or "treatment" and the like can include preventative (e.g., prophylactic) and palliative treatment. In some cases, the disease or disorder is an inflammatory disease or disorder. In some cases, the disease or disorder is a disease or disorder of the gastrointestinal tract, skin, lung, central nervous system ("CNS"), pancreas, eye, bones, or bone joints, a neuroinflammatory disease, or a neurodegenerative disease. In some cases, the disease or disorder is diabetes, cancer, a viral infection, or a bacterial infection.

In some cases, the disease or disorder of the gastrointestinal tract is colitis, inflammatory bowel disease, Crohn's disease, celiac disease, necrotizing enterocolitis, irritable bowel syndrome, chronic idiopathic constipation, traveler's diarrhea, or colorectal cancer. In some cases, the disease or disorder of the gastrointestinal tract is colitis, inflammatory bowel disease, Crohn's disease, celiac disease, necrotizing enterocolitis, or irritable bowel syndrome. In some cases, the disease or disorder of the eye is abnormal eye movements, inflammatory ocular disease, autoimmune ocular disease, hereditary ocular disease, degenerative ocular disease, vascularization ocular disease, dry and wet age-related macular degeneration ("AMD"), Uveitis, retinitis pigmentosa ("RP"), primary open-angle glaucoma ("POAG"), primary congenital glaucoma, Behcet's disease, or Leber congenital amaurosis ("LCA"). In some cases, the disease or disorder of the skin is atopic dermatitis, acne, psoriasis, or vitiligo. In some cases, the disease or disorder of the bone or bone joints is osteoporosis, rheumatoid arthritis, or bone cancer. In some cases, the disease or disorder of the central nervous system is Alzheimer's Disease and other memory disorders, Amyotrophic Lateral Sclerosis (ALS, Lou Gehrig's disease, or motor neuron disease). In some cases, the disease or disorder of the pancreas is type 1 diabetes. In some cases, the autoimmune disease or disorder is Hidradenitis Suppurativa, Systemic Sclerosis, Lupus Nephritis, Ankylosing Spondylitis, Non-Radiographic Axial Spondyloarthritis (NR-Ax-SpA), Rheumatoid Arthritis, Juvenile Idiopathic arthritis (JIA), Crohn's Disease, Ulcerative Colitis, Systemic Lupus Erythematosus, Atopic Dermatitis, Chronic Obstructive Pulmonary Disease, Severe Asthma, Psoriasis, Sjogren's Disease, Chronic Spontaneous Urticaria, Cutaneous Lupus Erythematosus, Still's Disease, Idiopathic Pulmonary Fibrosis, Dermatomyositis, Sarcoidosis, Psoriatic Arthritis, Pustular Psoriasis, Osteoarthritis, Alopecia Areata, Giant Cell Arteritis (GCA), ANCA Vasculitis, Vasculitis, Eosinophilic Esophagitis, Acute Respiratory Distress Syndrome, Severe Acne or Lichen Planus. In some cases, the neuroinflammatory disease or disorder is Multiple Sclerosis, Rasmussen's encephalitis, acute disseminated encephalomyelitis (ADEM), Autoimmune encephalitis, transverse myelitis (TM), or anti-NMDA receptor encephalitis. In some cases, the neurodegenerative disease or disorder is Uveitis, optic neuritis, neuromyelitis optica spectrum disorder (NMOSD), or Age-related Macular Degeneration (AMD).

In some cases, the disease or disorder is diabetes (e.g., type 1 diabetes or type 2 diabetes). In some cases, the disease or disorder is cancer. Contemplated cancers that can be treated using the compounds and methods described herein include, but are not limited to, a hematological cancer, a lymphoma, a myeloma, a leukemia, a neurological cancer, skin cancer, breast cancer, a prostate cancer, a cancer of the respiratory tract, a cancer of the reproductive organs, a cancer of the digestive tract, a colorectal cancer, lung cancer, head and neck cancer, a gastrointestinal cancer, a liver cancer, a pancreatic cancer, a genitourinary cancer, a bone cancer, renal cancer, and a vascular cancer.

In some cases, the disease or disorder is a viral infection. In some cases, the compounds of the disclosure (e.g., the compounds of Formula (I), the compounds listed in Tables A, B, C, and D, and pharmaceutically acceptable salts of any of the foregoing), or a composition thereof are useful for the treatment of a viral infection. In some cases, the viral infection to be treated using the compounds and methods described herein include, but are not limited to, coronavirus infections (e.g., SARS, MERS, and COVID-19, such as SARS-CoV infection, MERS-CoV infection, and SARS-CoV-2 infection, respectively) and flavivirus infections (e.g., West Nile virus infection, dengue virus infection, tick-borne encephalitis virus infection, yellow fever virus infection, Zika virus infection, and several other viruses which may cause encephalitis).

In some cases, the disease or disorder is a bacterial infection. In some cases, the compounds of the disclosure (e.g., the compounds of Formula (I), the compounds listed in Tables A, B, C, and D, and pharmaceutically acceptable salts of any of the foregoing), or a composition thereof are useful for the treatment of a bacterial infection. In some cases, the bacterial infection to be treated using the compounds and methods described herein include, but are not limited to, a pulmonary infection, gastrointestinal infection, skin infection, ear infection or a septicemia.

Another aspect of the disclosure provides the use of a compound disclosed herein (such as a compound of Formula (I), or a compound listed in Table A, B, C, or D), a pharmaceutically acceptable salt thereof, or a formulation comprising a compound or salt disclosed herein in the treatment of an inflammatory disease or disorder, such as disease or disorder of the gastrointestinal tract, skin, lung, central nervous system, pancreas, eye, bones or bone joints, neuroinflammatory diseases and disorders, or neurodegenerative diseases and disorders.

Also contemplated is the use of a compound disclosed herein (such as a compound of Formula (I), or a compound listed in Table A, B, C, or D), a pharmaceutically acceptable salt thereof, or a formulation comprising a compound or salt disclosed herein for the manufacture of a medicament in the treatment of an inflammatory disease or disorder, such as disease or disorder of the gastrointestinal tract, skin, lung, central nervous system, pancreas, eye, bones or bone joints, neuroinflammatory diseases and disorders, or neurodegenerative diseases and disorders.

Use of a compound disclosed herein (such as a compound of Formula (I), or a compound listed in Table A, B, C, or D), a pharmaceutically acceptable salt thereof, or a formulation comprising a compound or salt disclosed herein to treat an inflammatory disease or disorder, such as disease or disorder of the gastrointestinal tract, skin, lung, central nervous system, pancreas, eye, bones or bone joints, neuroinflammatory diseases and disorders, or neurodegenerative diseases and disorders also is contemplated.

In some cases, the compound of Formula (I) as disclosed herein, or pharmaceutically acceptable salt thereof, can be administered in combination with another therapeutic agent to treat a disease or disorder, such as a disease or disorder disclosed herein. In some cases, the combination therapy can be used to treat an inflammatory disease or disorder (e.g., a disease or disorder of the gastrointestinal tract, skin, eye, lung, or bone joints). The terms "co-administration," "administration with," "administration in combination with," or the like, as used herein, encompass administration of the selected therapeutic agents to a single patient, and are intended to include treatment regimens in which the agents are administered by the same or different route of administration or at the same or different time. Thus, in any of the methods disclosed herein, treatment of a disease or disorder disclosed herein, such as an inflammatory disease or disorder (e.g., a disease or disorder of the gastrointestinal tract, skin, lung, central nervous system, pancreas, eye, bones or bone joints, neuroinflammatory diseases and disorders, or neurodegenerative diseases and disorders) includes co-administration of a compound of the disclosure (a compound of Formula (I), or a compound listed in Table A, B, C, or D), a pharmaceutically acceptable salt thereof, or a formulation comprising a compound or salt disclosed herein in combination with another therapeutic agent. In some cases, the other therapeutic agent is an anti-inflammatory agent. Contemplated anti-inflammatory agents use for co-administration with the compounds of the disclosure include, e.g., mesalazine, naproxen, ibuprofen, diclofenac, celecoxib, sulindac, oxaprozin, piroxicam, indomethacin, meloxicam, fenoprofen, difunisal, etodolac, ketorolac tromethamine, meclofenamate, nabumetone, salsalate, or any combination of the foregoing.

In jurisdictions that forbid the patenting of methods that are practiced on the human body, the meaning of "administering" of a composition to a human subject or patient shall be restricted to prescribing a controlled substance that a human subject or patient will self-administer by any technique (e.g., orally, inhalation, topical application, injection, insertion, etc.). The broadest reasonable interpretation that is consistent with laws or regulations defining patentable subject matter is intended. In jurisdictions that do not forbid the patenting of methods that are practiced on the human body, the "administering" of compositions includes both methods practiced on the human body and also the foregoing activities.

### Synthesis of the Compounds of the Disclosure

The compounds of the disclosure can be synthesized by any method known in the art. For example, the compounds of the disclosure (compounds of Formula (I) and compounds listed in Tables A, B, C, and D) can be synthesized according to Schemes 1, 2, and 3.

Compounds in which A is an optionally substituted fused bicyclic heteroaryl group having 8-10 total ring atoms and 1, 2, or 3 heteroatoms selected from N, O, or S can be synthesized via, e.g., a Suzuki coupling, as shown in Scheme 1, below. 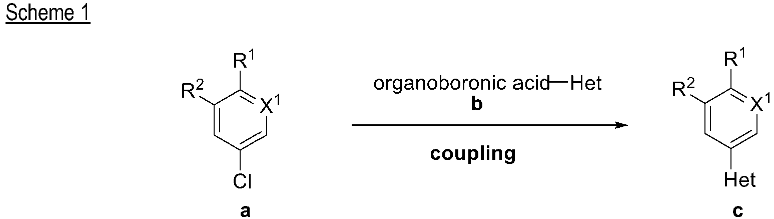 Coupling of a desired phenyl or pyridiyl moiety a with a desired fused bicyclic heteroaryl group (Het) that is conjugated to an organoboronic acid b results in a compound of the disclosure c.

Compounds of the disclosure in which A is 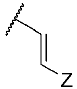 can be synthesized via, e.g., a Horner-Wadsworth-Emmons reaction, as shown in Scheme 2, below. 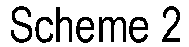 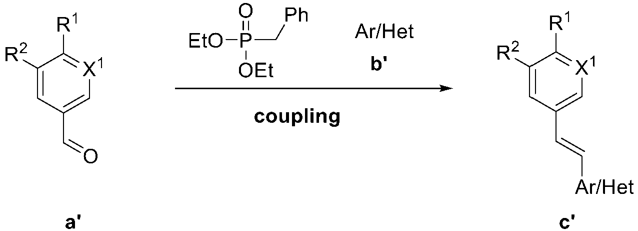 Reaction of a desired aldehyde a' with a desired aryl or heteroaryl group ("Ar/Het") b' and diethyl benzoylphosphonate results in a compound of the disclosure c'.

Compounds of the disclosure in which Y is O, S, S=O, or SO₂ can be synthesized, as shown in Scheme 3, below. 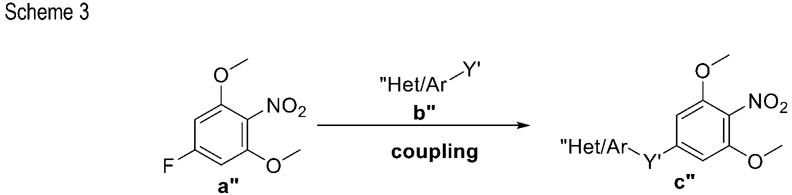 Coupling of 1,3,5-trifluoro-2-nitrobenzene a" with a desired aryl or heteroaryl group ("Ar/Het") conjugated to a OH or SNa (Y") b" in a nucleophilic aromatic substitution reaction results in compound c", which can be derivatized at the NO₂ group, the OMe groups, and/or the Y' group using standard methods known in the art to arrive at a compound of the disclosure.

### EXAMPLES

The following examples are provided for illustration and are not intended to limit the scope of the invention.

### Example 1 - Preparation of 5-aryl-2-isopropylbenzene-1,3-diol

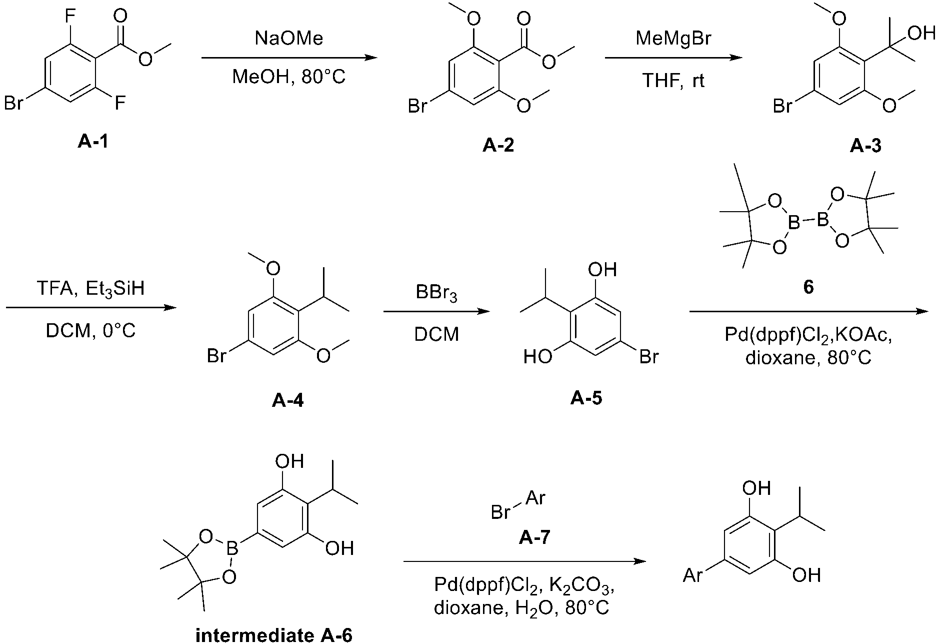

To a solution of methyl 4-bromo-2,6-difluorobenzoate (**A-1,** 20.0 g, 0.08 mol) in MeOH (200 mL) was added NaOCH₃ (13.0 g, 0.24 mol). The reaction mixture was stirred at 80°C for 12 h. The LCMS showed the desired MS was detected. The solvent was removed by vacuum. The residue was purified by flash chromatography (eluting with PE/EtOAc from 100/0 to 90/10 in 30 min) to afford compound **A-2.** LCMS (ESI): calcd for C₁₀H₁₁BrO₄ [M+H]⁺ m/z 275.0, found 275.1.

To a solution of methyl 4-bromo-2,6-dimethoxybenzoate (16.0 g, 0.058 mol) in THF at 0°C was added MeMgBr (11.7 mL, 1M in hexane). The reaction mixture was stirred at 25°C for 2 h. The LCMS showed the desired MS was detected. The residue was quenched by NH₄Cl aq and removed by vacuum. The residue was extracted with EtOAc (1000 mL), the organic layer was washed with brine, dried over Na₂SO₄, filtered and concentrated to furnish a crude product which was purified by flash chromatography (eluting with PE/EtOAc from 100/0 to 80/20 in 30 mins) to afford compound **A-3.** LCMS (ESI): calcd for C₁₁H₁₅BrO₃ [M+H] ⁺ m/z 274.0, found 257.1.

To a solution of 2-(4-bromo-2,6-dimethoxyphenyl)propan-2-ol (13.0 g, 0.047 mol) in DCM (300 mL) at 0°C was added TFA (13.8 g, 0.141 mol) and Triethylsilane (16.4 g, 0.141 mol). The reaction mixture was stirred at 0°C for 2 h. The LCMS showed the desired MS was detected. The solvent was removed by vacuum, and the crude residue was purified by flash chromatography (eluting with PE/EtOAc from 100/0 to 10/90 in 30 mins) to afford compound **A-4.** ¹H NMR (400 MHz, DMSO-*d₆*, ppm) 6.78 (s, 2H), 3.76 (s, 6H), 3.47 (hept, *J* = 7.2 Hz, 1H), 1.18 (d, *J = 7.2* Hz, 6H).

To a solution of 5-bromo-2-isopropyl-1,3-dimethoxybenzene (2.0 g, 0.00775 mol) in DCM (40 mL) was added BBr₃ (62.0 ml, 1 M in DCM) dropwise at 0°C. The reaction mixture was stirred at 25°C for 12 h. The LCMS showed the desired MS was detected. The residue was quenched by water. The mixture was concentrated and crude product was purified by flash chromatography (eluting with PE/EtOAc from 100/0 to 80/20 in 30 mins) to compound **A-5.** ¹H NMR (400 MHz, DMSO-*d₆*, ppm) 9.44 (s, 2H), 6.41 (s, 2H), 3.42-3.35 (m, 1H), 1.19 (d, *J* = 7.2 Hz, 6H).

To a solution of 5-bromo-2-isopropylbenzene-1,3-diol (1.5 g, 0.0065 mol) in 1,4-dioxane / H₂O =10:1 (22 mL) was added 4,4,4',4',5,5,5',5'-octamethyl-2,2'-bi(1,3,2-dioxaborolane) (2.0 g, 0.0078 mol), Pd(dppf)Cl₂ (475.0 mg, 0.00065 mmol) and K₂CO₃ (1.8 g, 0.013 mol). The reaction mixture was stirred at 80°C for 16 h under nitrogen. The LCMS showed the desired MS was detected. The solvent was removed by vacuum. The residue was extracted with EtOAc (200 mL), the organic layer was washed with brine, dried over Na₂SO₄, filtered and concentrated to get crude product which was purified by flash chromatography (eluting with PE/EtOAc from 100/0 to 40/60 in 30 mins) to afford compound **A-6.** LCMS (ESI): calcd for C₁₅H₂₃BO₄ [M+H]⁺ m/z 279.2, found 279.1

To a solution of 2-isopropyl-5-(4,4,5,5-tetramethyl-1,3,2-dioxaborolan-2-yl)benzene-1,3-diol (100.0 mg, 0.36 mmol) in 1,4-dioxane / H₂O (8 mL/2 mL) was added the corresponding bromo-derivative (0.36 mmol), Pd(dppf)Cl₂ (26.3 mg, 0.0359 mmol) and K₂CO₃ (99.2 mg, 0.719 mmol). The reaction mixture was stirred at 80°C for 16 h under nitrogen. The LCMS showed the desired MS was detected. The solvent was removed by vacuum. The residue was extracted with EtOAc (200 mL), the organic layer was washed with brine, dried over Na₂SO₄, filtered and concentrated to get crude product, which was purified by flash chromatography (eluting with PE/EtOAc from 100/0 to 40/60 in 30 mins) and Prep-HPLC (columns: Gemini 5 um C18 150 x 21.2 mm, mobile phase: ACN-H₂O (0.1% FA), gradient: 20-95, 10.0 min) to afford 2-isopropylbenzene-1,3-diol derivatives (e.g., compound nos. 1-51.

| Compound No. | Structure | LCMS (ESI): [M + H]⁺ | ¹H NMR |
|---|---|---|---|
| 1 | 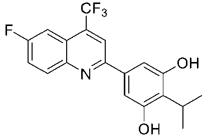 | 366.1 | ¹H NMR (400 MHz, DMSO-*d₆*, ppm) 9.39 (s, 2H), 8.33-8.21 (m, 1H), 8.16 (s, 1H), 7.88 (t, *J* = 7.6 Hz, 1H), 7.75 (d, *J* = 9.2 Hz, 1H), 7.20 (s, 2H), 3.67-3.43 (m, 1H), 1.28 (d, *J* = 6.8 Hz, 6H). |
| 2 | 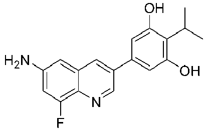 | 313.1 | ¹H NMR (400 MHz, DMSO-*d₆*, ppm) 9.27 (s, 2H), 8.60 (d, *J* = 2.0 Hz, 1H), 7.95 (s, 1H), 6.93 (dd, *J =* 13.2, 2.4 Hz, 1H), 6.67 (d, *J* = 2.4 Hz, 1H), 6.61 (s, 2H), 5.82 (s, 2H), 3.54-3.43 (m, 1H), 1.27 (d, *J =* 7.2 Hz, 6H). |
| 3 | 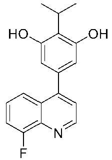 | 298.1 | ¹H NMR (400 MHz, DMSO-*d₆*, ppm) 9.36 (s, 2H), 8.94 (d, *J* = 4.4 Hz, 1H), 7.88-7.79 (m, 1H), 7.68-7.55 (m, 2H), 7.48 (d, *J* = 4.4 Hz, 1H), 6.42 (s, 2H), 3.60- 3.44 (m, 1H), 1.30 (d, *J* = 7.2 Hz, 6H). |
| 4 | 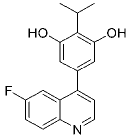 | 298.1 | ¹H NMR (400 MHz, DMSO-*d₆*, ppm) 9.37 (s, 2H), 8.89 (d, *J* = 4.4 Hz, 1H), 8.19-8.13 (m, 1H), 7.78-7.59 (m, 2H), 7.43 (d, *J* = 4.4 Hz, 1H), 6.44 (s, 2H), 3.61-3.47 (m, 1H), 1.31 (d, *J* = 7.2 Hz, 6H). |
| 5 | 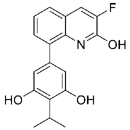 | 314.1 | ¹H NMR (400 MHz, DMSO-*d₆*, ppm) 10.22 (d, *J =* 5.2 Hz, 1H), 9.29 (s, 2H), 7.97 (d, *J* = 11.2 Hz, 1H), 7.68-7.54 (m, 1H), 7.51-7.14 (m, 2H), 6.33 (s, 2H), 3.59-3.41 (m, 1H), 1.30 (d, *J* = 7.2 Hz, 6H). |
| 6 | 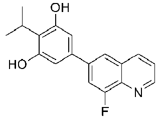 | 298.1 | ¹H NMR (400 MHz, DMSO-*d₆*, ppm) 9.29 (s, 2H), 8.93 (dd, *J* = 4.0, 1.6 Hz, 1H), 8.53-8.47 (m, 1H), 7.88 (d, *J* = 1.6 Hz, 1H), 7.75-7.59 (m, 2H), 6.65 (s, 2H), 3.55-3.43 (m, 1H), 1.28 (d, *J* = 6.8 Hz, 6H). |
| 7 | 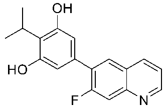 | 298.1 | ¹H NMR (400 MHz, DMSO-*d₆*, ppm) 9.26 (s, 2H), 8.91 (dd, *J* = 4.4, 1.6 Hz, 1H), 8.50-8.40 (m, 1H), 8.04 (d, *J* = 8.8 Hz, 1H), 7.83 (d, *J* = 12.0 Hz, 1H), 7.54 (dd, *J* = 8.0, 4.0 Hz, 1H), 6.55 (d, *J* = 1.6 Hz, 2H), 3.64-3.42 (m, 1H), 1.28 (d, *J* = 6.8 Hz, 6H). |
| 8 | 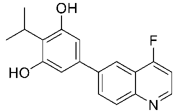 | 298.1 | ¹H NMR (400 MHz, DMSO-*d₆*, ppm) 9.28 (s, 2H), 8.89 (dd, *J* = 8.4, 4.8 Hz, 1H), 8.16 (d, *J* = 9.2 Hz, 1H), 8.10 (d, *J* = 1.6 Hz, 1H), 7.99 (dd, *J* = 8.8, 2.0 Hz, 1H), 7.47 (dd, *J* = 10.4, 5.2 Hz, 1H), 6.70 (s, 2H), 3.59-3.43 (m, 1H), 1.28 (d, *J* = 7.2 Hz, 6H). |
| 9 | 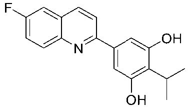 | 298.1 | ¹H NMR (400 MHz, DMSO-*d₆*, ppm) 9.29 (s, 2H), 8.40 (d, *J* = 8.4 Hz, 1H), 8.04 (dd, *J* = 9.2, 5.2 Hz, 1H), 7.86 (d, *J* = 8.8 Hz, 1H), 7.79 (dd, *J* = 9.2, 2.8 Hz, 1H), 7.69-7.64 (m, 1H), 7.14 (s, 2H), 3.56-3.44 (m, 1H), 1.28 (d, *J* = 6.8 Hz, 6H). |
| 10 | 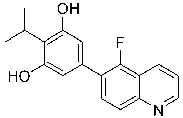 | 298.1 | ¹H NMR (400 MHz, DMSO-*d₆*, ppm) 9.28 (s, 2H), 8.98 (dd, *J =* 4.4, 1.6 Hz, 1H), 8.54 (d, *J* = 8.0 Hz, 1H), 7.94 (d, *J* = 8.8 Hz, 1H), 7.79 (t, *J* = 8.8 Hz, 1H), 7.69-7.64 (m, 1H), 6.60 (s, 2H), 3.55-3.46 (m, 1H), 1.29 (d, *J* = 7.2 Hz, 6H). |
| 11 | 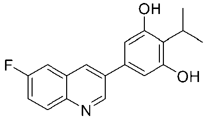 | 298.1 | ¹H NMR (400 MHz, DMSO-*d₆*, ppm) 9.40 (s, 2H), 9.01 (d, *J =* 2.0 Hz, 1H), 8.37 (d, *J* = 2.0 Hz, 1H), 8.09 (dd, *J* = 9.2, 5.6 Hz, 1H), 7.88 (dd, *J* = 9.6, 2.8 Hz, 1H), 7.70-7.62 (m, 1H), 6.68 (s, 2H), 3.55-3.45 (m, 1H), 1.28 (d, *J* = 7.2 Hz, 6H). |
| 12 | 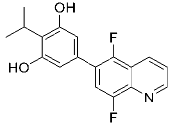 | 316.1 | ¹H NMR (400 MHz, DMSO-*d₆*, ppm) 9.31 (s, 2H), 9.03 (dd, *J* = 4.0, 1.2 Hz, 1H), 8.58 (d, *J* = 8.8 Hz, 1H), 7.76 (dd, *J* = 8.8, 4.4 Hz, 1H), 7.65 (dd, *J =* 11.6, 6.8 Hz, 1H), 6.59 (d, *J* = 1.2 Hz, 2H), 3.59-3.42 (m, 1H), 1.28 (d, *J* = 7.2 Hz, 6H). |
| 13 | 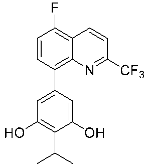 | 366.1 | ¹H NMR (400 MHz, DMSO-*d₆*, ppm) 9.10 (s, 2H), 8.86 (d, *J* = 8.8 Hz, 1H), 8.09 (d, *J* = 8.4 Hz, 1H), 7.84-7.78 (m, 1H), 7.77-7.58 (m, 1H), 6.55 (s, 2H), 3.67-3.41 (m, 1H), 1.30 (d, *J* = 7.1 Hz, 6H). |
| 14 | 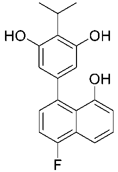 | 314.1 | ¹H NMR (400 MHz, DMSO-*d₆*, ppm) 11.93 (s, 1H), 9.23 (s, 2H), 8.02 (s, 1H), 7.86 (d, *J* = 6.8 Hz, 1H), 7.64 (d, *J* = 12.4 Hz, 1H), 6.61 (s, 2H), 6.11 (d,*J* = 7.2 Hz, 1H), 3.51-3.41 (m, 1H), 1.26 (d, *J* = 6.8 Hz, 6H). |
| 15 | 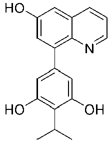 | 296.1 | ¹H NMR (400 MHz, DMSO-*d₆*, ppm) 10.08 (brs, 1H), 9.00 (s, 2H), 8.63 (dd, *J* = 4.1, 1.7 Hz, 1H), 8.26-8.08 (m, 1H), 7.42-7.37 (m, 1H), 7.18 (d, *J* = 2.8 Hz, 1H), 7.10 (d, *J* = 2.8 Hz, 1H), 6.50 (s, 2H), 3.67-3.48 (m, 1H), 1.30 (d, *J* = 7.2 Hz, 6H). |
| 16 | 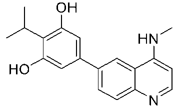 | 309.2 | ¹H NMR (400 MHz, DMSO-*d₆*, ppm) 9.17 (brs, 1H), 8.40 (d, *J* = 5.6 Hz, 1H), 8.27 (d, *J* = 1.6 Hz, 1H), 8.20 (s, 1H), 7.82 (d, *J* = 8.8 Hz, 1H), 7.74-7.67 (m, 1H), 7.66-7.57 (m, 1H), 6.60 (s, 2H), 6.42 (d, *J =* 5.6 Hz, 1H), 3.53-3.43 (m, 1H), 2.91 (d, *J* = 4.8 Hz, 3H), 1.28 (d, *J* = 7.2 Hz, 6H). |
| 17 | 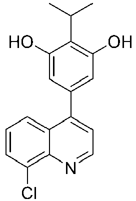 | 314.1 | ¹H NMR (400 MHz, DMSO-*d₆*, ppm) 9.04 (s, 2H), 8.84 (d, *J* = 4.8 Hz, 1H), 8.21 (dd, *J* = 8.0, 1.6 Hz, 1H), 7.84-7.68 (m, 3H), 6.49 (s, 2H), 3.69-3.41 (m, 1H), 1.30 (d, *J* = 7.2 Hz, 6H). |
| 18 | 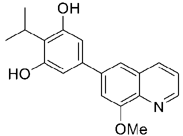 | 310.1 | ¹H NMR (400 MHz, DMSO-*d₆*, ppm) 9.22 (s, 2H), 8.81 (d, *J* = 2.8 Hz, 1H), 8.36 (dd, *J* = 8.4, 1.6 Hz, 1H), 7.57-7.52 (m, 2H), 7.26 (d, *J* = 1.2 Hz, 1H), 6.67 (s, 2H), 4.02 (s, 3H), 3.53-3.43 (m, 1H), 1.27 (d, *J* = 7.2 Hz, 6H). |
| 19 | 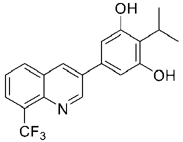 | 348.1 | ¹H NMR (400 MHz, DMSO-*d₆*, ppm) 9.38 (s, 2H), 9.18 (d, *J* = 2.4 Hz, 1H), 8.53 (d, *J* = 2.4 Hz, 1H), 8.39 (d, *J* = 8.4 Hz, 1H), 8.17 (d, *J* = 7.2 Hz, 1H), 7.77 (t, *J* = 8.0 Hz, 1H), 6.69 (s, 2H), 3.55-3.46 (m, 1H), 1.29 (d, *J* = 7.2 Hz, 6H). |
| 20 | 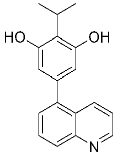 | 280.1 | ¹H NMR (400 MHz, DMSO-*d₆*, ppm) 9.22 (s, 2H), 8.91 (dd, *J* = 4.0, 1.6 Hz, 1H), 8.34 (d, *J* = 8.0 Hz, 1H), 8.00 (d, *J* = 8.4 Hz, 1H), 7.82-7.75 (m, 1H), 7.79-7.45 (dd, *J* = 8.8, 4.4 Hz, 1H), 7.79-7.45 (m, 1H), 6.36 (s, 2H), 3.58-3.44 (m, 1H), 1.31 (d, *J =* 7.2 Hz, 6H). |
| 21 | 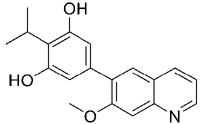 | 310.1 | ¹H NMR (400 MHz, DMSO-*d₆*, ppm) 9.06 (s, 2H), 8.82-8.75 (m, 1H), 8.28 (d, *J* = 8.0 Hz, 1H), 7.76 (s, 1H), 7.46 (s, 1H), 7.38 (dd, *J* = 8.0, 4.4 Hz, 1H), 6.46 (s, 2H), 3.92 (s, 3H), 3.54-3.42 (m, 1H), 1.28 (d, *J* = 7.2 Hz, 6H). |
| 22 | 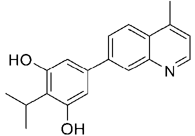 | 294.1 | ¹H NMR (400 MHz, DMSO-*d₆*, ppm) 9.27 (s, 2H), 8.76 (d, *J* = 4.0 Hz, 1H), 8.24-8.05 (m, 1H), 8.03 (d, *J* = 1.6 Hz, 1H), 7.75 (dd, *J* = 8.8, 2.0 Hz, 1H), 7.36 (dd, *J* = 4.0, 0.4 Hz, 1H), 6.70 (s, 1H), 3.54-3.44 (m, 1H), 2.70 (s, 1H), 1.28 (d, *J* = 7.2 Hz, 6H). |
| 23 | 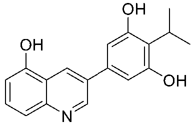 | 296.1 | ¹H NMR (400 MHz, DMSO-*d₆*, ppm) 10.56 (s, 1H), 9.28 (s, 2H), 8.99 (d, *J* = 2.4 Hz, 1H), 8.50 (d, *J =* 2.0 Hz, 1H), 7.65-7.30 (m, 2H), 6.95 (dd, *J* = 7.6, 1.2 Hz, 1H), 6.70 (s, 2H), 3.61-3.42 (m, 1H), 1.28 (d, *J* = 6.8 Hz, 6H). |
| 24 | 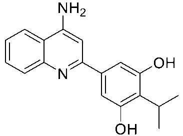 | 295.1 | ¹H NMR (400 MHz, DMSO-*d₆*, ppm) 9.18 (s, 1H), 8.16 (s, 1H), 8.12 (d, *J* = 7.6 Hz, 1H), 7.75 (d, *J* = 7.6 Hz, 1H), 7.64-7.55 (m, 1H), 7.42-7.28 (m, 1H), 7.02 (s, 2H), 6.90 (s, 1H), 6.86 (s, 2H), 3.55-3.45 (m, 1H), 1.27 (d, *J* = 7.2 Hz, 6H). |
| 25 | 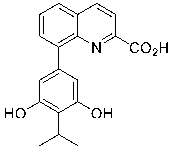 | 324.1 | ¹H NMR (400 MHz, DMSO-*d₆*, ppm) 9.04 (s, 2H), 8.57 (d, *J* = 8.8 Hz, 1H), 8.10 (d, *J* = 8.8 Hz, 1H), 8.02 (dd, *J* = 7.2, 2.4 Hz, 1H), 7.79-7.66 (m, 2H), 6.59 (s, 2H), 3.55-3.48 (m, 1H), 1.31 (d, *J* = 6.8 Hz, 6H). |
| 26 | 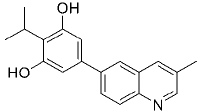 | 294.1 | ¹H NMR (400 MHz, DMSO-*d₆*, ppm) 9.23 (d, *J* = 2.4 Hz, 2H), 8.74 (d, *J* = 2.4 Hz, 1H), 8.17 (s, 1H), 8.02 (d, *J* = 8.8 Hz, 1H), 7.91 (d, *J* = 1.6 Hz, 1H), 7.78 (dd, *J* = 8.8, 2.4 Hz, 1H), 6.64 (s, 2H), 3.55-3.43 (m, 1H), 1.28 (d, *J* = 7.2 Hz, 6H). |
| 27 | 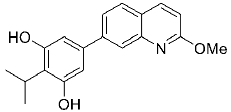 | 310.1 | ¹H NMR (400 MHz, DMSO-*d₆*, ppm) 9.21 (s, 2H), 8.23 (d, *J* = 8.8 Hz, 1H), 7.92 (d, *J* = 8.4 Hz, 1H), 7.81 (s, 1H), 7.55 (dd, *J* = 8.0, 1.6 Hz, 1H), 6.99 (d, *J* = 8.8 Hz, 1H), 6.68 (s, 2H), 4.01 (s, 3H), 3.54-3.43 (m, 1H), 1.28 (d, *J* = 7.2 Hz, 6H). |
| 28 | 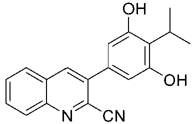 | 305.1 | ¹H NMR (400 MHz, DMSO-*d₆*, ppm) 9.45 (s, 2H), 8.59 (s, 1H), 8.16 (d, *J* = 8.8 Hz, 2H), 7.95-7.91 (m, 1H), 7.86-7.80 (m, 1H), 6.56 (s, 2H), 3.58-3.46 (m, 1H), 1.30 (d, *J* = 7.2 Hz, 6H). |
| 29 | 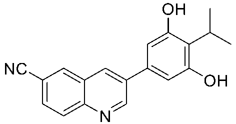 | 305.1 | ¹H NMR (400 MHz, DMSO-*d₆*, ppm) 9.39 (s, 2H), 9.20 (d, *J* = 2.4 Hz, 1H), 8.76 (s, 1H), 8.50 (d, *J =* 1.6 Hz, 1H), 8.19 (d, *J* = 8.8 Hz, 1H), 8.04 (dd, *J* = 8.8, 1.6 Hz, 1H), 6.69 (s, 2H), 3.54-3.47 (m, 1H), 1.28 (d, *J* = 7.2 Hz, 6H). |
| 30 | 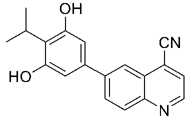 | 305.1 | ¹H NMR (400 MHz, DMSO-*d₆*, ppm) 9.38 (s, 2H), 9.09 (d, *J* = 4.4 Hz, 1H), 8.26 (d, *J* = 8.8 Hz, 1H), 8.16 (d, *J* = 4.4 Hz, 1H), 8.13 (d, *J* = 1.6 Hz, 1H), 8.09 (dd, *J* = 8.8, 2.0 Hz, 1H), 6.76 (s, 2H), 3.55 - 3.44 (m, 1H), 1.28 (d, *J* = 7.2 Hz, 6H). |
| 31 | 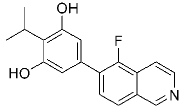 | 298.1 | ¹H NMR (400 MHz, DMSO-*d₆*, ppm) 9.40 (s, 1H), 9.30 (s, 2H), 8.63 (d, *J* = 6.0 Hz, 1H), 8.04 (d, *J =* 8.4 Hz, 1H), 7.95 (d, *J* = 5.6 Hz, 1H), 7.73-7.69 (m, 1H), 6.60 (d, *J* = 0.8 Hz, 2H), 3.54-3.47 (m, 1H), 1.28 (d, *J* = 7.2 Hz, 6H). |
| 32 | 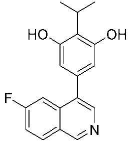 | 298.1 | ¹H NMR (400 MHz, DMSO-*d₆*, ppm) 9.31 (s, 1H), 9.29 (s, 2H), 8.39 (s, 1H), 8.34 (dd, *J* = 9.2, 6.0 Hz, 1H), 7.68-7.60 (m, 2H), 6.42 (s, 2H), 3.58-3.47 (m, 1H), 1.31 (d, *J* = 7.2 Hz, 6H). |
| 33 | 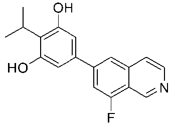 | 298.1 | ¹H NMR (400 MHz, DMSO-*d₆*, ppm) 9.44 (s, 1H), 9.34 (s, 2H), 8.62 (d, *J* = 6.0 Hz, 1H), 7.97 (d, *J =* 5.6 Hz, 1H), 7.88 (s, 1H), 7.56 (dd, *J* = 12.0, 1.2 Hz, 1H), 6.66 (s, 2H), 3.52-3.45 (m, 1H), 1.27 (d, *J* = 7.2 Hz, 6H). |
| 34 | 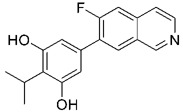 | 298.1 | ¹H NMR (400 MHz, DMSO-*d₆*, ppm) 9.36 (s, 1H), 9.27 (s, 2H), 8.51 (d, *J* = 6.0 Hz, 1H), 8.19 (d, *J =* 8.0 Hz, 1H), 7.87-7.82 (m, 2H), 6.55 (d, *J* = 1.6 Hz, 2H), 3.58-3.43 (m, 1H), 1.28 (d, *J* = 7.2 Hz, 6H). |
| 35 | 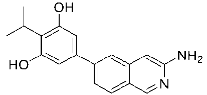 | 295.1 | ¹H NMR (400 MHz, DMSO-*d₆*, ppm) 9.19 (s, 2H), 8.79 (s, 1H), 7.82 (d, *J* = 8.4 Hz, 1H), 7.52 (s, 1H), 7.25 (d, *J* = 8.4 Hz, 1H), 6.63 (s, 1H), 6.60 (s, 2H), 5.91 (s, 2H), 3.56-3.41 (m, 1H), 1.27 (d, *J* = 7.2 Hz, 6H). |
| 36 | 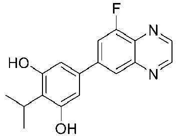 | 299.1 | ¹H NMR (400 MHz, DMSO-*d₆*, ppm) 9.35 (s, 2H), 9.05 (d, *J* = 2.0 Hz, 1H), 8.97 (d, *J* = 2.0 Hz, 1H), 7.95 (d, *J* = 1.2 Hz, 1H), 7.81 (dd, *J* = 11.6, 1.6 Hz, 1H), 6.70 (s, 2H), 3.55 - 3.42 (m, 1H), 1.28 (d, *J =* 7.2 Hz, 6H). |
| 37 | 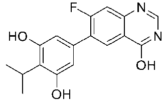 | 315.1 | ¹H NMR (400 MHz, DMSO-*d₆*, ppm) 12.35 (s, 1H), 9.25 (s, 2H), 8.14 (s, 1H), 8.10 (d, *J* = 8.4 Hz, 1H), 7.53 (d, *J* = 11.6 Hz, 1H), 6.54 (s, 2H), 3.54-3.42 (m, 1H), 1.27 (d, *J* = 6.8 Hz, 6H). |
| 38 | 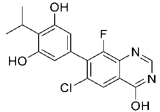 | 349.1 | ¹H NMR (400 MHz, DMSO-*d₆*, ppm) 12.58 (s, 1H), 9.28 (s, 2H), 8.19 (s, 1H), 7.99 (d, *J* = 1.6 Hz, 1H), 6.25 (s, 2H), 3.58-3.42 (m, 1H), 1.28 (d, *J* = 7.2 Hz, 6H). |
| 39 | 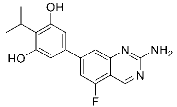 | 314.1 | ¹H NMR (400 MHz, DMSO-*d₆*, ppm) 9.30 (s, 2H), 9.22 (s, 1H), 7.25 (s, 1H), 7.12 (s, 2H), 7.04 (dd, *J* = 10.8, 1.0 Hz, 1H), 6.59 (s, 2H), 3.53-3.41 (m, 1H), 1.26 (d, *J* = 7.2 Hz, 6H). |
| 40 | 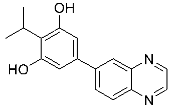 | 281.1 | ¹H NMR (400 MHz, DMSO-*d₆*, ppm) 9.32 (s, 2H), 8.96 (d, *J* = 2.0 Hz, 1H), 8.93 (d, *J* = 2.0 Hz, 1H), 8.16 (d, *J* = 8.8 Hz, 1H), 8.09 (d, *J* = 2.0 Hz, 1H), 7.99 (dd, *J* = 8.8, 2.0 Hz, 1H), 6.72 (s, 2H), 3.58-3.40 (m, 1H), 1.28 (d, *J* = 7.2 Hz, 6H). |
| 41 | 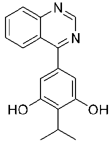 | 281.1 | ¹H NMR (400 MHz, DMSO-*d₆*, ppm) 9.42 (s, 2H), 9.28 (s, 1H), 8.25 (d, *J* = 8.4 Hz, 1H), 8.09-7.99 (m, 2H), 7.77-7.73 (m, 1H), 6.73 (s, 2H), 3.58-3.51 (m, 1H), 1.31 (d, *J* = 7.2 Hz, 6H). |
| 42 | 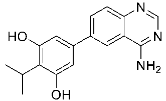 | 296.1 | ¹H NMR (400 MHz, DMSO-*d₆*, ppm) 9.18 (s, 2H), 8.38 (s, 1H), 8.33 (d, *J* = 2.0 Hz, 1H), 7.83 (dd, *J =* 8.8, 2.0 Hz, 2H), 7.70 (d, *J* = 8.4 Hz, 1H), 6.57 (s, 2H), 3.56-3.42 (m, 1H), 1.27 (d, *J* = 7.2 Hz, 6H). |
| 43 | 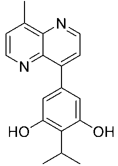 | 295.1 | ¹H NMR (400 MHz, DMSO-*d₆*, ppm) 9.16 (s, 2H), 8.98 (d, *J* = 4.4 Hz, 1H), 8.83 (d, *J* = 4.4 Hz, 1H), 7.66 (dd, *J* = 4.4, 0.8 Hz, 1H), 7.64 (d, *J* = 4.4 Hz, 1H), 6.63 (s, 2H), 3.59-3.46 (m, 1H), 2.79 (s, 3H), 1.30 (d, *J* = 7.2 Hz, 6H). |
| 44 | 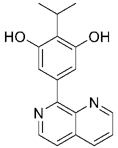 | 281.1 | ¹H NMR (400 MHz, DMSO-*d₆*, ppm) 9.08 (s, 2H), 9.05 (dd, *J* = 4.0, 2.0 Hz, 1H), 8.63 (d, *J* = 5.2 Hz, 1H), 8.46 (dd, *J* = 8.4, 1.6 Hz, 1H), 7.84 (d, *J =* 5.6 Hz, 1H), 7.78 (dd, *J* = 8.4, 4.0 Hz, 1H), 7.04 (s, 2H), 3.60-3.44 (m, 1H), 1.30 (d, *J* = 7.2 Hz, 6H). |
| 45 | 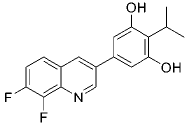 | 316.1 | ¹H NMR (400 MHz, DMSO-*d₆*, ppm) 9.37 (s, 2H), 9.12 (d, *J* = 2.0 Hz, 1H), 8.50 (t, *J* = 1.6 Hz, 1H), 8.02-7.97 (m, 1H), 7.82-7.73 (m, 1H), 6.67 (s, 2H), 3.55-3.44 (m, 1H), 1.28 (d, *J* = 6.8 Hz, 6H). |
| 46 | 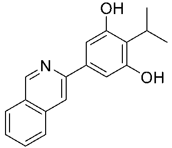 | 280.1 | ¹H NMR (400 MHz, DMSO-d6, ppm): 9.34 (s, 1H), 9.18 (s, 2H), 8.16-8.06 (m, 1H), 8.03 (d, *J* = 8.2 Hz, 2H), 7.77 (t, *J =* 7.2 Hz, 1H), 7.64 (t, *J =* 7.6 Hz, 1H), 7.13 (s, 2H), 3.55-3.42 (m, 1H), 1.28 (d, *J* = 7.1 Hz, 6H). |
| 47 | 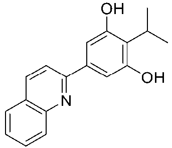 | 280.2 | ¹H NMR (400 MHz, DMSO-*d₆*, ppm) 9.28 (s, 2H), 8.40 (d, *J =* 8.8 Hz, 1H), 7.98 (t, *J* = 8.8 Hz, 2H), 7.82 (d, *J* = 8.8 Hz, 1H), 7.80-7.71 (m, 1H), 7.61-7.53 (m, 1H), 7.16 (s, 2H), 3.56-3.44 (m, 1H), 1.28 (d, *J =* 7.2 Hz, 6H). |
| 48 | 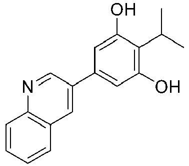 | 280.2 | ¹H NMR (400 MHz, DMSO-*d₆*, ppm) 9.34 (s, 2H), 9.03 (d, *J =* 2.4 Hz, 1H), 8.37 (d, *J* = 2.0 Hz, 1H), 8.09-7.98 (m, 2H), 7.81-7.71 (m, 1H), 7.68-7.59 (m, 1H), 6.68 (s, 2H), 3.55-3.44 (m, 1H), 1.28 (d, *J =* 8.0 Hz, 6H). |
| 49 | 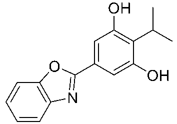 | 270.2 | ¹H NMR (400 MHz, DMSO-*d₆*, ppm) 9.58 (s, 2H), 7.78-7.73 (m, 2H), 7.42-7.36 (m, 2H), 7.15 (s, 2H), 3.51 (hept, J = 6.8 Hz, 1H), 1.28 (d, *J* = 7.2 Hz, 6H). |
| 50 | 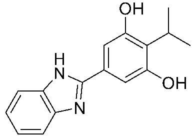 | 268.9 | ¹H NMR (400 MHz, DMSO-*d₆*, ppm): 9.87-9.55 (br, 2H), 7.80-7.63 (m, 2H), 7.53-7.30 (m, 2H), 7.00 (s, 2H), 3.58-3.52 (m, 1H), 1.29 (d, *J* = 7.2 Hz, 6H). |
| 51 | 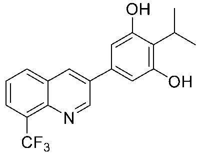 | 348.2 | ¹H NMR (400 MHz, DMSO-*d₆*, ppm) 9.38 (s, 2H), 9.18 (d, *J* = 2.4 Hz, 1H), 8.53 (d, *J* = 2.4 Hz, 1H), 8.39 (d, *J* = 7.2 Hz, 1H), 8.17 (d, *J* = 7.2 Hz, 1H), 7.77 (t, *J =* 7.6 Hz, 1H), 6.69 (s, 2H), 3.56-3.45 (m, 1H), 1.29 (d, *J* = 7.2 Hz, 6H). |

### Example 2 - Preparation of 5-aryl-2-isopropylpyridin-3-ol

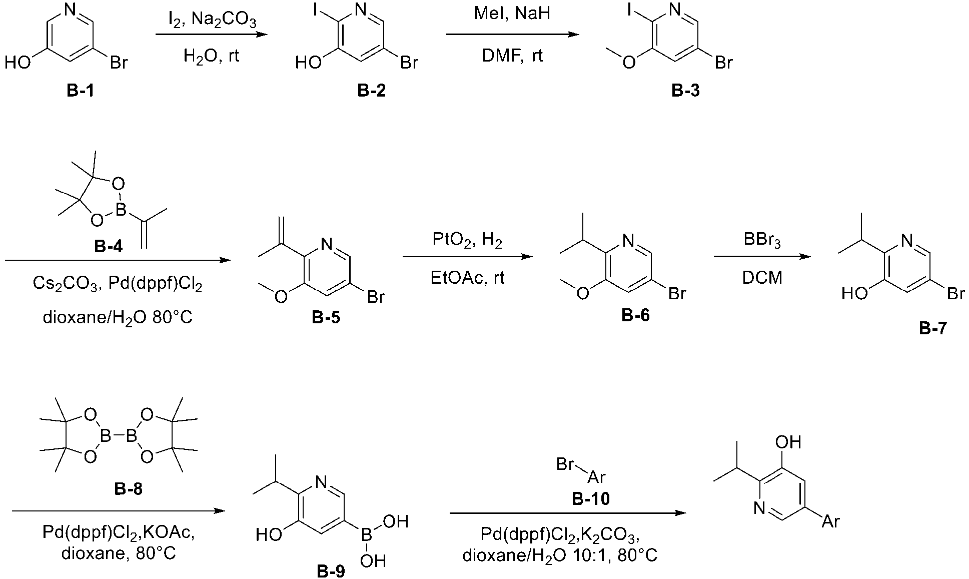

To a mixture of Na₂CO₃ (12.5 g, 118.0 mmol) and 3-bromo-5-hydroxypyridine (B-1, 10.3 g, 59.0 mmol) in H₂O (200 ml) was added iodine (15.7 g, 62.0 mmol) at 0°C). The reaction was stirred at room temperature for 1 h. The mixture was then poured slowly into 2M HCl (aq.), and the pH was adjusted to about 3. The product was collected by filtration followed by crystallization from EtOH/water to afford compound **B-2**. LCMS (ESI): calcd for C₅H₄BrINO [M+H]⁺ m/z 299.8, found 300.0.

To a stirred solution of 5-bromo-2-iodopyridin-3-ol (17.6 g, 58.90 mmol) in DMF (200.00 mL) was added NaH (2.8 g, 70.68 mmol) at 0 °C. After 30 min at 0°C. Mel (16.0 g, 115.2 mmol) was added at 0 °C. The resulting mixture was stirred at room temperature for 16 hours. After completion, the reaction mixture was diluted with NH₄Cl(aq) and water (100 mL each) at 0 °C, and diluted with EtOAc (150 mL). The separated aqueous layer was extracted with EtOAc (2 × 150 mL) and the combined organic layers were washed with water and saturated sodium chloride, dried over Na₂SO₄, and concentrated to give the crude residue which was purified with flash column chromatography (eluting with PE/EtOAc from 100/0 to 90/10 in 30 mins) to obtain compound **B-3**. LCMS (ESI): calcd for C₆H₅BrINO [M+H]⁺ m/z 313.8, found 313.8.

A solution of 5-bromo-2-iodo-3-methoxypyridine (7.8 g, 0.0248 mol), 4,4,5,5-tetramethyl-2-(prop-1-en-2-yl)-1,3,2-dioxaborolane **(B-4,** 4.6 g, 0.0272 mol), Cs₂CO₃ (16.2 g, 0.0496 mol) and Pd(dppf)Cl₂ (910.0 mg, 0.0012 mmol) in 200 mL dioxane:H₂O = 10:1 was charged with N₂ and stirred at 100°C for 16 h. The solvent was removed under reduced pressure. The residue was purified by silica gel chromatography (eluting with PE/DCM from 100/0 to 85/15 in 30 mins) to afford compound **B-5**. LCMS (ESI): calcd for C₉H₁₁BrNO [M+H]⁺ m/z 228.0, found 228.0.

A solution of 5-bromo-3-methoxy-2-(prop-1-en-2-yl)pyridine (3.4 g, 14.9 mmol) in 150 mL EtOAc was added PtO₂ (440.0 mg, 2.0 mmol). The reaction was stirred at rt for 16 h under H₂ (1 atm). The solvent was removed under reduced pressure. The residue was purified by silica gel chromatography (eluting with PE/EtOAc from 100/0 to 85/15 in 30 mins) to afford compound **B-6**. LCMS (ESI): calced for C₉H₁₃BrNO [M+H]⁺ m/z 230.0, found 230.1.

BBr₃ (10.9 g, 43.5 mmol) was added slowly to a solution of 5-bromo-2-isopropyl-3-methoxypyridine (2.0 g, 8.7 mmol) in 20 mL DCM. Then the mixture was stirred at room temperature for 16 h. The solution was added DCM (30 mL) and washed with aq. NaHCO₃ until the water phase pH 8- 9, and then extracted with DCM (20 mL x 3). The solvent was removed under reduced pressure. The residue was purified by silica gel chromatography (eluting with PE/EtOAc from 100/0 to 85/15 in 30 mins) to afford compound **B-7**. LCMS (ESI): calced for C₈H₁₁BrNO [M+H]⁺ m/z 216.0, found 216.0.

A solution of 5-bromo-2-isopropylpyridin-3-ol (40.0 mg, 0.18 mmol), 4,4,5,5-tetramethyl-2-(4,4,5,5-tetramethyl-1,3,2-dioxaborolan-2-yl)-1,3,2-dioxaborolane **(B-8,** 56.0 mg, 0.22 mmol), AcOK (54.5 mg, 0.55 mmol) and Pd(dppf)Cl₂ (13.5 mg, 0.018 mmol) in 2 mL dioxane was charged with N₂ and stirred at 80°C for 16 h to produce **B-9**. After the reaction was completed as monitored by LCMS, the corresponding bromo-derivative **(B-10,** 0.17 mmol), K₂CO₃ (23.0 mg, 0.17 mmol), Pd(dppf)Cl₂ (7.0 mg, 0.009 mmol) and 0.2 mL H₂O was added. The mixture was charged with N₂ and stirred at 80°C for 16 h. The solvent was removed under reduced pressure. The residue was purified by silica gel chromatography (for example, eluting with PE/EtOAc from 100/0 to 75/25 in 30 mins) to afford 5-aryl-2-isopropylpyridin-3-ol, such as compounds 52-102.

| Compound No. | Structure | LCMS (ESI): [M + H] ⁺ | ¹H NMR |
|---|---|---|---|
| 52 | 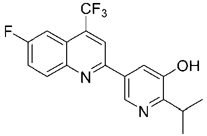 | 351.1 | ¹H NMR (400 MHz, DMSO-*d₆*, ppm) 10.14 (s, 1H), 8.90 (s, 1H), 8.49 (s, 1H), 8.35-8.28 (m, 1H), 8.03 (d, *J* = 1.6 Hz, 1H), 7.96-7.87 (m, 1H), 7.82-7.75 (m, 1H), 3.55-3.41 (m, 1H), 1.24 (d, *J* = 6.8 Hz, 6H). |
| 53 | 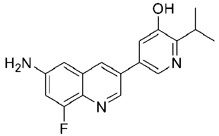 | 298.1 | ¹H NMR (400 MHz, DMSO-*d₆*, ppm) 10.05 (s, 1H), 8.73 (d, *J* = 2.4 Hz, 1H), 8.39 (d, *J* = 2.0 Hz, 1H), 8.18 (t, *J* = 2.0 Hz, 1H), 7.44 (d, *J* = 2.0 Hz, 1H), 6.97 (dd, *J* = 12.8, 2.0 Hz, 1H), 6.70 (d, *J =* 2.4 Hz, 1H), 5.88 (s, 2H), 3.46-3.38 (m, 1H), 1.22 (d, *J* = 6.8 Hz, 6H). |
| 54 | 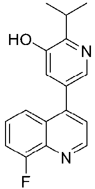 | 283.1 | ¹H NMR (400 MHz, DMSO-*d₆*, ppm) 9.01 (d, *J =* 4.4 Hz, 1H), 8.15 (d, *J* = 2.0 Hz, 1H), 7.84-7.54 (m, 4H), 7.28 (d, *J =* 2.0 Hz, 1H), 3.61-3.41 (m, 1H), 1.26 (d, *J* = 7.2 Hz, 6H). |
| 55 | 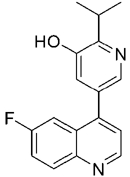 | 283.1 | ¹H NMR (400 MHz, DMSO-*d₆*, ppm) 10.17 (s, 1H), 8.95 (d, *J* = 4.4 Hz, 1H), 8.23-8.18 (m, 1H), 8.16 (d, *J* = 2.0 Hz, 1H), 7.79-7.72 (m, 1H), 7.58-7.53 (m, 2H), 7.29 (d, *J =* 1.6 Hz, 1H), 3.60-3.41 (m, 1H), 1.26 (d, *J* = 6.8 Hz, 6H). |
| 56 | 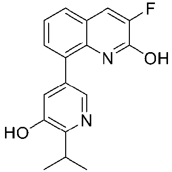 | 299.1 | ¹H NMR (400 MHz, DMSO-*d₆*, ppm) 11.18 (s, 1H), 9.89 (s, 1H), 7.99-7.95 (m, 2H), 7.70 (d, *J* = 6.8 Hz, 1H), 7.38 (d, *J* = 6.8 Hz, 1H), 7.31 (t, *J =* 7.6 Hz, 1H), 7.10 (d, *J =* 1.6 Hz, 1H), 3.46-3.38 (m, 1H), 1.24 (d, *J* = 6.8 Hz, 6H). |
| 57 | 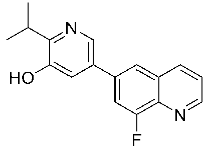 | 283.1 | ¹H NMR (400 MHz, DMSO-*d₆*, ppm) 10.01 (s, 1H), 8.96 (dd, *J* = 4.4, 1.6 Hz, 1H), 8.54-8.49 (m, 1H), 8.45 (d, *J* = 2.0 Hz, 2H), 8.08 (d, *J* = 1.6 Hz, 1H), 7.92 (dd, *J* = 12.0, 2.0 Hz, 1H), 7.68 (dd, *J* = 8.4, 4.0 Hz, 1H), 7.47 (d, *J* = 2.0 Hz, 1H), 3.48-3.39 (m, 1H), 1.22 (d, *J* = 6.8 Hz, 6H). |
| 58 | 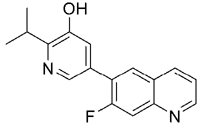 | 283.1 | ¹H NMR (400 MHz, DMSO-*d₆*, ppm) 10.00 (s, 1H), 8.95 (dd, *J* = 4.4, 1.6 Hz, 1H), 8.50-8.42 (m, 1H), 8.33-8.17 (m, 2H), 7.90 (d, *J* = 12.0 Hz, 1H), 7.57 (dd, *J =* 8.0, 4.0 Hz, 1H), 7.40 (t, *J* = 2.0 Hz, 1H), 3.48-3.38 (m, 1H), 1.23 (d, *J* = 6.8 Hz, 6H). |
| 59 | 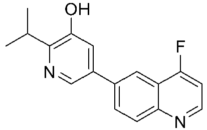 | 283.1 | ¹H NMR (400 MHz, DMSO-*d₆*, ppm) 9.99 (s, 1H), 8.96-8.90 (m, 1H), 8.47 (d, *J* = 2.0 Hz, 1H), 8.27 (d, *J* = 2.0 Hz, 1H), 8.23-8.10 (m, 2H), 7.55-7.45 (m, 2H), 3.50-3.34 (m, 1H), 1.23 (d, *J* = 6.8 Hz, 6H). |
| 60 | 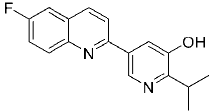 | 283.1 | ¹H NMR (400 MHz, DMSO-*d₆*, ppm) 10.05 (s, 1H), 8.80 (d, *J* = 1.6 Hz, 1H), 8.46 (d, *J* = 8.8 Hz, 1H), 8.17 (d, *J* = 8.8 Hz, 1H), 8.13-8.08 (m, 1H), 8.01 (d, *J* = 2.0 Hz, 1H), 7.83 (dd, *J* = 9.2, 2.8 Hz, 1H), 7.75-7.65 (m, 1H), 3.43 (m, 1H), 1.23 (d, *J* = 6.8 Hz, 6H). |
| 61 | 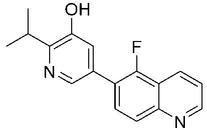 | 283.1 | ¹H NMR (400 MHz, DMSO-*d₆*, ppm) 10.07 (s, 1H), 9.05-8.95 (m, 1H), 8.62-8.55 (m, 1H), 8.30 (s, 1H), 7.95 (m, 2H), 7.69 (dd, *J* = 8.8, 4.0 Hz, 1H), 7.44 (s, 1H), 3.48-3.40 (m, 1H), 1.23 (d, *J* = 6.8 Hz, 6H). |
| 62 | 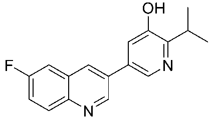 | 283.1 | ¹H NMR (400 MHz, DMSO-*d₆*, ppm) 10.09 (s, 1H), 9.17 (d, *J =* 2.4 Hz, 1H), 8.59 (d, *J* = 2.0 Hz, 1H), 8.46 (d, *J* = 2.0 Hz, 1H), 8.13 (dd, *J* = 9.2, 5.6 Hz, 1H), 7.87 (dd, *J* = 9.6, 2.8 Hz, 1H), 7.75-7.67 (m, 1H), 7.49 (d, *J* = 1.6 Hz, 1H), 3.49-3.40 (m, 1H), 1.23 (d, *J* = 6.8 Hz, 6H). |
| 63 | 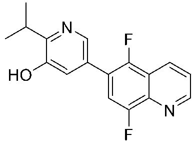 | 301.1 | ¹H NMR (400 MHz, DMSO-*d₆*, ppm) 10.06 (s, 1H), 9.06 (dd, *J* = 4.0, 1.6 Hz, 1H), 8.61 (d, *J* = 8.4 Hz, 1H), 8.31 (s, 1H), 7.88 (dd, *J* = 11.2, 6.8 Hz, 1H), 7.79 (dd, *J* = 8.4, 4.0 Hz, 1H), 7.45 (s, 1H), 3.48-3.40(m, 1H), 1.23 (d, *J* = 6.8 Hz, 6H). |
| 64 | 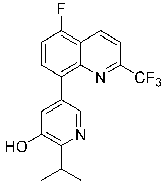 | 351.1 | ¹H NMR (400 MHz, DMSO-*d₆*, ppm) 10.06 (brs, 1H), 8.90 (d, *J* = 8.8 Hz, 1H), 8.26 (d, *J* = 1.6 Hz, 1H), 8.14 (d, *J* = 8.8 Hz, 1H), 7.99 (dd, *J* = 8.0, 6.0 Hz, 1H), 7.79-7.71 (m, 1H), 7.47 (d, *J* = 1.6 Hz, 1H), 3.48-3.40 (m, 1H), 1.25 (d, *J* = 6.8 Hz, 6H). |
| 65 | 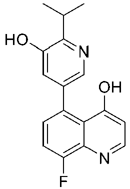 | 299.1 | ¹H NMR (400 MHz, DMSO-*d₆*, ppm) 12.02 (s, 1H), 10.40 (brs, 1H), 8.44 (s, 1H), 8.11 (s, 1H), 8.00 (d, *J =* 12.4 Hz, 1H), 7.91 (d, *J* = 7.6 Hz, 1H), 7.60 (s, 1H), 6.16 (d, *J* = 7.2 Hz, 1H), 3.48-3.44 (m, 1H), 1.25 (d, *J* = 6.8 Hz, 6H). |
| 66 | 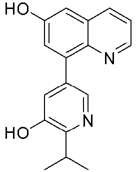 | 281.1 | ¹H NMR (400 MHz, DMSO-*d₆*, ppm) 10.17 (s, 1H), 9.76 (s, 1H), 8.66 (dd, *J* = 4.0, 1.6 Hz, 1H), 8.25-8.15 (m, 2H), 7.47-7.40 (m, 2H), 7.30 (d, *J* = 2.4 Hz, 1H), 7.19 (d, *J =* 2.8 Hz, 1H), 3.48-3.40 (m, 1H), 1.24 (d, *J* = 6.8 Hz, 6H). |
| 67 | 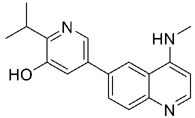 | 294.2 | ¹H NMR (400 MHz, DMSO-*d₆*, ppm) 8.46 (s, 1H), 8.44-8.40 (m, 2H), 8.19 (s, 1H), 7.86 (s, 2H), 7.61 (s, 1H), 7.45 (d, *J* = 2.0 Hz, 1H), 6.44 (d, *J =* 5.2 Hz, 1H), 3.48-3.40 (m, 1H), 2.93 (d, *J* = 4.4 Hz, 3H), 1.22 (d, *J =* 6.8 Hz, 6H). |
| 68 | 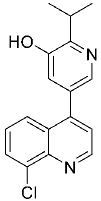 | 299.1 | ¹H NMR (400 MHz, DMSO-*d₆*, ppm) 9.82 (s, 1H), 8.86 (d, *J* = 4.4 Hz, 1H), 8.28 (dd, *J =* 8.4, 1.6 Hz, 1H), 8.19 (d, *J* = 2.0 Hz, 1H), 7.94-7.82 (m, 3H), 7.41 (d, *J =* 2.0 Hz, 1H), 3.44 (s, 1H), 1.24 (d, *J* = 6.8 Hz, 6H). |
| 69 | 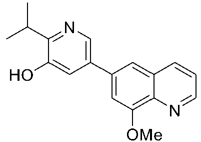 | 295.1 | ¹H NMR (400 MHz, DMSO-*d₆*, ppm) 9.96 (s, 1H), 8.84 (dd, *J* = 4.0, 1.6 Hz, 1H), 8.46 (d, *J* = 2.0 Hz, 1H), 8.38 (dd, *J* = 8.4, 1.6 Hz, 1H), 7.73 (d, *J* = 1.6 Hz, 1H), 7.58 (dd, *J* = 8.4, 4.0 Hz, 1H), 7.48 (d, *J* = 2.0 Hz, 1H), 7.40 (d, *J* = 1.6 Hz, 1H), 4.06 (s, 3H), 3.49-3.40 (m, 1H), 1.23 (d, *J* = 6.8 Hz, 6H). |
| 70 | 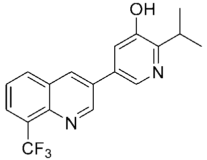 | 333.1 | ¹H NMR (400 MHz, DMSO-*d₆*, ppm) 10.13 (s, 1H), 9.36 (d, *J* = 2.4 Hz, 1H), 8.76 (d, *J* = 2.0 Hz, 1H), 8.51 (d, *J* = 1.6 Hz, 1H), 8.39 (d, *J* = 8.0 Hz, 1H), 8.21 (d, *J* = 7.2 Hz, 1H), 7.80 (t, *J =* 7.6 Hz, 1H), 7.53 (d, *J =* 1.6 Hz, 1H), 3.48-3.40 (m, 1H), 1.23 (d, *J* = 6.8 Hz, 6H). |
| 71 | 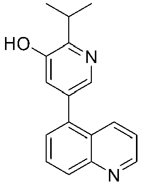 | 265.1 | ¹H NMR (400 MHz, DMSO-*d₆*, ppm) 9.32 (s, 2H), 8.96 (d, *J* = 1.6 Hz, 1H), 8.93 (d, *J =* 2.0 Hz, 1H), 8.16 (d, *J* = 8.8 Hz, 1H), 8.09 (d, *J* = 2.0 Hz, 1H), 7.99 (dd, *J =* 8.8, 2.0 Hz, 1H), 6.72 (s, 2H), 3.48-3.40 (m, 1H), 1.28 (d, *J* = 7.1 Hz, 6H). |
| 72 | 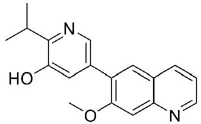 | 295.1 | ¹H NMR (400 MHz, DMSO-*d₆*, ppm) 9.81 (s, 1H), 8.84 (dd, *J* = 4.4, 1.6 Hz, 1H), 8.30 (dd, *J =* 8.0, 1.6 Hz, 1H), 8.16 (d, *J* = 2.0 Hz, 1H), 7.92 (s, 1H), 7.52 (s, 1H), 7.41 (dd, *J* = 8.0, 4.4 Hz, 1H), 7.34 (d, *J =* 2.0 Hz, 1H), 3.95 (s, 3H), 3.48-3.40 (m, 1H), 1.22 (d, *J* = 6.8 Hz, 6H). |
| 73 | 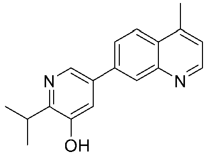 | 279.1 | ¹H NMR (400 MHz, DMSO-*d₆*, ppm) 10.02 (s, 1H), 8.79 (d, *J =* 4.4 Hz, 1H), 8.46 (d, *J* = 2.0 Hz, 1H), 8.26-8.16 (m, 2H), 7.91 (dd, *J* = 8.8, 2.0 Hz, 1H), 7.50 (d, *J* = 2.0 Hz, 1H), 7.40 (dd, *J* = 4.4, 0.8 Hz, 1H), 3.49-3.40 (m, 1H), 2.72 (s, 3H), 1.23 (d, *J* = 6.8 Hz, 6H). |
| 74 | 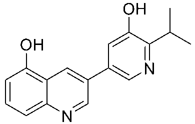 | 281.1 | ¹H NMR (400 MHz, DMSO-*d₆*, ppm) 10.71 (s, 1H), 10.08 (s, 1H), 9.14 (d, *J* = 2.0 Hz, 1H), 8.61 (d, *J* = 2.0 Hz, 1H), 8.45 (d, 69*J* = 1.6 Hz, 1H), 7.62-7.55 (m, 1H), 7.53-7.46 (m, 2H), 6.98 (d, *J* = 7.2 Hz, 1H), 3.48-3.40 (m, 1H), 1.23 (d, *J* = 6.8 Hz, 6H). |
| 75 | 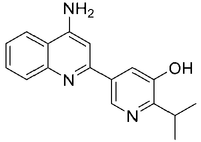 | 280.1 | ¹H NMR (400 MHz, DMSO-*d₆*, ppm) 9.90 (s, 1H), 8.58 (d, *J* = 1.6 Hz, 1H), 8.23-8.08 (m, 2H), 7.88 (d, *J* = 1.6 Hz, 1H), 7.80 (d, *J* = 8.4 Hz, 1H), 7.62 (t, *J* = 8.0 Hz, 1H), 7.38 (t, *J* = 8.0 Hz, 1H), 7.05 (s, 1H), 6.89 (s, 2H), 3.45-3.38 (m, 1H), 1.22 (d, *J* = 6.8 Hz, 6H). |
| 76 | 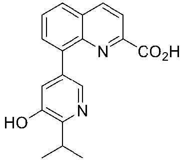 | 309.1 | ¹H NMR (400 MHz, DMSO-*d*₆) δ 10.23 (s, 1H), 9.36 (s, 1H), 8.40 (d, *J* = 2.0 Hz, 1H), 8.17 (d, *J* = 8.4 Hz, 1H), 8.13 - 8.04 (m, 2H), 7.78 (td, *J* = 8.4, 6.4, 1.6 Hz, 1H), 7.55 (d, *J =* 2.0 Hz, 1H), 3.49 (p, *J* = 6.8 Hz, 1H), 1.26 (d, *J* = 6.8 Hz, 6H). |
| 77 | 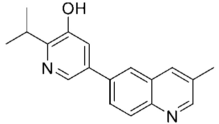 | 279.1 | ¹H NMR (400 MHz, DMSO-*d*₆) δ 9.97 (s, 1H), 8.78 (d, *J* = 2.0 Hz, 1H), 8.42 (d, *J* = 2.0 Hz, 1H), 8.20 (s, 1H), 8.12 (d, *J =* 2.0 Hz, 1H), 8.07 (d, *J* = 8.8 Hz, 1H), 7.93 (dd, *J* = 8.8, 2.0 Hz, 1H), 7.45 (d, *J* = 2.0 Hz, 1H), 3.47 - 3.39 (m, 1H), 2.51 (s, 3H), 1.23 (d, *J* = 6.8 Hz, 6H). |
| 78 | 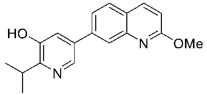 | 295.1 | ¹H NMR (400 MHz, DMSO-*d*₆) δ 9.98 (s, 1H), 8.42 (d, *J* = 2.0 Hz, 1H), 8.27 (d, *J* = 8.8 Hz, 1H), 8.00 - 7.91 (m, 2H), 7.70 (dd, *J* = 8.4, 1.6 Hz, 1H), 7.46 (d, *J* = 2.0 Hz, 1H), 7.04 (d, *J* = 8.8 Hz, 1H), 4.02 (s, 3H), 3.47 - 3.38 (m, 1H), 1.23 (d, *J* = 6.8 Hz, 6H). |
| 79 | 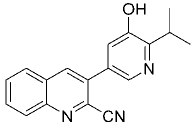 | 290.1 | 1H NMR (400 MHz, DMSO-*d*6) δ 10.24 (s, 1H), 8.75 (s, 1H), 8.30 (d, *J* = 2.0 Hz, 1H), 8.18 (d, *J* = 9.6 Hz, 1H), 7.97 (ddd, *J* = 8.4, 6.8, 1.6 Hz, 1H), 7.88 - 7.83 (m, 1H), 7.47 (d, *J* = 2.0 Hz, 1H), 3.51 - 3.43 (m, 1H), 1.25 (d, *J* = 6.8 Hz, 6H). |
| 80 | 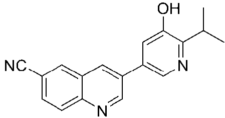 | 290.1 | ¹H NMR (400 MHz, DMSO-*d₆*, ppm) 10.17 (s, 1H), 9.37 (d, *J =* 2.4 Hz, 1H), 8.72 (dd, *J* = 6.8, 1.6 Hz, 2H), 8.49 (d, *J =* 2.0 Hz, 1H), 8.22 (d, *J* = 8.8 Hz, 1H), 8.09-8.07 (m, 1H), 7.51 (d, *J* = 2.0 Hz, 1H), 3.48-3.42 (m, 1H), 1.23 (d, *J* = 6.8 Hz, 6H). |
| 81 | 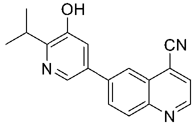 | 290.1 | ¹H NMR (400 MHz, DMSO-*d*₆) δ 10.11 (s, 1H), 9.13 (d, *J = 4.4* Hz, 1H), 8.50 (d, *J* = 2.0 Hz, 1H), 8.32 - 8.25 (m, 2H), 8.23 - 8.18 (m, 2H), 7.56 (d, *J* = 2.0 Hz, 1H), 3.50 - 3.40 (m, 1H), 1.24 (d, *J* = 6.8 Hz, 6H). |
| 82 | 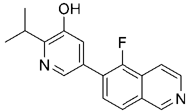 | 283.1 | ¹H NMR (400 MHz, DMSO-*d*₆) δ 10.09 (s, 1H), 9.44 (s, 1H), 8.66 (d, *J* = 5.9 Hz, 1H), 8.31 (s, 1H), 8.10 (d, *J =* 8.6 Hz, 1H), 7.98 (d, *J =* 5.6 Hz, 1H), 7.85 (d, *J* = 8.0 Hz, 1H), 7.45 (d, *J* = 1.6 Hz, 1H), 3.44 (d, *J* = 6.8 Hz, 1H), 1.23 (d, *J* = 6.8 Hz, 6H). |
| 83 | 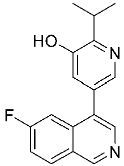 | 283.1 | ¹H NMR (400 MHz, DMSO-*d*₆) δ 10.06 (s, 1H), 9.39 (s, 1H), 8.49 (s, 1H), 8.38 (dd, *J =* 9.2, 6.0 Hz, 1H), 8.15 (d, *J* = 2.0 Hz, 1H), 7.69 (td, *J* = 8.8, 2.4 Hz, 1H), 7.51 (dd, *J* = 10.8, 2.4 Hz, 1H), 7.28 (d, *J* = 2.0 Hz, 1H), 3.46 (d, *J =* 6.8 Hz, 1H), 1.26 (d, *J* = 6.8 Hz, 6H). |
| 84 | 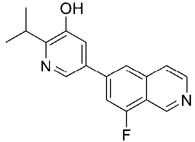 | 283.1 | ¹H NMR (400 MHz, DMSO-*d*₆) δ 10.05 (s, 1H), 9.48 (s, 1H), 8.66 (d, *J* = 5.6 Hz, 1H), 8.47 (d, *J* = 2.0 Hz, 1H), 8.09 (s, 1H), 7.98 (d, *J* = 5.6 Hz, 1H), 7.83 (d, *J* = 12.0 Hz, 1H), 7.48 (d, *J* = 2.0 Hz, 1H), 3.44 (d, *J* = 6.8 Hz, 1H), 1.22 (d, *J* = 6.8 Hz, 6H). |
| 85 | 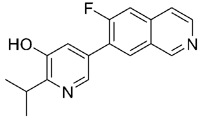 | 283.1 | ¹H NMR (400 MHz, DMSO-*d*₆) δ 10.08 (s, 1H), 9.38 (s, 1H), 8.54 (d, *J* = 6.0 Hz, 1H), 8.38 (d, *J* = 8.0 Hz, 1H), 8.28 (d, *J =* 2.0 Hz, 1H), 7.93 (d, *J* = 11.6 Hz, 1H), 7.86 (d, *J =* 5.6 Hz, 1H), 7.40 (d, *J* = 2.0 Hz, 1H), 3.44 (p, *J* = 6.8 Hz, 1H), 1.23 (d, *J* = 6.8 Hz, 6H). |
| 86 | 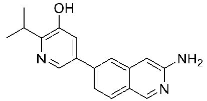 | 280.1 | ¹H NMR (400 MHz, DMSO-*d₆*, ppm) 9.96 (s, 1H), 8.85 (s, 1H), 8.38 (d, *J* = 2.0 Hz, 1H), 7.90 (d, *J* = 8.4 Hz, 1H), 7.74 (s, 1H), 7.46-7.29 (m, 2H), 6.71 (s, 1H), 6.08 (s, 2H), 3.45-3.40 (m, 1H), 1.22 (d, *J* = 6.8 Hz, 6H). |
| 87 | 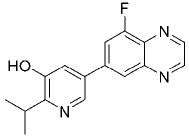 | 284.1 | ¹H NMR (400 MHz, MeOD, ppm) 9.00 (d, *J* = 2.0 Hz, 1H), 8.92 (d, *J* = 2.0 Hz, 1H), 8.39 (d, *J* = 2.0 Hz, 1H), 8.16 (s, 1H), 7.92 (dd, *J* = 11.2, 1.6 Hz, 1H), 7.52 (d, *J* = 2.0 Hz, 1H), 3.65-3.44 (m, 1H), 1.31 (d, *J* = 7.2 Hz, 6H). |
| 88 | 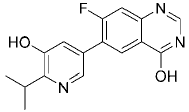 | 300.1 | ¹H NMR (400 MHz, DMSO-*d*₆) δ 12.42 (s, 1H), 10.02 (s, 1H), 8.25 - 8.20 (m, 1H), 8.20 - 8.15 (m, 2H), 3.42 (d, *J* = 6.8 Hz, 1H), 1.22 (d, *J* = 6.8 Hz, 6H). |
| 89 | 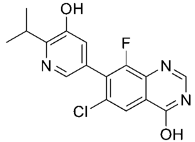 | 334.1 | ¹H NMR (400 MHz, DMSO-*d*₆) δ 10.08 (s, 1H), 8.22 (s, 1H), 8.05 (d, *J* = 1.2 Hz, 1H), 8.02 (d, *J* = 2.0 Hz, 1H), 7.17 (d, *J =* 2.0 Hz, 1H), 3.43 (p, *J* = 6.8 Hz, 1H), 1.23 (d, *J* = 6.8 Hz, 6H). |
| 90 | 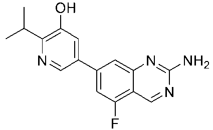 | 299.1 | ¹H NMR (400 MHz, DMSO-*d*₆) δ 9.26 (s, 1H), 8.39 (d, *J* = 2.0 Hz, 1H), 7.43 - 7.37 (m, 2H), 7.28 (dd, *J =* 11.2, 1.2 Hz, 1H), 7.16 (s, 2H), 3.42 (p, *J* = 6.8 Hz, 1H), 1.21 (d, *J* = 6.8 Hz, 6H). |
| 91 | 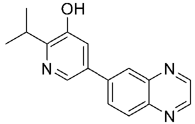 | 266.1 | ¹H NMR (400 MHz, DMSO-*d*₆) δ 10.07 (s, 1H), 9.00 (d, *J* = 2.0 Hz, 1H), 8.97 (d, *J* = 1.6 Hz, 1H), 8.50 (d, *J =* 2.0 Hz, 1H), 8.30 (d, *J =* 2.0 Hz, 1H), 8.21 (d, *J =* 8.8 Hz, 1H), 8.15 (dd, *J* = 8.8, 2.0 Hz, 1H), 3.44 (p, *J =* 6.8 Hz, 1H), 1.23 (d, *J* = 6.8 Hz, 6H). |
| 92 | 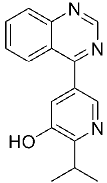 | 266.1 | ¹H NMR (400 MHz, DMSO) δ 9.80 (s, 1H), 8.55 (d, *J =* 8.4 Hz, 1H), 8.33 (d, *J* = 2.0 Hz, 1H), 8.11 - 8.06 (m, 2H), 7.86 - 7.83 (m, 1H), 7.77 (t, *J* = 7.6 Hz, 1H), 7.50 (s, 1H), 3.45 (p, *J* = 6.8 Hz, 1H), 1.26 (d, *J* = 6.8 Hz, 6H). |
| 93 | 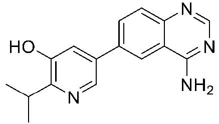 | 281.1 | |
| 94 | 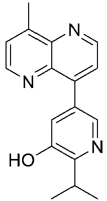 | 280.1 | ¹H NMR (400 MHz, DMSO-*d*₆) δ 9.94 (s, 1H), 9.06 (d, *J* = 4.4 Hz, 1H), 8.87 (d, *J =* 4.4 Hz, 1H), 8.31 (d, *J =* 2.0 Hz, 1H), 7.82 (d, *J* = 4.4 Hz, 1H), 7.71 (dd, *J* = 4.4, 1.2 Hz, 1H), 7.57 (d, *J* = 2.0 Hz, 1H), 3.46 (p, *J =* 6.8 Hz, 1H), 1.25 (d, *J* = 6.8 Hz, 6H). |
| 94 | 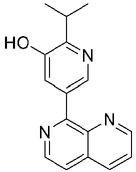 | 266.1 | ¹H NMR (400 MHz, DMSO-*d*₆) δ 9.92 (s, 1H), 9.09 (dd, *J* = 4.0, 1.6 Hz, 1H), 8.74 - 8.67 (m, 2H), 8.51 (dd, *J =* 8.4, 1.6 Hz, 1H), 7.93 (d, *J* = 5.2 Hz, 1H), 7.86 (d, *J =* 2.0 Hz, 1H), 7.83 (dd, *J* = 8.4, 4.0 Hz, 1H), 3.46 (d, *J =* 6.8 Hz, 1H), 1.25 (d, *J* = 6.8 Hz, 6H). |
| 96 | 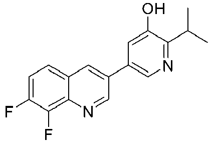 | 301.1 | ¹H NMR (400 MHz, DMSO-*d*₆) δ 10.10 (s, 1H), 9.28 (d, *J* = 2.0 Hz, 1H), 8.73 (d, *J* = 2.0 Hz, 1H), 8.48 (d, *J =* 2.0 Hz, 1H), 8.03 - 7.95 (m, 1H), 7.86 - 7.75 (m, 1H), 7.50 (d, *J* = 2.0 Hz, 1H), 3.45 (p, *J* = 6.8 Hz, 1H), 1.23 (d, *J* = 6.8 Hz, 6H). |
| 97 | 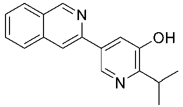 | 265.1 | ¹H NMR (400 MHz, DMSO-*d₆*, ppm) 9.93 (s, 1H), 9.41 (s, 1H), 8.77 (d, *J* = 1.6 Hz, 1H), 8.37 (s, 1H), 8.15 (d, *J* = 8.0 Hz, 1H), 8.04 (d, *J* = 8.0 Hz, 1H), 7.93 (d, *J* = 1.6 Hz, 1H), 7.83-7.79 (m, 1H), 7.71-7.67 (m, 1H), 3.46-3.40 (m, 1H), 1.23 (d, *J* = 6.8 Hz, 6H). |
| 98 | 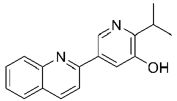 | 265.2 | ¹H NMR (400 MHz, DMSO-*d₆*, ppm) 10.03 (s, 1H), 8.81 (d, *J* = 2.0 Hz, 1H), 8.47 (d, *J =* 8.8 Hz, 1H), 8.12 (d, *J* = 8.8 Hz, 1H), 8.07-7.98 (m, 3H), 7.82-7.77(m, 1H), 7.64-7.55 (m, 1H), 3.50-3.38 (m, 1H), 1.23 (d, *J =* 6.8 Hz, 6H). |
| 99 | 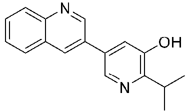 | 265.0 | ¹H NMR (400 MHz, DMSO-*d₆*, ppm) 10.05 (s, 1H), 9.18 (d, *J* = 2.4 Hz, 1H), 8.60 (d, *J* = 2.4 Hz, 1H), 8.48 (d, *J* = 2.0 Hz, 1H), 8.12-8.05 (m, 2H), 7.83-7.76 (m, 1H), 7.72-7.62 (m, 1H), 7.51 (d, *J* = 2.0 Hz, 1H), 3.49-3.41 (m, 1H), 1.23 (d, *J* = 6.8 Hz, 6H). |
| 100 | 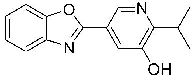 | 255.1 | ¹H NMR (400 MHz, DMSO-*d₆*, ppm) 10.42 (s, 1H), 8.77 (d, *J =* 1.6 Hz, 1H), 7.88-7.76 (m, 3H), 7.47-7.35 (m, 2H), 3.54-3.38 (m, 1H), 1.22 (d, *J* = 6.8 Hz, 6H). |
| 101 | 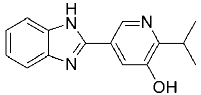 | 254.1 | ¹H NMR (400 MHz, DMSO-*d₆*, ppm) 12.96 (s, 1H), 10.16 (s, 1H), 8.72 (d, *J* = 1.8 Hz, 1H), 7.85 (d, *J =* 2.0 Hz, 1H), 7.66 (d, *J* = 8.0 Hz, 1H), 7.53 (d, *J =* 7.6 Hz, 1H), 7.26-7.19 (m, 2H), 3.49-3.37 (m, 1H), 1.22 (d, *J =* 6.8 Hz, 6H). |
| 102 | 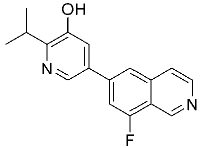 | 283.2 | ¹H NMR (400 MHz, DMSO-*d*₆. ppm) δ 10.05 (s, 1H), 9.48 (s, 1H), 8.66 (d, *J* = 5.6 Hz, 1H), 8.47 (d, *J* = 2.0 Hz, 1H), 8.09 (s, 1H), 7.98 (d, *J* = 5.6 Hz, 1H), 7.83 (d, *J = 12.0* Hz, 1H), 7.48 (d, *J* = 2.0 Hz, 1H), 3.44 (d, *J =* 6.8 Hz, 1H), 1.22 (d, *J* = 6.8 Hz, 6H). |

### Example 3. Preparation of (E)-2-isopropyl-5-styrylpyridin-3-ol (Compound 103)

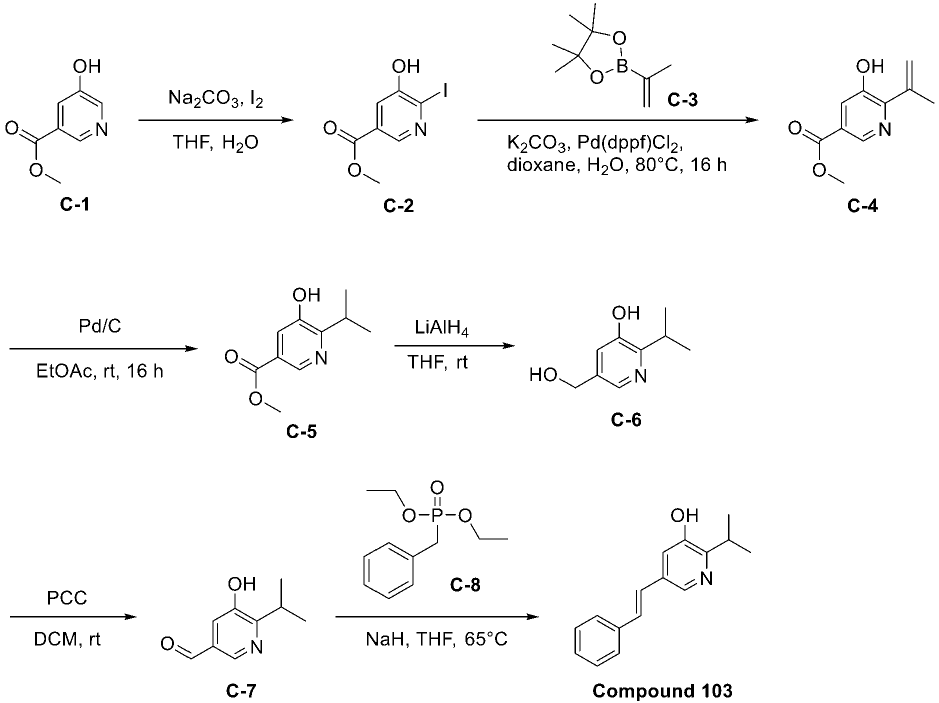

To a solution of methyl 5-hydroxypyridine-3-carboxylate **(C-1,** 5.0 g, 0.033 mol) in THF/H₂O (50 ml / 50 ml) was added Na₂CO₃ (10.4 g, 0.098 mol) and I₂ (20.8 g, 0.082 mol). The reaction mixture was stirred at 25 °C for 12 h. The solvent was removed by vacuum, and the residue was purified by flash chromatography (eluting with DCM / MeOH from 100/0 to 90/10 in 30 mins) to afford compound **C-2.** LCMS (ESI): calcd for C₇H₆INO₃ [M+H]⁺ m/z 280.0, found 279.8.

To a solution of compound C-2 (705.0 mg, 2.53 mmol) in dioxane/H₂O=10:1 (15 mL) was added 4,4,5,5-tetramethyl-2-(prop-1-en-2-yl)-1,3,2-dioxaborolane **(C-3,** 509.5 mg, 3.03 mmol) and Pd(PPh₃)₄ (292.0 mg, 2.53 mmol) and K₂CO₃ (697.3 mg, 5.05 mmol). The reaction mixture was stirred at 80 °C for 12 h under nitrogen. The solvent was removed by vacuum to furnish a crude product which was purified by flash chromatography (eluting with DCM/MeOH from 100/0 to 90/10 in 30 mins) to afford compound **C-4.** LCMS (ESI): calcd for C₁₀H₁₁NO₃ [M+H]⁺ m/z 194.0, found 194.0.

To a solution of methyl 5-hydroxy-6-(prop-1-en-2-yl)pyridine-3-carboxylate (134.0 mg, 0.69 mmol) in ethyl acetate (10 mL) was added Pd/C (73.8 mg, 10%). The reaction mixture was stirred at 25 °C for 12 h under 1 atm of hydrogen. The residue was filtered over celite and the filtrate was concentrated under reduced pressure to afford compound **C-5,** which was used directly for the next step. LCMS (ESI): calcd for C₁₀H₁₃NO₃ [M+H]⁺ m/z 196.0, found 196.0.

To a solution of methyl 5-hydroxy-6-isopropylpyridine-3-carboxylate (104.0 mg, 0.53 mmol) in THF (5 mL) was added LiAlH₄ (30.3 mg, 0.80 mmol) slowly at 0°C. The reaction mixture was stirred at 25°C for 2 h. The residue was quenched by water and removed by vacuum. The residue crude product was purified by flash chromatography (eluting with DCM/MeOH from 100/0 to 80/20 in 20 mins) to afford compound **C-6.** LCMS (ESI): calcd for C₉H₁₃NO₂ [M+H]⁺ m/z 168.0, found 168.0.

To a solution of 5-(hydroxymethyl)-2-isopropylpyridin-3-ol (80.0 mg, 0.48 mmol) in DCM (5 mL) was added PCC (154.7 mg, 0.72 mmol) slowly at 0°C. The reaction mixture was stirred at 25 °C for 4 h. The solvent was removed by vacuum to furnish a crude product which was purified by flash chromatography (DCM/MeOH = 100/0 to 80/20) to afford compound **C-7.** LCMS (ESI): calcd for C₉H₁₁NO₂ [M+H]⁺ m/z 166.0, found 166.0.

To a solution of diethyl benzylphosphonate **(C-8,** 134.7 mg, 0.59 mmol) in THF (3 mL) was added NaH (19.0 mg, 0.79 mmol, 60%) at 0°C. After 2 h, the 5-hydroxy-6-isopropylpyridine-3-carbaldehyde (65.0 mg, 0.39 mmol) in THF was added dropwise at 0°C. The reaction mixture stirred at 25°C for 16 h. After completion, the mixture was poured onto ice, extracted with EtOAc (10 mL×3), the combined organic layer was then washed with sat. NaCl (20 mL) and dried over anhydrous Na₂SO₄. After filtration, the solvent was evaporated under reduced pressure, the crude product was purified by silica column chromatography (PE/EtOAc = 100/0 to 50/50) to afford compound **103.** LCMS (ESI): calcd for C₁₆H₁₇NO [M+H]⁺ m/z 240.1, found 240.1. ¹H NMR (400 MHz, CDCl₃, ppm): 8.26 (s, 1H), 7.49 (d, *J =* 7.6 Hz, 2H), 7.36 (t, *J =* 7.2 Hz, 2H), 7.28 (d, *J* = 5.6 Hz, 2H), 7.11-6.97 (m, 2H), 3.61-3.15 (m, 1H), 1.33 (d, *J* = 6.8 Hz, 6H).

### Example 4 - Preparation of (E)-1-isopropyl-6-methoxy-4-styrylpyridin-2(1H)-one (Compound 104)

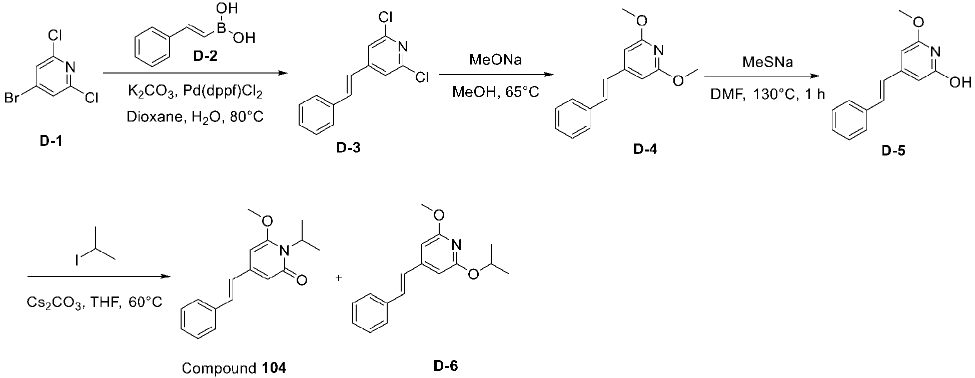

To a solution of 4-bromo-2,6-dichloropyridine (2.0 g, 8.8 mmol), (E)-2-phenylethenyl]boranediol (1.56 g, 10.5 mmol) and K₂CO₃ (2.43 g, 17.6 mmol) in Dioxane/H₂O=10:1 (42 mL), stirred under nitrogen at rt was added Pd(dppf)Cl₂ (643.0 mg, 0.88 mmol) . The reaction mixture was stirred at 80°C for 16 h. The mixture was quenched with water (50 mL) and extracted with EtOAc (3 × 100 mL), the organic layer was washed with brine, dried over Na₂SO₄, filtered out and concentrated to get crude product which was purified by flash chromatography (eluting with PE/EtOAc from 100/0 to 80/20 in 30 min) to afford 2,6-dichloro-4-[(E)-2-phenylethenyl]pyridine. LCMS (ESI): calcd for C₁₃H₉Cl₂N [M+H]⁺ m/z 250.0, found 250.0.

To a solution of 2,6-dichloro-4-[(E)-2-phenylethenyl]pyridine (1.8 g, 7.2 mmol) in MeOH (50 mL) stirred under nitrogen was added a solution of MeONa (7.8 g, 144 mmol). The reaction mixture was stirred at 65°C for 3 d. The mixture was quenched with water (50 mL) and extracted with EtOAc (3 × 50 mL), the organic layer was washed with brine, dried over Na₂SO₄, filtered and concentrated to get crude product 2,6-dimethoxy-4-[(E)-2-phenylethenyl]pyridine. LCMS (ESI): calcd for C₁₅H₁₅O₂N [M+H]⁺ m/z 242.1, found 242.1.

To a solution of 2,6-dimethoxy-4-[(E)-2-phenylethenyl]pyridine (1.6 g, 6.64 mmol) in DMF (32 ml) stirred under nitrogen at rt was added MeSNa (697.0 mg, 9.96 mmol). The reaction mixture was stirred at 130°C for 1 h. The mixture was quenched with water (50 ml) and extracted with EtOAc (3 × 50 mL), the organic layer was washed with brine, dried over Na₂SO₄, filtered and concentrated to get crude product 6-methoxy-4-[(E)-2-phenylethenyl]pyridin-2-ol. LCMS (ESI): calcd for C₁₄H₁₃O₂N [M+H]⁺ m/z 228.2, found 228.2.

To a solution of 6-methoxy-4-[(E)-2-phenylethenyl]-1H-pyridin-2-one(1.2 g, 5.3 mmol), 2-iodopropane (1.8 g, 10.6 mmol) in THF (24 mL) stirred under nitrogen was added Cs₂CO₃ (3.4 g, 10.6 mmol). The reaction mixture was stirred at 60°C for 16 h. The mixture was quenched with water (50 mL) and extracted with EtOAc (3 × 50 mL), the organic layer was washed with brine, dried over Na₂SO₄, filtered and concentrated to get crude product which was purified by reversed-phase column (eluting with H₂O/MeCN from 100/0 to 50/50 in 30 mins) to afford compound **104** and compound **D-6.**

### Example 5 - Preparation of (E)-6-hydroxy-1-isopropyl-4-styrylpyridin-2(1H)-one (Compound 105)

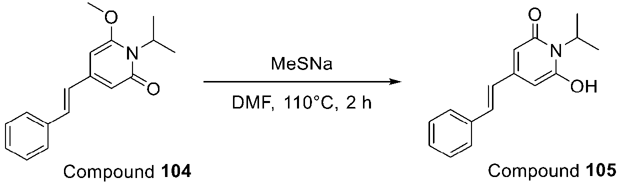

To a solution of 2,6-dimethoxy-4-[(E)-2-phenylethenyl]pyridine (50.0 mg, 0.19 mmol) in DMF (2 mL) stirred under nitrogen at rt was added MeSNa (52.0 mg, 0.74 mmol). The reaction mixture was stirred at 110°C for 2 h. The reaction was monitored by LCMS. After the reaction was completed, the mixture was concentrated in vacuo to afford crude product, which was purified by prep-HPLC (columns: Gemini 5 um C18 150 × 21.2 mm, mobile phase: ACN - H₂O (0.1% FA), gradient: 50 - 95, 10.0 min) to afford compound **105.**

### Example 6 - Preparation of (E)-6-isopropoxy-4-styrylpyridin-2-ol (Compound 106)

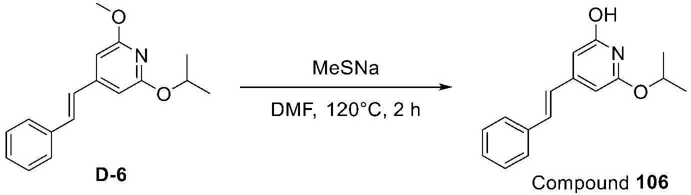

To a solution of 2-isopropoxy-6-methoxy-4-[(E)-2-phenylethenyl]pyridine (300.0 mg, 1.1138 mmol) in DMF (7 mL) was added MeSNa (155.9 mg, 2.2276 mmol). The reaction was stirred at 120°C for 2 h. The reaction was monitored by LCMS. After the reaction was completed, the mixture was concentrated in vacuo to afford crude product, which was purified by prep-HPLC (columns: Gemini 5 um C18 150 × 21.2 mm, mobile phase: ACN - H₂O (0.1% NH₃.H₂O), gradient: 30 - 95, 10.0 min) to afford compound **106.**

### Example 7 - Preparation of 1-{2,6-dimethoxy-4-[(E)-2-phenylethenyl]phenyl}ethenone (Compound 107)

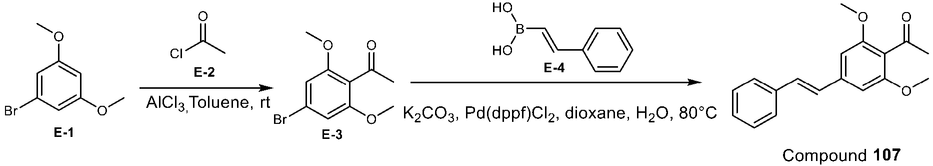

To a solution of 1-bromo-3,5-dimethoxybenzene (1.0 g, 0.46 mmol) in toluene was added AlCl₃ (610.0 mg, 0.46 mol) at 0 °C. After 2 h, the acetyl chloride (540.0 mg, 0.69 mmol) was added. The reaction was stirred for 0.5 h. The reaction was monitored by TLC. After the reaction was completed, the reaction mixture was then quenched ice water, and extracted with EtOAc (100 ml × 3). The combined organic phase was washed with sat. NaCl (500 ml), dried over anhydrous Na₂SO₄, filtered and concentrated to get crude product which was purified by column chromatography on silica gel (eluting with PE/EtOAc from 100 / 0 to 20 / 80 in 30 mins) to afford 1-(4-bromo-2,6-dimethoxyphenyl) ethenone. ¹H NMR (400 MHz, CDCl₃, ppm): 6.69 (s, 2H), 3.44 (s, 6H), 2.42 (s, 3H).

To a solution of 1-(4-bromo-2,6-dimethoxyphenyl) ethenone (1.0 g, 3.86 mmol) in dioxane (50 mL) and H₂O (5 mL) was added [(E)-2-phenylethenyl] boranediol (628.3 mg, 4.26mmol), K₂CO₃(1.1 g, 7.72 mmol) and Pd(dppf)Cl₂ (282.0 mg, 0.386 mmol). The reaction mixture was stirred for 16 h at 80°C under nitrogen. The reaction was monitored by LCMS. After the reaction was completed, the reaction mixture was cooled down and concentrated on a rotary evaporator under reduced pressure. The residue was purified by silica gel chromatography (eluting with PE/EtOAc from 100/0 to 20/80 in 30 mins) to afford compound **107.**

### Example 8 - Preparation of 3,5-dimethoxy-4-(prop-1-en-2-yl)benzaldehyde (Compound 108)

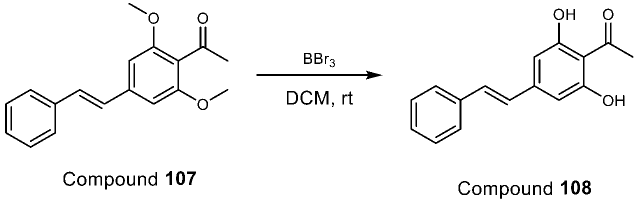

To a solution of 1-{2,6-dimethoxy-4-[(E)-2-phenylethenyl]phenyl}ethenone (170.0 mg, 0.602 mmol) in DCM (5 mL) was added tribromoborane (1.2 mL, 1 M in DCM) at 0°C. The reaction mixture was stirred at 25°C for 16 h. The reaction was monitored by TLC. After the reaction was completed, the residue was quenched by ice water, adjusted with NaHCO₃ (aq.) to 7-8, extracted with DCM (10 mL × 3). The organic phases were combined, washed with brine, dried over Na₂SO₄, and concentrated in vacuo. The residue was purified by prep-HPLC (columns: Gemini 5 um C18 150 × 21.2 mm, mobile phase: ACN - H₂O (0.1% FA), gradient: 35 - 65, 9 min) to compound **108.**

### Example 9 - Preparation of 5-isopropyl-3-[(E)-2-phenylethenyl]-1,2-oxazol-4-ol (Compound 109)

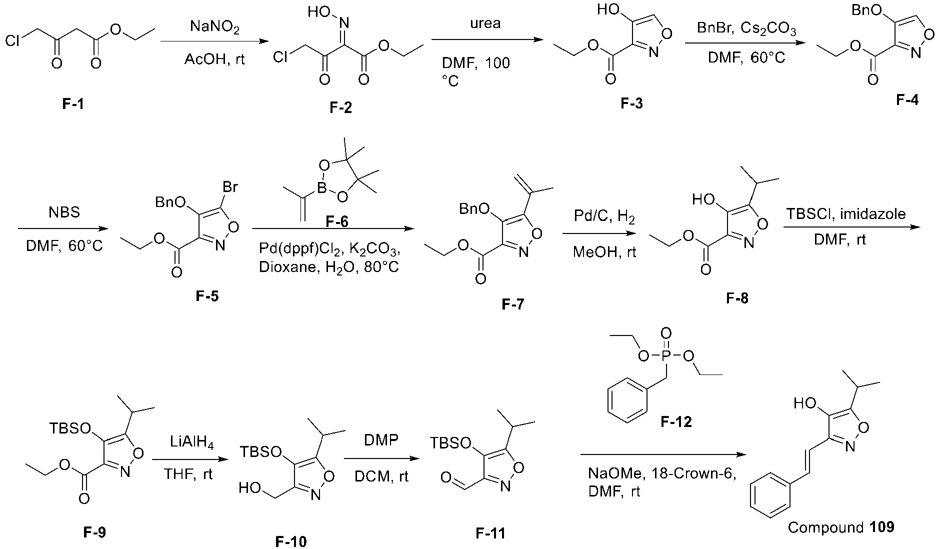

To a solution of ethyl 4-chloro-3-oxobutanoate (8.8 g, 0.0535 mol) in AcOH (50 mL) was added NaNO₂ (4.6 g, 0.0668 mol) in water (40 mL) dropwise at -10°C. The resulting solution was stirred for 2 h at -10°C. The reaction mixture was then treated with water (50 mL) and extracted with EtOAc (3 × 100 mL). The organic lager was dried over Na₂SO₄ and concentrated to give ethyl (2E)-4-chloro-2-(hydroxyimino)-3-oxobutanoate (11.0 g, red oil, yield: 99.4%), which was used directly for the next step without purification. LCMS (ESI): calcd for C₆H₉ClNO₄ [M+H]⁺ m/z = 194.1, found 194.1.

To a solution of ethyl (2E)-4-chloro-2-(hydroxyimino)-3-oxobutanoate (11.0 g, 0.0570 mol) in DMF (80 ml) was added urea (43.7 g, 0.7272 mol). The reaction mixture was stirred at 100°C for 30 min. After completion, saturated aqueous ice water (100 mL) was added. The residue was extracted with EtOAc (3 × 100mL). The organic phase was washed with saturated brine, dried over Na₂SO₄, filtered out, and concentrated to get crude product to give ethyl 4-hydroxy-1,2-oxazole-3-carboxylate, which was used directly for the next step without purification. LCMS (ESI): calcd for C₆H₈NO₄ [M+H]⁺ m/z 158.0, found 158.2.

To a solution of ethyl 4-hydroxy-1,2-oxazole-3-carboxylate (3.0 g, 19.1 mmol) in DMF (20 ml) was added BnBr (4.9 g, 28.6 mmol) and Cs₂CO₃ (12.4 g, 38.2 mmol). The reaction was stirred at 60°C for 2 h. The solvent was extracted with EtOAc and H₂O, the organic layer was washed with brine, dried over Na₂SO₄, filtered out and concentrated to get crude product which was purified by flash chromatography (eluting with PE/EtOAc from 100/0 to 75/25 in 30 mins) to afford ethyl 4-(benzyloxy)-1,2-oxazole-3-carboxylate. LCMS (ESI): calcd for C₁₃H₁₄NO₄ [M+H]⁺ m/z 248.2, found 248.2.

To a solution of ethyl 4-(benzyloxy)-1,2-oxazole-3-carboxylate (2.7 g, 10.9 mmol) in DMF (15 mL) was added NBS (3.9 g, 21.8 mmol). The reaction was stirred at 60°C for 16 h. The solvent was extracted with EtOAc and H₂O, the organic layer was washed with brine, dried over Na₂SO₄, filtered out and concentrated to get crude product which was purified by flash chromatography (eluting with PE/EtOAc from 100/0 to 75/25 in 30 mins) to afford ethyl 4-(benzyloxy)-5-bromo-1,2-oxazole-3-carboxylate. LCMS: (ESI) calcd for C₁₃H₁₃BrNO₄ [M+H]⁺ m/z = 327.8, found 327.8.

To a solution of ethyl 4-(benzyloxy)-5-bromo-1,2-oxazole-3-carboxylate (2.0 g, 6.13 mmol) in 1,4-dioxane / H₂O (20 mL/2 mL) was added 4,4,5,5-tetramethyl-2-(prop-1-en-2-yl)-1,3,2-dioxaborolane (1.4 g, 7.97 mmol), Pd(dppf)Cl₂ (450.0 mg, 0.61 mmol) and K₂CO₃ (1.7 g, 12.3 mmol). The reaction mixture was stirred at 80°C for 16 h under nitrogen. The LCMS showed the desired MS was detected. The solvent was removed by vacuum. The residue was purified by flash chromatography (eluting with PE/EtOAc from 100/0 to 80/20 in 30 mins) to afford ethyl 4-(benzyloxy)-5-(prop-1-en-2-yl)-1,2-oxazole-3-carboxylate. LCMS (ESI): calcd for C₁₆H₁₈NO₄ [M+H]⁺ m/z 288.2, found 288.2.

To a solution of ethyl 4-(benzyloxy)-5-(prop-1-en-2-yl)-1,2-oxazole-3-carboxylate (1.1 g, 3.83 mmol) in MeOH (25 mL) was added Pd/C (200.0 mg, 20wt%). The reaction mixture was stirred at 25°C for 16 h under H₂ (1 atm). The LCMS showed the desired MS was detected. The reaction mixture is filtered through celite and concentrated to get the ethyl 4-hydroxy-5-isopropyl-1,2-oxazole-3-carboxylate. LCMS (ESI): calcd for C₉H₁₄NO₄ [M+H]⁺ m/z 200.2, found 200.2.

To a solution of ethyl 4-hydroxy-5-isopropyl-1,2-oxazole-3-carboxylate (500.0 mg, 2.5 mmol) in DMF (5 mL) was added TBSCI (760.0 mg, 5.0 mmol) and imidazole (512.6 mg, 7.5 mmol). The reaction mixture was stirred at 25°C for 16 h. The LCMS showed the desired MS was detected. The solvent was removed by vacuum and the residue was extracted with DCM (50 mL), the organic layer was washed with brine, dried over Na₂SO₄, filtered and concentrated to get crude product which was purified by flash chromatography (eluting with PE/EtOAc from 100/0 to 80/20 in 30 mins) to afford ethyl 4-[(tert-butyldimethylsilyl)oxy]-5-isopropyl-1,2-oxazole-3-carboxylate. LCMS (ESI): calcd for C₁₅H₂₈NO₄Si [M+H]⁺ m/z 314.0, found 314.0.

To a solution of 4-[(tert-butyldimethylsilyl)oxy]-5-isopropyl-1,2-oxazole-3-carboxylate (700.0 mg, 2.23 mmol) in THF (7 mL) was added LiAlH₄ (101.7 mg, 2.7 mmol). The reaction mixture was stirred at 25°C for 2 h. The LCMS showed the desired MS was detected. The reaction was quenched with Na₂SO₄.10H₂O. The solvent was removed by vacuum and the residue was extracted with EtOAc (50 mL), the organic layer was washed with brine, dried over Na₂SO₄, filtered and concentrated to get crude product which was purified by flash chromatography (eluting with PE/EtOAc from 100/0 to 80/20 in 30 mins) to afford {4-[(tert-butyldimethylsilyl)oxy]-5-isopropyl-1,2-oxazol-3-yl}methanol. LCMS (ESI): calcd for C₁₃H₂₇NO₃Si [M+H]⁺ m/z 272.2, found 272.2.

To a solution of {4-[(tert-butyldimethylsilyl)oxy]-5-isopropyl-1,2-oxazol-3-yl}methanol (170.0 mg, 0.627 mmol) in DCM (5 mL) was added DMP (532.1 mg, 1.25 mmol). The reaction mixture was stirred at 25°C for 16 h. The LCMS showed the desired MS was detected. The solvent was extracted with DCM (202 mL), the organic layer was washed with brine, dried over Na₂SO₄, filtered and concentrated to get crude product which was purified by flash chromatography (eluting with PE/EtOAc from100/0 to 95/5 in 10 mins) to afford 4-[(tertbutyldimethylsilyl)oxy]-5-isopropyl-1,2-oxazole-3-carbaldehyde. LCMS (ESI): calcd for C₁₃H₂₄NO₃Si [M+H]⁺ m/z 269.1, found 269.1.

To a solution of diethyl benzyl phosphonate (30.5 mg, 0.134 mmol) in DMF (2 mL) was added 18-Crown-6 (14.7 mg, 0.056 mmol) and NaOMe (30wt%, 48.0 mg, 0.267 mmol). After 30 min, the mixture was cold to 0 °C, then 4-[(tert-butyldimethylsilyl)oxy]-5-isopropyl-1,2-oxazole-3-carbaldehyde (30.0 mg, 0.111 mmol) was added. The reaction mixture was stirred at 25°C for 2 h. The LCMS showed the desired MS was detected. The solvent was quenched with water (1 mL), the solution was purified on reversed-phase column directly (eluting with H₂O (0.1% FA) - MeCN from 100/0 to 0/100 in 30 mins) to afford compound **109.**

### Example 10 - Preparation of 1,3-dimethoxy-5-[(E)-2-phenylethenyl]-2-(prop-1-en-2-yl)benzene (Compound 110)

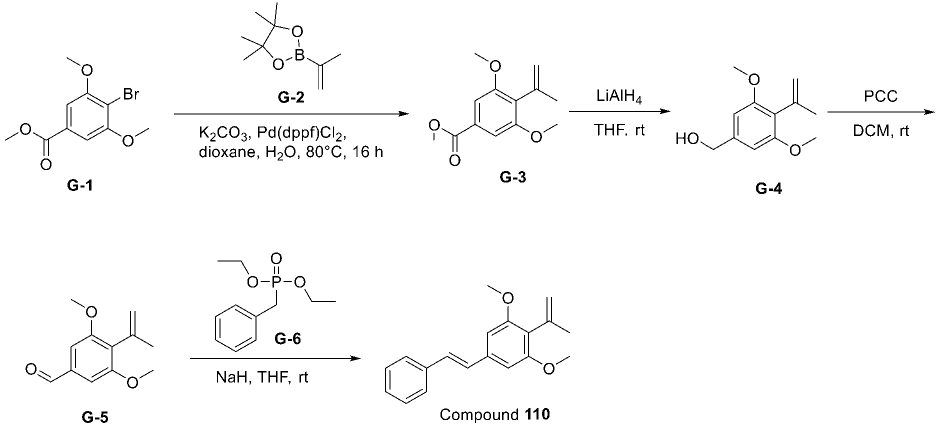

To a solution of methyl 4-bromo-3,5-dimethoxybenzoate (5.0 g, 0.0182 mol) in sat. K₂CO₃ (300 mL) and dioxane (300 mL) was added Pd(dppf)Cl₂ (1.3 g, 0.0018 mol) and 4,4,5,5-tetramethyl-2-(prop-1-en-2-yl) - 1,3,2-dioxaborolane (4.6 g, 0.0273 mol). The reaction mixture was stirred at 80°C for 16 h under N₂. The reaction was monitored by TLC. After the reaction was completed and then cooled to ambient temperature. The mixture was extracted with EtOAc (300 mL × 3). The combined organic phase was washed with sat. NaCl (1 L), dried over anhydrous Na₂SO₄, filtered and concentrated. The residue was purified by column chromatography on silica gel (eluting with PE/EtOAc from 0/100 to 88/12 in 30 mins) to afford methyl 3,5-dimethoxy-4-(prop-1-en-2-yl)benzoate. ¹H NMR (400 MHz, CDCl₃, ppm) 7.26 (s, 2H), 5.36-5.35 (m, 1H), 4.88-4.87 (m, 1H), 3.92 (s, 3H), 3.86 (s, 6H), 2.01 (s, 3H).

To a solution of methyl 3,5-dimethoxy-4-(prop-1-en-2-yl)benzoate (3.5 g, 0148 mol) in THF (60 mL) was slowly added LiAlH₄ (2.1 g, 0.0547 mol) at 0 °C. The resulting was stirred at 25°C for 1 h. The reaction was monitored by TLC. After the reaction was completed, water (20 mL) was added slowly to the mixture to quench excess LiAlH₄, and the mixture was acidified with 10% HCl (aq.). The organic layer was washed with sat. NaCl (200 mL×2), dried over Na₂SO₄ and the solvent was evaporated under reduced pressure to afford [3,5-dimethoxy-4-(prop-1-en-2-yl)phenyl]methanol, which was used directed for the next step without purification. ¹H NMR (400 MHz, DMSO-*d₆*, ppm): 6.61 (s, 2H), 5.15 (s, 1H), 4.71-4.66 (m, 1H), 3.71 (s, 6H), 1.88 (s, 3H).

To a solution of [3,5-dimethoxy-4-(prop-1-en-2-yl)phenyl]methanol (1.0 g, 4.8 mmol) in DCM (20 mL) was added PCC (1.55g, 7.2 mmol). The reaction mixture was stirred at 25°C for 1 h. The reaction was monitored by TLC. After the reaction was completed, the solvent was removed by vacumm to crude product which was purified by column chromatography on silica gel (eluting with PE/EtOAc from 100/0 to 92/8 in 30 mins) to afford 3,5-dimethoxy-4-(prop-1-en-2-yl) benzaldehyde. ¹H NMR (400 MHz, DMSO-*d₆*, ppm): 9.94 (s, 1H), 7.21 (s, 2H), 5.24 (s, 1H), 4.76 (s, 1H), 3.81 (s, 6H), 1.90 (s, 3H).

To a solution of diethyl benzylphosphonate (1.2 g, 5.04 mmol) in THF (20 mL) was added NaH (161.3 mg, 6.72 mmol) at 0°C. After 1 h, the [3,5-dimethoxy-4-(prop-1-en-2-yl)phenyl]methanol (700.0 mg, 3.36 mmol) in THF (3 mL) was added dropwise. The reaction mixture was stirred at 25°C for 16 h. The reaction was monitored by LCMS. After the reaction was completed, the mixture was poured onto ice, extracted with EtOAc (50 mL×3), the combined organic layer was then washed with sat. NaCl (100 mL) and dried over anhydrous Na₂SO₄. After filtration, the solvent was evaporated under reduced pressure, the crude product was purified by silica column chromatography (eluting with CH₂Cl/CH₃OH from 100/0 to 90/10 in 30 mins) to afford compound **110.**

### Example 11 - Preparation of 2-{2,6-dimethoxy-4-[(E)-2-phenylethenyl]phenyl} propan-2-ol (compound 111)

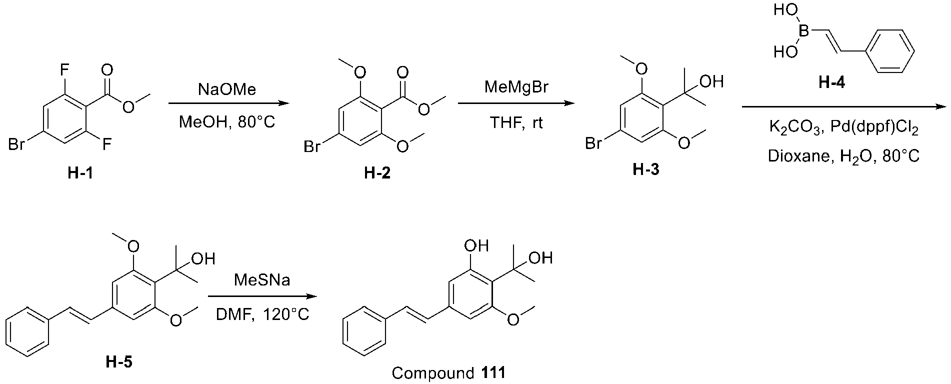

To a solution of methyl 4-bromo-2,6-difluorobenzoate (1.5 g, 6 mmol) in CH₃OH (30 mL) was added MeONa (4.0 mL, 24 mmol). The reaction mixture was stirred for 16 h at 80°C under nitrogen. The reaction was monitored by TLC. After the reaction was completed and then cooled to ambient temperature. The mixture was extracted with EtOAc (100 mL × 3). The combined organic phase was washed with sat. NaCl (200 mL), dried over anhydrous Na₂SO₄, filtered and concentrated. The residue was purified by column chromatography on silica gel chromatography (eluting with PE/EA from 100/0 to 20/80 in 30 mins) to afford methyl 4-bromo-2,6-dimethoxybenzoate. ¹H NMR (400 MHz, DMSO-*d₆*, ppm) 6.95 (s, 2H), 3.78 (s, 6H), 3.74 (s, 3H).

To a solution of methyl 4-bromo-2,6-dimethoxybenzoate (1.0 g, 3.6 mmol) in THF (20 mL) was added MeMgBr (4.3 ml, 4.32 mmol) dropwise at -78 °C under nitrogen. The reaction mixture was stirred at rt for 4 h under nitrogen. The reaction was monitored by TLC. After the reaction was completed, the reaction mixture was quenched to water, extracted with EtOAc (50 mL × 3). The combined organic phase was washed with sat. NaCl (100 mL), dried over anhydrous Na₂SO₄, filtered and concentrated. The residue was purified by silica gel chromatography (eluting with PE/EtOAc from 100/0 to 20/80 in 30 min) to afford 2-(4-bromo-2,6-dimethoxyphenyl)propan-2-olethanone. ¹H NMR (400 MHz, DMSO-*d₆*, ppm) 6.88 (s, 2H), 5.20 (s, 1H), 3.80 (s, 6H), 1.50 (s, 6H).

To a solution of 2-(4-bromo-2,6-dimethoxyphenyl)propan-2-ol (100.0 mg, 0.268mmol) in 1,4-dioxane:H₂O = 2:1 was added [(E)-2-phenylethenyl] boranediol (26.9 mg, 0.0.322 mmol), K₂CO₃ (100.3 mg, 0.536 mmol) and Pd(dppf)Cl₂ (26.6mg, 0.0268mmol). The reaction mixture was stirred at 100°C for 16 h under nitrogen. The reaction was monitored by TLC. After the reaction was completed, the mixture resulting was quenched with water, extracted twice with DCM. The combined organic phase was washed with sat. NaCl. dried over anhydrous Na₂SO₄, filtered and concentrated. The residue was purified by silica gel chromatography (eluting with CH₂Cl/CH₃OH from 100/0 to 90/10 in 30 mins) to afford 2-{2,6-dimethoxy-4-[(E)-2-phenylethenyl]phenyl}propan-2-ol. ¹H NMR (400 MHz, CDCl₃, ppm) 7.56 (d, *J =* 8.0 Hz, 2H), 7.36 (t, *J =* 7.6 Hz, 2H), 7.28-7.11 (m, 3H), 6.86 (s, 2H), 5.45 (s, 1H), 3.89 (s, 6H), 1.58 (s, 6H).

To a solution of 2-{2,6-dimethoxy-4-[(E)-2-phenylethenyl] phenyl} propan-2-ol (50.0 mg, 0.1675 mmol) in DMF (2 mL) was added Sodium thiomethoxide (47.0 mg, 0.67 mmol). The reaction mixture was stirred at 120°C for 2 h. The reaction was monitored by TLC. After the reaction was completed, the residue was purified by prep-HPLC (columns: Gemini 5 um C18 150 × 21.2 mm, mobile phase: ACN - H₂O (0.1% FA), gradient: 35 - 90, 9.0 min) to afford compound **111.**

### Example 12 - Preparation of 5-[(E)-2-(2-fluorophenyl) ethenyl]-2-isopropylpyridin-3-ol (Compound 112)

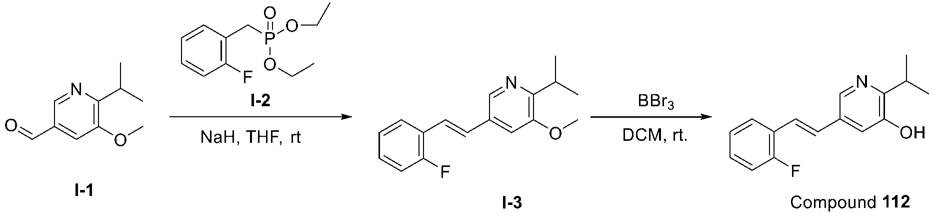

To a solution of diethyl [(2-fluorophenyl)methyl]phosphonate (131.9 mg, 0.536 mmol) in THF (5 mL) was added NaH (26.8 mg, 0.670 mmol, 60% in oil), then the reaction mixture was stirred at 25°C for 1 h. The mixture was added 6-isopropyl-5-methoxypyridine-3-carbaldehyde (80.0 mg, 0.446 mmol) then the reaction mixture was stirred at 25°C for 2 h. The mixture was added water (10 mL) and extracted with EtOAc (20 mL × 3). The organic phases were combined and washed with brine, dried over Na₂SO₄, filtered out and concentrated in vacuo. The solvent was removed under reduced pressure. The residue was purified by silica gel chromatography (eluting with PE/EtOAc from 100/0 to 95/5 in 20 mins) to afford 5-[(E)-2-(2-fluorophenyl) ethenyl]-2-isopropyl-3-methoxypyridine ethenyl] pyridine. LCMS (ESI): calcd for C₁₇H₁₈FNO [M+H]⁺ m/z 272.1, found 272.1.

To a solution of 5-[(E)-2-(2-fluorophenyl)ethenyl]-2-isopropyl-3-methoxypyridine (60.0 mg, 0.221 mmol) in DCM (5 mL) was added BBr₃ (554.0 mg, 2.21 mmol), then the mixture was stirred at 25°C for 16 h. The solution was added water (10 mL) and extracted with DCM (10 mL × 3). The organic phases were combined and washed with aq. NaHCO₃ and brine, dried over Na₂SO₄, filtered out and concentrated in vacuo. The residue was purified by prep-HPLC (eluting with H₂O (0.1% NH₃.H₂O)/MeCN from 70/30 to 5/95 in 40 mins) to afford compound **112.**

### Example 13 - Preparation of (E)-5-(3-fluorostyryl)-2-isopropylpyridin-3-ol (Compound 113)

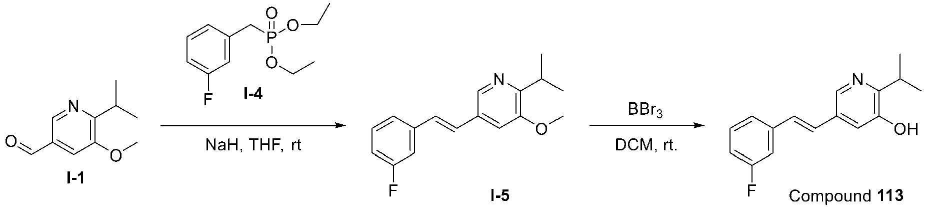

To a solution of diethyl [(3-fluorophenyl)methyl]phosphonate (89.0 mg, 0.362 mmol) in THF (5 mL) was added NaH (22.0 mg, 0.558 mmol, 60% in oil), then the reaction mixture was stirred at 25°C for 1 h. The mixture was added 6-isopropyl-5-methoxypyridine-3-carbaldehyde (50.0 mg, 0.279 mmol) then the reaction mixture was stirred at 25°C for 2 h. The mixture was added water (10 mL) and extracted with EtOAc (20 mL × 3). The organic phases were combined and washed with brine, dried over Na₂SO₄, filtered out and concentrated in vacuo. The residue was purified by silica gel chromatography (eluting with PE/EtOAc from 100/0 to 95/5 in 20 mins) to afford (E)-5-(3-fluorostyryl)-2- isopropyl-3-methoxypyridine. LCMS (ESI): calced for C₁₇H₁₉FNO [M+H]⁺ m/z 272.1, found 272.1.

To a solution of (E)-5-(3-fluorostyryl)-2-isopropyl-3-methoxypyridine (50.0 mg, 0.175 mmol) in DCM (5 mL) was added BBr₃ (554.0 mg, 2.21 mmol), then the mixture was stirred at 25°C for 16 h. The solution was added water (10 mL) and extracted with DCM (10 mL × 3). The organic phases were combined and washed with NaHCO₃ (aq.) and brine, dried over Na₂SO₄, filtered out and concentrated in vacuo. The solvent was removed under reduced pressure. The residue was purified by prep-HPLC (columns: Gemini 5 um C18 150 × 21.2 mm, mobile phase: ACN - H₂O (0.1% FA), gradient: 20 - 95, 10.0 min) to afford compound **113.**

### Example 14 - Preparation of 5-[(E)-2-(4-fluorophenyl)ethenyl]-2-isopropylpyridin-3-ol (compound 114)

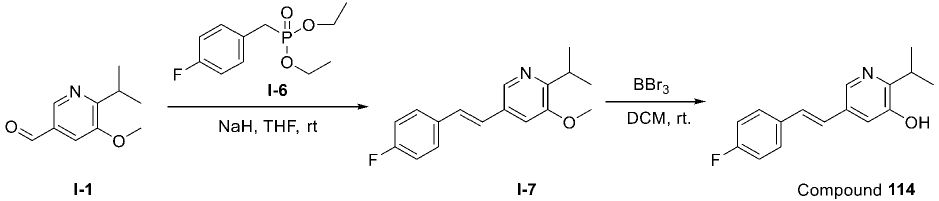

To a solution of diethyl [(4-fluorophenyl)methyl]phosphonate (54.0 mg, 0.218 mmol) in THF (5 mL) was added NaH (60%) (16.0 mg, 0.338 mmol). After 30 min, 6-isopropyl-5-methoxypyridine-3-carbaldehyde (30.0 mg, 0.167 mmol) was added. The reaction mixture was stirred at 25°C for 2 h. LCMS showed the desired MS was detected. The mixture was added water (10 mL) and extracted with EtOAc (20 mL × 3). The organic phases were combined and washed with brine, dried over Na₂SO₄, filtered out and concentrated in vacuo. The residue was purified by silica gel chromatography (eluting with PE/EtOAc from 100/0 to 95/5 in 20 mins) to afford 5-[(E)-2-(4-fluorophenyl) ethenyl]-2-isopropyl-3-methoxypyridine. LCMS (ESI): calcd for C₁₇H₁₉FNO [M+H]⁺ m/z 272.1, found 272.1.

To a solution of 5-[(E)-2-(4-fluorophenyl)ethenyl]-2-isopropyl-3-methoxypyridine (40.0 mg, 0.133 mmol) in DCM (5 mL) was added BBr₃ (332.0 mg, 1.33 mmol), then the mixture was stirred at 25°C for 16 h. The solution was added water (10 mL) and extracted with DCM (10 mL × 3). The organic phases were combined and washed with NaHCO₃ (aq.) and brine, dried over Na₂SO₄, filtered out and concentrated in vacuo. The solvent was removed under reduced pressure. The residue was purified by prep-HPLC (columns: Gemini 5 um C18 150 × 21.2 mm, mobile phase: ACN - H₂O (0.1% FA), gradient: 20 - 95, 10.0 min) to afford compound **114.**

### Example 15 - Preparation of 2-isopropyl-5-[(E)-2-(2,4,6-trifluorophenyl)ethenyl] pyridin-3-ol (Compound 115)

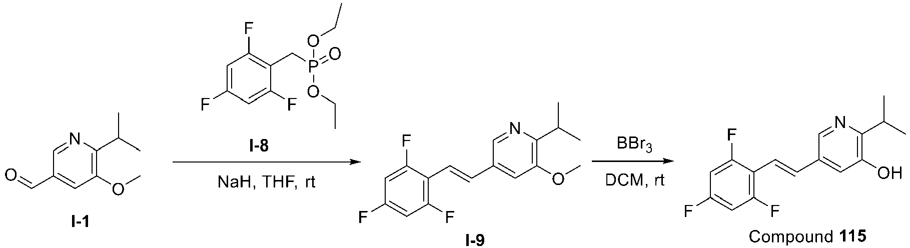

To a solution of diethyl [(2,4,6-trifluorophenyl) methyl] phosphonate (113.4 mg, 0.402 mmol) in THF (4 mL) was added NaH (20.1 mg, 0.502 mmol, 60% in oil), After 30 min, then 6-isopropyl-5-methoxypyridine-3-carbaldehyde (60.0 mg, 0.334 mmol) in THF (1 ml) was added at 0°C dropwise. The reaction was stirred at 25°C for 2 h. The LCMS showed the desired MS was detected. The solution was added water (10 mL) and extracted with EtOAc (20 mL × 3). The organic phases were combined and washed with brine, dried over Na₂SO₄, filtered out and concentrated in vacuo. The residue was purified by reversed-phase column (eluting with ACN - H₂O (0.1% FA) from 0-100 in 30 mins) to afford 2-isopropyl-3-methoxy-5-[(E)-2-(2,4,6-trifluorophenyl) ethenyl] pyridine. LCMS (ESI): calcd for C₁₇H₁₇F₃NO [M+H]⁺ m/z 308.2, found 308.2.

To 2-isopropyl-3-methoxy-5-[(E)-2-(2,4,6-trifluorophenyl) ethenyl] pyridine (60.0 mg, 0.166 mmol) was added BBr₃ (5 mL, 1M in DCM) at 0°C, then the reaction was stirred at 25°C for 16 h. The LCMS showed the desired MS was detected. The solution was quenched with ice water, adjusted with aq. NaHCO₃, extracted with DCM (10 mL × 3). The organic phases were washed with and brine, dried over Na₂SO₄, filtered out and concentrated in vacuo. The crude was purified on reversed-phase column directly (eluting with ACN - H₂O (0.1% FA) form 0-100 in 30 mins) to afford compound **115.**

### Example 16 - Preparation of (E)-5-(2,4-difluorostyryl)-2-isopropylpyridin-3-ol (Compound 116)

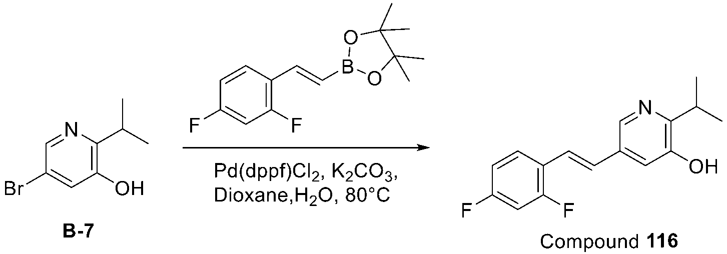

To a solution of 2-[(E)-2-(2,4-difluorophenyl)ethenyl]-4,4,5,5-tetramethyl-1,3,2-dioxaborolane (20.0 mg, 0.0752 mmol) in 1,4-dioxane / H₂O (4 mL/1 mL) was added 5-bromo-2-isopropylpyridin-3-ol (17.9 mg, 0.0826 mmol), Pd(dppf)Cl₂ (5.5 mg, 0.0074 mmol) and K₂CO₃ (20.8 mg, 0.1504 mmol). The reaction mixture was stirred at 80°C for 12 h under nitrogen. The LCMS showed the desired MS was detected. The solvent was removed by vacuum. The residue was quenched with water, extracted with EtOAc, the organic layer was washed with brine, dried over Na₂SO₄, filtered and concentrated. The crude product was purified by prep-TLC (eluting with PE/EtOAc from 100/0 to 75/25 in 30 mins) to afford compound 116.

### Example 17 - Preparation of 2-isopropyl-5-[(E)-2-phenylethenyl] pyridin-3-amine (Compound 117)

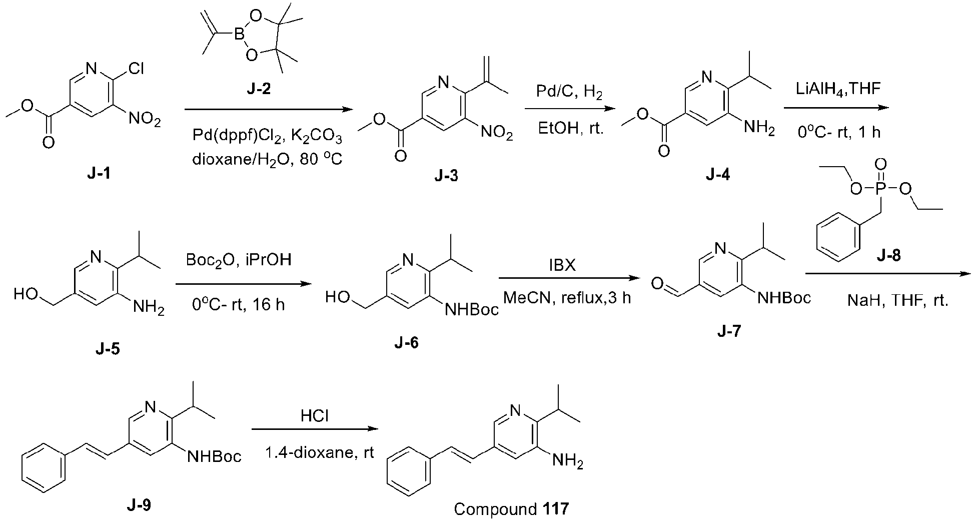

To a solution of methyl 6-chloro-5-nitropyridine-3-carboxylate (2.0 g, 9.23 mmol) in 1,4-dioxane (20 mL) and H₂O (4 mL) was added 4,4,5,5-tetramethyl-2-(prop-1-en-2-yl)-1,3,2-dioxaborolane (1.9 g, 11.1 mmol), Pd(dppf)Cl₂ (676.0 mg, 0.923 mmol) and K₂CO₃ (2.6 g, 18.46 mmol), then the mixture was stirred at 80°C for 2 h. The solution was added water (20 mL) and extracted with EtOAc (50 mL × 3). The organic phases were combined and washed with brine, dried over Na₂SO₄, filtered and concentrated in vacuo. The solvent was removed under reduced pressure. The residue was purified by silica gel chromatography (eluting with PE/EtOAc from 100/0 to 90/10) to afford methyl 5-nitro-6-(prop-1-en-2-yl) pyridine-3-carboxylate. LCMS (ESI): calcd for C₁₀H₁₀N₂O₄ [M+H]⁺ m/z 223.1, found 223.0.

To a solution of methyl 5-nitro-6-(prop-1-en-2-yl)pyridine-3-carboxylate (1.7 g, 7.56 mmol) in EtOH (16.8 mL) was added Pd/C (336.0 mg), then the mixture was purged with H₂ under 2 atm, and stirred at 25°C for 12 h. The solution was filtered, the cake was washed with EtOH (10 mL × 3), and the filtrate was combined. The resolution was removed under reduced pressure to afford methyl 5-amino-6-isopropylpyridine-3-carboxylate. LCMS (ESI): calcd for C₁₀H₁₄N₂O₂ [M+H]⁺ m/z 195.2, found 195.1.

To a solution of methyl 5-amino-6-isopropylpyridine-3-carboxylate (1.0 g, 5.1 mmol) in THF (10 mL) was added LiAlH₄ (230.0 mg, 6.18 mmol) at 0°C, then the mixture was stirred at 0°C for 1 h. The solution was added water (10 mL) and extracted with EtOAc (20 mL × 3). The organic phases were combined and washed with brine, dried over Na₂SO₄, filtered and concentrated in vacuo. The resolution was removed under reduced pressure to afford (5-amino-6-isopropylpyridin-3-yl) methanol. LCMS (ESI) calced for C₉H₁₄N₂O [M+H]⁺ m/z 167.1, found 167.1.

To a solution of (5-amino-6-isopropylpyridin-3-yl)methanol (900.0 mg, 5.42 mmol) in i-PrOH (9 mL) was added Boc₂O (1.8 g, 8.13 mmol), then the mixture was stirred at 25°C for 16 h. The solution was concentrated in vacuo to remove solvent. The residue was added water (20 mL) and extracted with EtOAc (50 mL × 2). The organic phases were combined and washed with brine, dried over Na₂SO₄, filtered and concentrated in vacuo. The resolution was removed under reduced pressure to afford tert-butyl N-[5-(hydroxymethyl)-2-isopropylpyridin-3-yl] carbamate. LCMS (ESI): calcd for C₁₄H₂₂N₂O₃ [M+H]⁺ m/z 267.3, found 267.2.

To a solution of tert-butyl N-[5-(hydroxymethyl)-2-isopropylpyridin-3-yl]carbamate (1.0 g, 3.76 mmol) in MeCN (15 mL) was added IBX (1.4 g, 4.88 mmol), then the mixture was stirred at 95°C for 3 h. The mixture was filtered through a filter paper and the cake was washed with MeCN (10 mL × 3), and the filtrate was combined. The solvent was removed under reduced pressure. The residue was purified by silica gel chromatography (eluting with PE/EtOAc from 100/0 to 95/5 in 10 mins) to afford tert-butyl N-(5-formyl-2-isopropylpyridin-3-yl) carbamate. LCMS (ESI): calcd for C₁₄H₂₀N₂O₃ [M+H]⁺ m/z 265.1, found 265.1.

To a solution of diethyl benzyl phosphonate (545.5 mg, 2.27 mmol) in THF (10 mL) was added NaH (68.1 mg, 2.84 mmol, 60% in oil), After 1h at 25°C, tert-butyl N-(5-formyl-2-isopropylpyridin-3-yl) carbamate (500.0 mg, 1.89 mmol) dissolved in THF (5 ml) was added and stirred at 40°C for 2 h. The solution was added water (10 mL) and extracted with EtOAc (20 mL × 3). The organic phases were combined and washed with brine, dried over Na₂SO₄, filtered and concentrated in vacuo. The residue was purified by silica gel chromatography (eluting with PE/EtOAc from 90/10 to 80/20 in 15 mins) to afford tert-butyl N-{2-isopropyl-5-[(E)-2-phenylethenyl]pyridin-3-yl}carbamate. LCMS (ESI) calced for C₂₁H₂₆N₂O₂ [M+H]⁺ m/z 339.2, found 339.2.

A solution of tert-butyl N-{2-isopropyl-5-[(E)-2-phenylethenyl]pyridin-3-yl}carbamate(300.0 mg, 0.886 mmol) in 1,4-dioxane (5 mL) was added HCl (1 mL, 12 M) at 0 °C, then the reaction mixture was stirred at 25°C for 2 h. The solution was concentrated in vacuo to remove the 1,4-dioxane. The residue was dissolved with water (5 ml) and adjusted the pH = 9 with aq. NaHCO₃. Then the mixture was extracted by EtOAc (10 ml × 3). The organic phases were combined and washed with brine, dried over Na₂SO₄, filtered and concentrated in vacuo. The residue was purified by silica gel chromatography (eluting with PE/EtOAc from 90/10 to 80/20 in 15 mins) to afford compound **117.**

### Example 18 - Preparation of 2-isopropyl-5-(quinolin-3-yl) pyridin-3-amine (Compound 118)

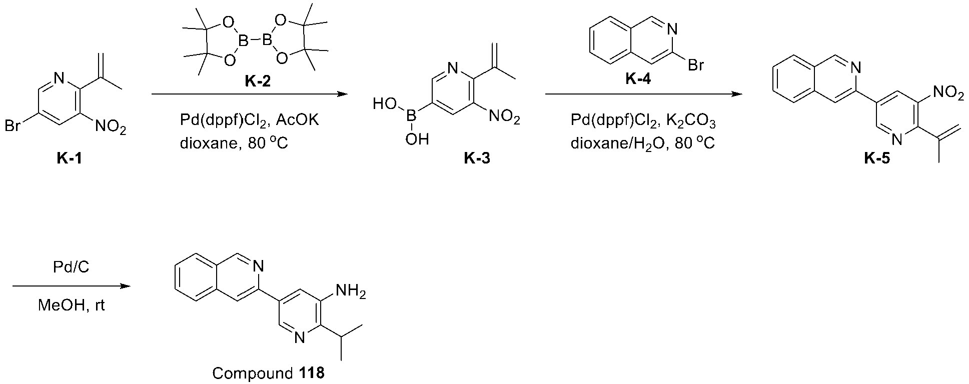

To a solution of 5-bromo-3-nitro-2-(prop-1-en-2-yl) pyridine (200.0 mg, 0.823 mmol), tetramethyl-1,3,2-dioxaborolan-2-yl)-1,3,2-dioxaborolane (250.7 mg, 0.987 mmol), Pd(dppf)Cl₂ (60.2 mg, 0.082 mmol) and AcOK (161.5 mg, 1.64 mmol) in 1,4-dioxane (5 ml). The reaction mixture was stirred at 80°C for 16 h under Ar₂. LCMS showed the desired MS was detected. 2-bromoquinoline (100.0 mg, 0.48 mmol), Pd(dppf)Cl₂ (35.2 mg, 0.048 mmol), K₂CO₃ (265.1 mg, 1.922 mmol) in H₂O (1 mL) was added. The reaction mixture was stirred at 80°C for another 6 h under Ar₂. The mixture was added water (30 mL) and extracted with EtOAc (20 mL × 3). The organic phases were combined and washed with brine, dried over Na₂SO₄, filtered out and concentrated in vacuo. The residue was purified by silica gel chromatography (eluting with PE/EtOAc from 100/0 to 80/20 in 30 mins) to afford 3-(5-nitro-6-(prop-1-en-2-yl)pyridin-3-yl)isoquinoline.

To a solution of 3-(5-nitro-6-(prop-1-en-2-yl)pyridin-3-yl)isoquinoline (80.0 mg, 0.275 mmol) in MeOH (10 mL) was added Pd/C (40.0 mg, 10% Pd). The reaction mixture was stirred at 25°C for 16 h under H₂ (1 atm). The mixture was filtration and collect filtrate. The solvent was removed under reduced pressure. The residue was purified by prep-HPLC (columns: Gemini 5 um C18 150 × 21.2 mm, mobile phase: ACN - H₂O (0.05% NH₃.H₂O), gradient: 30 - 70, 9.0 min) to afford compound **118.**

### Example 19 - Preparation of 2-isopropyl-5-(quinolin-2-yl)pyridin-3-amine (Compound 119)

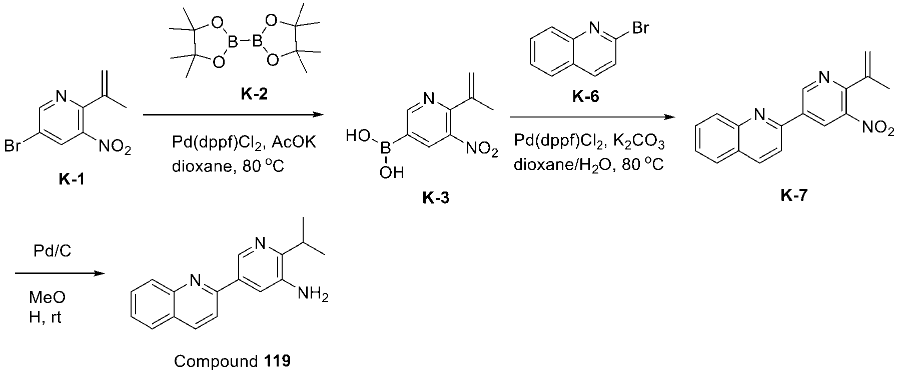

To a solution of 5-bromo-3-nitro-2-(prop-1-en-2-yl) pyridine (200.0 mg, 0.823 mmol), tetramethyl-1,3,2-dioxaborolan-2-yl)-1,3,2-dioxaborolane (250.7 mg, 0.987 mmol), Pd(dppf)Cl₂(60.2 mg, 0.082 mmol) and AcOK (161.5 mg, 1.64 mmol) in 1,4-dioxane (5 mL). The reaction mixture was stirred at 80°C for 16 h under Ar₂. LCMS showed the desired MS was detected. 2-bromoquinoline (100.0 mg, 0.48 mmol), Pd(dppf)Cl₂ (35.2 mg, 0.048 mmol), K₂CO₃ (265.1mg, 1.922 mmol) in H₂O (1 mL) was added. The reaction mixture was stirred at 80°C for another 6 h under Ar₂. The mixture was added water (30 mL) and extracted with EtOAc (20 mL × 3). The organic phases were combined and washed with brine, dried over Na₂SO₄, filtered out and concentrated in vacuo. The residue was purified by silica gel chromatography (eluting with PE/EtOAc from 100/0 to 80/20 in 30 mins) to afford 2-(5-nitro-6-(prop-1-en-2-yl)pyridin-3-yl)isoquinoline.

To a solution of 2-[5-nitro-6-(prop-1-en-2-yl) pyridin-3-yl] quinoline (80.0 mg, 0.273 mmol) in MeOH (10 mL) was added Pd/C (40.0 mg, 10% Pd). The reaction mixture was stirred at 25°C for 16 h under H₂. The mixture was filtration and collect filtrate. The solvent was removed under reduced pressure. The residue was purified by prep-HPLC (columns: Gemini 5 um C18 150 × 21.2 mm, mobile phase: ACN - H₂O (0.05% NH₃.H₂O), gradient: 30 - 95, 10.0 min) to afford compound **119.**

### Example 20 - Preparation of 2-isopropyl-5-(quinolin-3-yl) pyridin-3-amine (Compound 120)

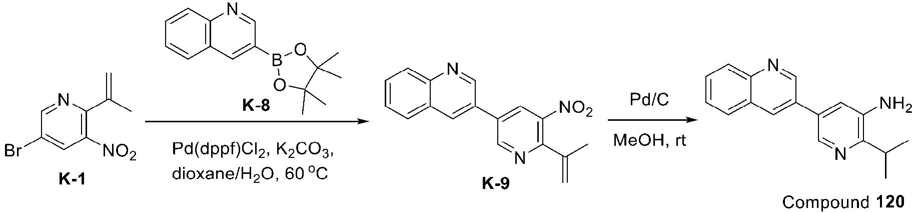

To a solution of 3-(4,4,5,5-tetramethyl-1,3,2-dioxaborolan-2-yl) quinoline (200.0 mg, 0.82 mmol), 5-bromo-3-nitro-2-(prop-1-en-2-yl)pyridine (228.6 mg, 0.90 mmol), Pd(dppf)Cl₂ (57.4 mg, 0.078 mmol) and K₂CO₃ (216.7 mg, 1.57 mmol) in 1,4-dioxane:H₂O=10:1 (11 mL). The reaction mixture was stirred at 80°C for 12 h under N₂. LCMS showed the desired MS was detected. The mixture was added water (10 mL) and extracted with EtOAc (20 mL × 3). The organic phases were combined and washed with brine, dried over Na₂SO₄, filtered out and concentrated in vacuo. The residue was purified by silica gel chromatography (eluting with PE/EtOAc from 100/0 to 90/10 in 20 mins) to afford 2-[5-nitro-6-(prop-1-en-2-yl) pyridin-3-yl] quinoline. LCMS (ESI) calcd for C₁₇H₁₄N₃O₂ [M+H]⁺ m/z 292.1, found 292.2.

To a solution of 2-[5-nitro-6-(prop-1-en-2-yl) pyridin-3-yl] quinoline (80.0 mg, 0.27 mmol) in MeOH (10 ml) was added Pd/C (40.0 mg, 10% Pd). The reaction mixture was stirred at 25°C for 16 h under H₂ (1 atm). The mixture was filtration and collect filtrate. The solvent was removed under reduced pressure. The residue was purified by prep-HPLC (columns: Gemini 5 um C18 150 × 21.2 mm, mobile phase: ACN - H₂O (0.05% NH₃.H₂O), gradient: 30 - 60, 9.0 min) to afford compound **120.**

### Example 21 - Preparation of 5-(1,3-benzoxazol-2-yl)-2-isopropylpyridin-3-amine (Compound 121)

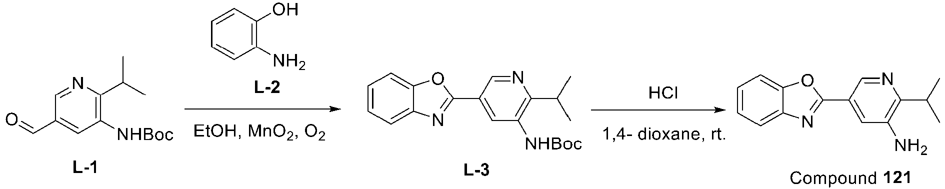

To a solution of tert-butyl N-(5-formyl-2-isopropylpyridin-3-yl)carbamate (200.0 mg, 0.757 mmol) in EtOH (5 mL) was added 2-aminophenol (99.1 mg, 0.908 mmol) and MnO₂ (263.1 mg, 3.027 mmol), then the reaction mixture was purged with O₂ for 3 times and stirred at 50°C for 12 h. The mixture was filtrated to remove MnO₂. The solution was added water (10 mL) and extracted with EtOAc (20 mL × 3). The organic phases were combined and washed with brine, dried over Na₂SO₄, filtered out and concentrated in vacuo. The solvent was removed under reduced pressure. The residue was purified by silica gel chromatography (eluting with PE/EtOAc from 100/0 to 90/10 in 10 mins) to afford tert-butyl N-[5-(1,3-benzoxazol-2-yl)-2- isopropylpyridin-3-yl]carbamate. LCMS (ESI): calcd for C₂₀H₂₃N₃O₃ [M+H]⁺ m/z 354.2, found 354.1.

A solution of tert-butyl N-[5-(1,3-benzoxazol-2-yl)-2-isopropylpyridin-3-yl]carbamate (150.0 mg, 0.424 mmol) in 1,4-dioxane (5 mL) was added HCl (0.5 mL, 12M) at 0 °C, then the reaction mixture was stirred at 25°C for 16 h. The solution was concentrated in vacuo to remove the 1,4-dioxane. The residue was dissolved with water (5 ml) and adjusted the pH = 9 with aq. NaHCO₃. Then the mixture was extracted by EtOAc (10 ml × 3). The organic phases were combined and washed with brine, dried over Na₂SO₄, filtered out and concentrated in vacuo. The residue was purified by Prep-TLC to afford compound **121.**

### Example 22 - Preparation of 5-(benzo[d]oxazol-2-yl)-2-isopropyl-3-methoxyphenol (Compound 122)

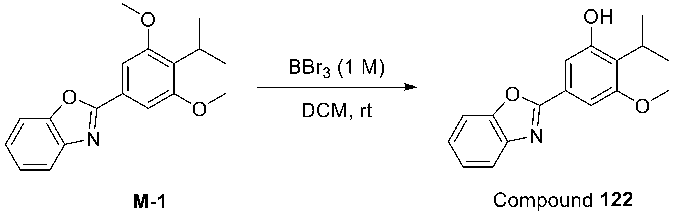

To a solution of 2-(4-isopropyl-3,5-dimethoxyphenyl)benzo[d]oxazole (100.0 mg, 0.3363 mmol) in DCM (3 mL) was added BBr₃ (0.6 mL, 1 M in DCM). The reaction mixture was stirred at 25°C for 2 h. The reaction was quenched with water, extracted with DCM (10 mL × 3). The organic phases were combined, washed with brine, dried over Na₂SO₄, filtered out and concentrated in vacuo. The residue was purified by Prep-HPLC (columns: Gemini 5 um C18 150 × 21.2 mm, mobile phase: ACN - H₂O (0.1% FA), gradient: 50 - 95, 10.0 min) to afford compound **122.**

### Example 23 - Preparation of (E)-2-(4-methoxy-6-styrylpyridazin-3-yl)propan-2-ol (Compound 123)

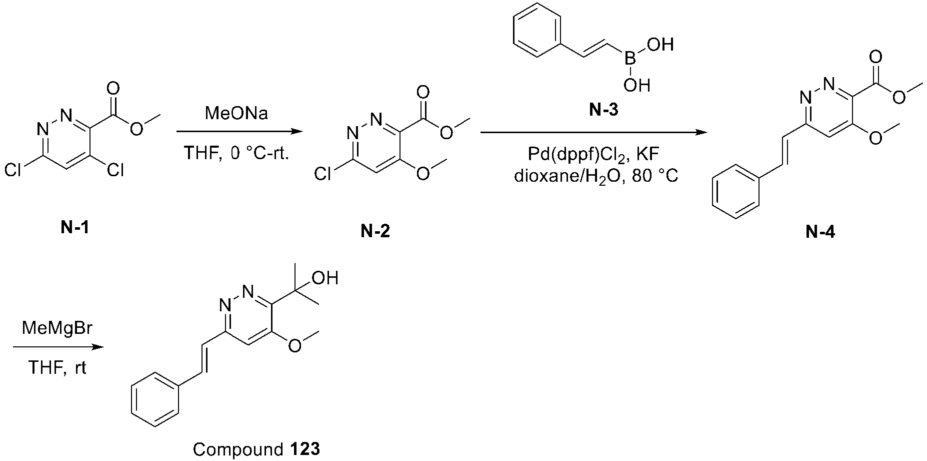

Sodium methoxide (30wt% in methanol, 2.7 mL) was added to a stirred solution of methyl 4,6-dichloropyridazine-3-carboxylate (3.0 g, 514.49 mmol) in THF (100 mL) cooled to 0 °C. The reaction mixture was stirred at room temperature overnight and LCMS showed the desired MS was detected, then poured into 1N HCl (20 mL). The resulting solution was then neutralized by saturated NaHCO₃. EtOAc (100 mL) was added and the layers are separated. The aqueous layer was extracted twice with EtOAc (100 mL) and the combined extracts were washed with brine (50 mL), dried (NaSO₄) and evaporated. The residue was then purified by flash chromatography (eluting with PE/EtOAc from 100/0 to 60/40 in 30 mins) to give methyl 6-chloro-4-methoxypyridazine-3-carboxylate. LCMS (ESI): calcd for C₇H₇ClN₂O₃ [M+H]⁺ m/z 203.0, found 203.1.

To a solution of methyl 6-chloro-4-methoxypyridazine-3-carboxylate (970.0 mg, 4.79 mmol) in 1,4-dioxane / H₂O (50 mL/10 mL) was added (E)-styrylboronic acid (919.3 mg, 6.21 mmol), Pd(dppf)Cl₂ (349.8 mg, 0.48 mmol) and KF (554.3 mg, 9.56 mmol). The reaction mixture was stirred at 80°C for 12 h under nitrogen. The LCMS showed the desired MS was detected. The solvent was removed by vacuum. The residue was extracted with EtOAc (200 mL×3), the organic layer was washed with brine, dried over Na₂SO₄, and concentrated to get crude product which was purified by flash chromatography (eluting with PE/EtOAc from 100/0 to 50/50 in 30 mins) to afford methyl (E)-4-methoxy-6-styrylpyridazine-3-carboxylate. LCMS (ESI): calcd for C₁₅H₁₄N₂O₃ [M+H]⁺ m/z 271.1, found 271.2.

To a solution of methyl (E)-4-methoxy-6-styrylpyridazine-3-carboxylate (560.0 mg, 2.07 mmol) in THF (50 mL) was added MeMgBr (1.38 ml, 4.14 mmol, 3M in hexane) at 0 °C. The reaction was stirred at rt for 2 h under nitrogen. LCMS showed the desired MS was detected. The solvent was quenched with saturated aq. NH₄Cl. The solvent was extracted with EtOAc (150 mL×3). The combined organic layers was washed with brine (50 mL), dried over anhydrous Na₂SO₄, filtered, and concentrated under vacuum. The residue was then purified by flash chromatography (eluting with PE/EtOAc from 100/0 to 50/50 in 30 mins) to give compound **123.**

### Example 23 - Preparation of (E)-3-isopropyl-6-styrylpyridazin-4-ol (Compound 124)

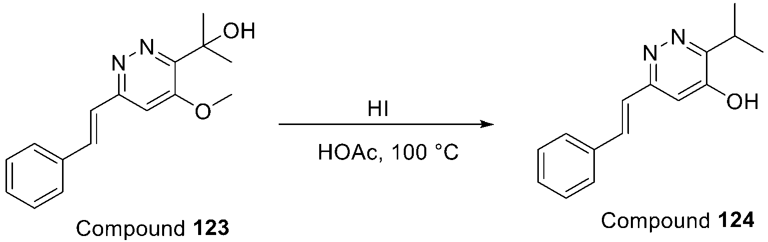

To a solution of compound **123** (90.0 mg, 0.33 mmol) in HOAc (3 mL) was added HI (57 wt% in water, 0.26 mL) stirred for 1h at 100 °C. LCMS showed the desired MS was detected The solvent is removed. Water (5 mL) is added to the residue and the mixture is extracted with EtOAc (3 × 10 mL). The combined organic layers are washed with brine (5 mL), dried over anhydrous Na₂SO₄, filtered, and concentrated under vacuum. The residue was purified by prep-HPLC (columns: Gemini 5 um C18 150 × 21.2 mm, mobile phase: ACN - H₂O (0.1% FA), gradient: 20 - 95, 9.0 min) to afford compound **124.**

### Example 25 - Preparation of 3-fluoro-5-[(E)-2-phenylethenyl]-2-propylphenol (Compound 125)

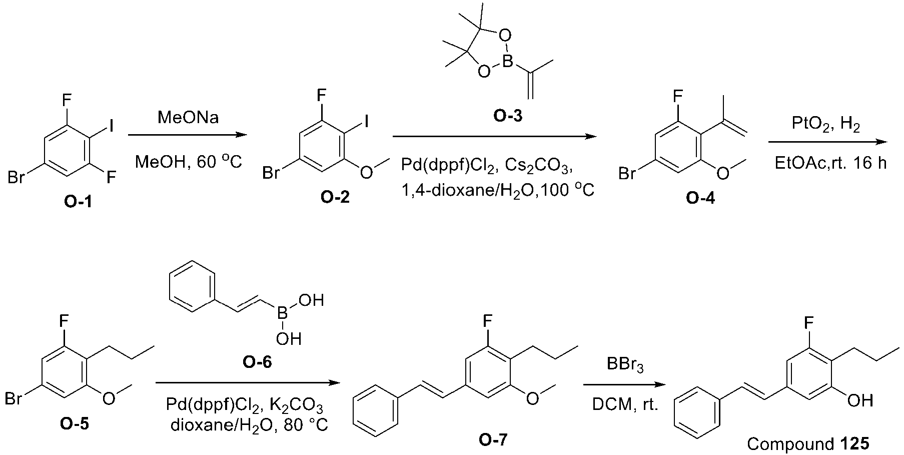

To 5-bromo-1,3-difluoro-2-iodobenzene (2.0 g, 6.3 mmol) in MeOH (20 ml) was added MeONa (30 % in MeOH, 2.1 ml). The reaction was stirred at 60°C for 16 h. The reaction solvent was removed by vacuum to afford white solid. The solvent was diluted with DCM (20 mL) and water (20 mL). The aqueous phase was extracted with DCM (20 mL). The organic phase was combined and washed with brine, dried over anhydrous NaSO₄ and concentrated to get crude product which was purified by flash chromatography (eluting with PE/EtOAc from 100/0 to 98/2 in 30 mins) to afford 5-bromo-1-fluoro-2-iodo-3-methoxybenzene. ¹H NMR (400 MHz, CDCl₃, ppm) 6.91 (dd, J= 7.2. 2.0 Hz, 1H), 6.76 (t, *J* = 1.6 Hz, 1H), 3.90 (s, 3H).

To a solution of 5-bromo-1-fluoro-2-iodo-3-methoxybenzene (2.0 g, 6.04 mmol) in 1,4-dioxane / H₂O (40 mL/4 mL) was added 4,4,5,5-tetramethyl-2-(prop-1-en-2-yl)-1,3,2-dioxaborolane (1.5 g, 9.07 mmol), Pd(dppf)Cl₂ (442.2 mg, 0.604 mmol) and K₂CO₃ (3.9 g, 12.08 mmol). The reaction mixture was stirred at 100°C for 16 h under nitrogen. The solvent was removed by vacuum. The residue was purified by flash chromatography (eluting with PE/EA from 100/0 to 98/2 in 30 mins) to afford 5-bromo-1-fluoro-3-methoxy-2-(prop-1-en-2-yl) benzene. ¹H NMR (400 MHz, CDCl₃, ppm) 6.90 (dd, *J =* 8.4, 1.6 Hz, 1H)., 6.82 (t, *J =* 1.6 Hz 1H), 5.35 (dd, *J* = 3.2, 1.6 Hz, 1H), 4.97-4.96(m, 1H), 3.82 (s, 3H), 2.01(s, 3H).

To a solution of 5-bromo-1-fluoro-3-methoxy-2-(prop-1-en-2-yl) benzene (200.0 mg, 0.816 mmol) in EtOAc (5 ml) was added Pt/C (20.0 mg, 10wt%). The reaction mixture was stirred at 25°C for 16 h under H₂ (1atm). The solvent was removed by vacuum. The residue was purified by flash chromatography (eluting with PE/EA from 100/0 to 95/5 in 30 mins) to afford 5-bromo-1-fluoro-3-methoxy-2-propylbenzene. ¹H NMR (400 MHz, CDCl₃, ppm) 6.84 (dd, *J* = 8.8, 1.6 Hz, 1H)., 6.77 (t, *J* = 1.6 Hz 1H), 3.83-3.80 (m, 3H), 2.55 (td, *J* = 7.6, 1.6 Hz, 2H), 1.51 (dt, *J* = 14.8, 7.6 Hz, 2H), 0.91 (t, *J* = 8.0 Hz, 3H).

To a solution of 5-bromo-1-fluoro-3-methoxy-2-propylbenzene (50.0 mg, 0.202 mmol) in 1,4-dioxane / H₂O (2 mL/0.2 mL) was added [(E)-2-phenylethenyl] boranediol (44.9 mg, 0.304 mmol), Pd(dppf)Cl₂ (11.8 mg, 0.020 mmol) and K₂CO₃ (55.9 mg, 0.405 mmol). The reaction mixture was stirred at 80°C for 16 h under nitrogen. The LCMS showed the desired MS was detected. The solvent was removed by vacuum. The residue was purified by flash chromatography (eluting with PE/EA from 100/0 to 95/5 in 30 mins) to afford 1-fluoro-3-methoxy-5-[(E)-2-phenylethenyl]-2-propylbenzene. ¹H NMR (400 MHz, CD₃OD, ppm) 7.56 (d, *J* = 8.0 Hz, 2H), 7.37-7.33 (m, 2H), 7.27-7.23 (m, 1H), 7.16 -7.08 (m, 2H), 6.95-6.86 (m, 2H), 3.90 (s, 3H), 2.664-2.59 (m, 2H), 1.60-1.51 (m, 2H), 0.94 (t, *J* = 8.0 Hz, 3H).

To 1-fluoro-3-methoxy-5-[(E)-2-phenylethenyl]-2-propylbenzene (45.0 mg, 0.166 mmol) was added BBr₃ (1M in DCM,5 mL), then the reaction was stirred at 25°C for 16 h. The solution was added water (10 mL) and extracted with DCM (10 mL × 3). The organic phases were combined and washed with aq. NaHCO₃ and brine, dried over Na₂SO₄, filtered out and concentrated in vacuo. The solvent was removed under reduced pressure to afford crude. The crude was purified on reversed-phase column directly (eluting with ACN - H₂O (0.1% FA) from 0 to 100 in 30 mins) to afford compound **125.**

### Example 26 - Preparation of 6-isopropyl-2-phenyl-2H,3H-furo[2,3-b] pyridin-5-ol (Compound 127)

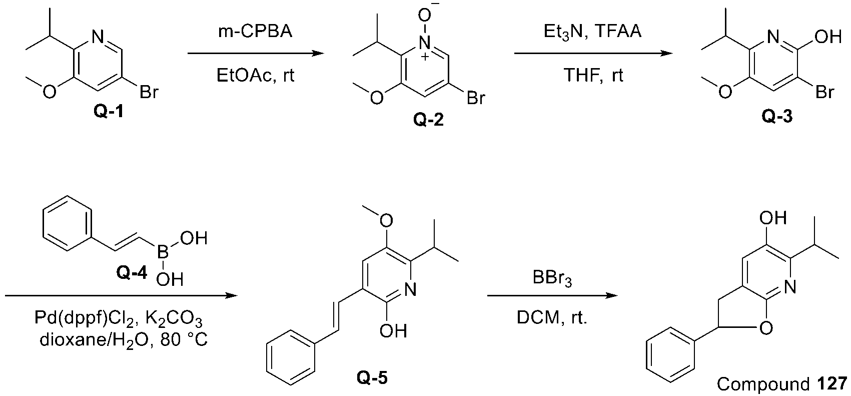

The m-CPBA (1.31 g, 7.61 mmol) was added at 0 °C to a stirred solution of 5-bromo-2-isopropyl-3-methoxypyridine (350.0 mg, 1.52 mmol) in ethyl acetate (5 mL). The resulting reaction mixture was stirred at room temperature for 16 h. The reaction mixture was quenched with saturated sodium bicarbonate solution (5 ml.) and the ethyl acetate layer was separated. The aqueous layer was extracted with 10% methanol in dichloromethane (5x10 mL). The combined organic layer was dried over anhydrousNa₂SO₄, filtered out and solvent was evaporated under reduced pressure to afford 5-bromo-2-isopropyl-3-methoxypyridin-1-ium-1-olate. LCMS (ESI): calcd for C₉H₁₂BrNO₂[M+H]⁺ m/z 248.2, found 248.2.

To a solution of 5-bromo-2-isopropyl-3-methoxypyridin-1-ium-1-olate (1.5 g, 6.10 mmol) in dry THF (45 ml) was added TFAA (5.1 g, 24.38 mmol) and triethylamine (4.5 ml, 32.4 mmol) at 0 °C, then the reaction was stirred at 0-10°C for 2 h. The solvent was evaporated under reduced pressure; the residue was dissolved in 100 ml. of dichloromethane and washed with water (25 ml ×2). The organic phases were combined and dried over Na₂SO₄, filtered out and concentrated in vacuo. The solution was purified on reversed-phase column directly (eluting with ACN - H₂O (0.1% FA) from 0-100 in 30 mins) to afford 3-bromo-6-isopropyl-5-methoxypyridin-2-ol. LCMS (ESI): calcd for C₉H₁₂BrNO₂ [M+H]⁺ m/z 246.1, found 246.1.

To a solution of 3-bromo-6-isopropyl-5-methoxypyridin-2-ol (80.0 mg, 0.325 mmol) in 1,4-dioxane / H₂O (2 mL/0.2 mL) was added [(E)-2-phenylethenyl] boranediol (72.1 mg, 0.488 mmol), Pd(dppf)Cl₂ (23.8 mg, 0.033 mmol) and K₂CO₃ (89.9 mg, 0.650 mmol). The reaction mixture was stirred at 80°C for 16 h under nitrogen. The LCMS showed the desired MS was detected. The solvent was removed by vacuum. The residue was purified by flash chromatography (eluting with PE/EtOAc from 100/0 to 80/20 in 30 mins) to afford 6-isopropyl-5-methoxy-3-[(E)-2-phenylethenyl] pyridin-2-ol. LCMS (ESI): calcd for C₁₇H₁₉NO [M+H]⁺m/z 270.2, found 270.2.

To 6-isopropyl-5-methoxy-3-[(E)-2-phenylethenyl] pyridin-2-ol (40.0 mg, 0.149 mmol) in DCM (5 ml) was added BBr₃ (1 mL), then the reaction was stirred at 25°C for 16 h. The solution was added water (10 mL) and extracted with DCM (20 mL × 3). The organic phases were combined and washed with aq. NaHCO₃ and brine, dried over Na₂SO₄, filtered out and concentrated in vacuo. The crude was purified by prep-HPLC (columns: Gemini 5 um C18 150 × 21.2 mm, mobile phase: ACN - H₂O (0.1% FA), gradient: 25 - 95, 11.5 min) to afford compound **127.**

### Example 27 - Preparation of (E)-4-hydroxy-3-isopropyl-1-methyl-6-styrylpyridin-2(1H)-one (Compound 128)

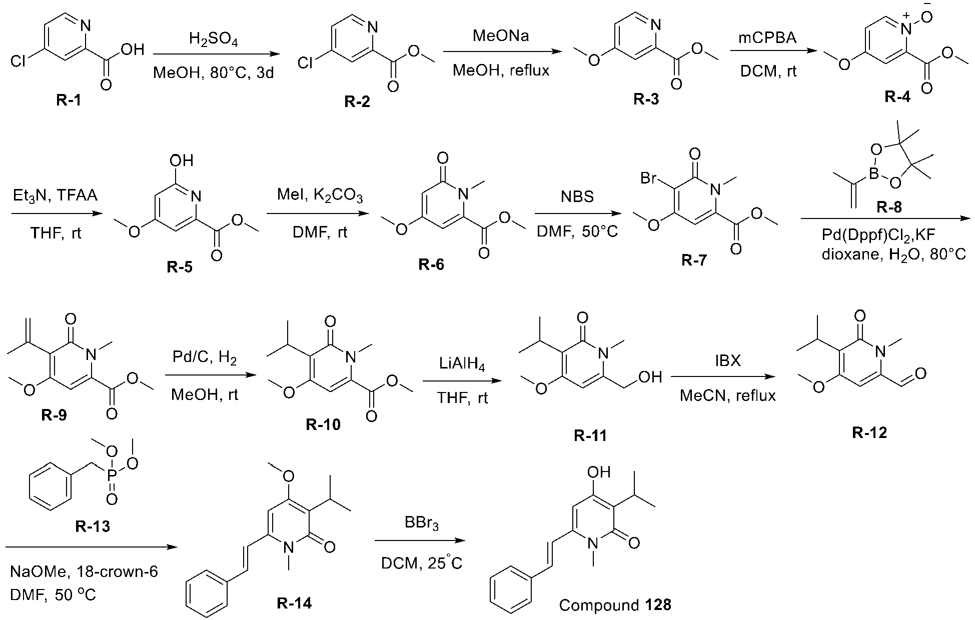

To a solution of 4-chloropicolinic acid (17.0 g, 108.28 mmol) in MeOH (800 mL) was added H₂SO₄ (4 mL, 18.4 mol/L) stirred for 72 h at 80 °C. The mixture was cooled to room temperature. The solvent concentrated under vacuum, saturated aq. NaHCO₃ was poured into and the mixture is extracted with EtOAc (4 × 800 mL). The combined organic layers are washed with brine (300 mL), dried over anhydrous Na₂SO₄, filtered, and concentrated under vacuum. The residue was then purified by flash chromatography (eluting with PE/EtOAc from 100/0 to 50/50 in 30 mins) to give methyl 4-methoxypicolinate and (eluting with PE/EtOAc from 100/0 to 10/90 in 30 mins) to give methyl 4-chloropicolinate (9.0 g, 52.32 mmol).

To a solution of methyl 4-chloropicolinate (9.0 g, 52.32 mmol) in MeOH (800 mL) was added Sodium methoxide (30 wt% in methanol, 7.8 g) stirred for 12 h at reflux. The mixture was cooled to room temperature and then poured into 1N HCI. The solvent is removed. Water (200 mL) is added to the residue and the mixture is extracted with EtOAc (4 × 500 mL). The combined organic layers are washed with brine (200 mL), dried over anhydrous Na₂SO₄, filtered, and concentrated under vacuum. The residue was then purified by flash chromatography (eluting with PE/EtOAc from 100/0 to 50/50 in 30 mins) to give 4-methoxypicolinate. LCMS (ESI): calcd for C₈H₁₀NO₃ [M+H]⁺ m/z 168.1, found 168.2.

To a solution of 4-methoxypicolinate (6.2 g, 37.13 mmol) in DCM (250 mL) was added mCPBA (77 %, 13.3 g, 59.54 mmol) portion at 0 °C and stirred at room temperature for 22 h. Additional mCPBA (77%, 5.0 g, 22.33 mmol) was added and the mixture was stirred for another 5 h. The reaction was diluted with ethyl acetate (300 mL) and washed with saturated aqueous sodium bicarbonate (100 mL). The separated aqueous phase was extracted with DCM/MeOH=9/1 (5×500 mL) and the combined organic lavers were dried over Na₂SO₄, filtered out and concentrated under reduced pressure. The resultant material was purified by silica gel column chromatography (0-10% methanol in dichloromethane) to provide 4-methoxy-2-(methoxycarbonyl)pyridine 1-oxide. LCMS (ESI): calcd for C₈H₁₀NO₄ [M+H]⁺ m/z 184.1, found 184.0.

To a solution of 4-methoxy-2-(methoxycarbonyl)pyridine 1-oxide (3.5 g, 19.12 mmol) and triethylamine (5.8 g. 57.43 mmol) in THF (200 mL) was added trifluoroacetic anhydride (12.0 g. 57.46 mmol) dropwise over 5 min at 0 °C. The mixture was stirred for another 4 h at 0 °C. After completion, the reaction was diluted with dichloromethane (5×500 mL) and extracted with a mixed solution of saturated aqueous sodium bicarbonate (200 mL) and brine (200 mL). The organic laver was separated and dried over anhydrous sodium sulfate, filtered out and concentrated under reduced pressure. The resultant crude material was purified by silica gel column chromatography (0-10% methanol in dichloromethane) to provide methyl 6-hydroxy-4-methoxypicolinate. LCMS (ESI): calcd for C₈H₉NO₄ [M+H]⁺ m/z 184.1, found 184.2.

To a mixture of methyl 6-hydroxy-4-methoxypicolinate (3.2 g, 17.52 mmol), potassium carbonate (3.6 g, 26.24 mmol) in DMF (150 mL) was added iodoethane (5.0 g. 35.00 mmol) and the mixture was stirred overnight at room temperature. After completion, Water (150 mL) is added to the residue and the mixture is extracted with EtOAc (3 × 200 mL). The combined organic layers are washed with brine (200 mL ×3), dried over anhydrous Na₂SO₄, filtered, and concentrated under vacuum. The residue was then purified by flash chromatography (eluting with PE/EtOAc from 100/0 to 60/40 in 30 mins) to give methyl 6-hydroxy-4-methoxypicolinate. LCMS (ESI): calcd for C₈H₁₀NO₄ [M+H]⁺ m/z 198.1, found 198.2.

To a solution of methyl 6-hydroxy-4-methoxypicolinate (1.7 g, 8.63 mmol) in DMF (100 mL) was added NBS (1.5 g, 8.42 mmol) stirred for 12 h at 50°C. The mixture was cooled to room temperature. After completion, Water (100 mL) is added to the residue and the mixture is extracted with EtOAc (3 × 100 mL). The combined organic layers are washed with brine (3 × 300 mL), dried over anhydrous Na₂SO₄, filtered, and concentrated under vacuum. The residue was then purified by flash chromatography (eluting with PE/EtOAc from 100/0 to 65/35 in 30 mins) to give methyl 5-bromo-4-methoxy-1-methyl-6-oxo-1,6-dihydropyridine-2-carboxylate. LCMS (ESI): calcd for C₉H₁₁BrNO₄ [M+H]⁺ m/z 276.0, found 276.1.

To a solution of methyl 5-bromo-4-methoxy-1-methyl-6-oxo-1,6-dihydropyridine-2-carboxylate (1.8 g, 6.52 mmol) in 1,4-dioxane:H₂O=5:1 (100 mL) was added 4,4,5,5-tetramethyl-2-(prop-1-en-2-yl)-1,3,2-dioxaborolane (1.3 g, 7.82 mmol), Pd(Dppf)Cl₂ (477.4 mg, 0.65 mmol) and KF (756.5 mg, 13.05 mmol) stirred for 12 h at 80°C under nitrogen. The mixture was cooled to room temperature. The solvent is removed. Water (100 mL) is added to the residue and the mixture is extracted with EtOAc (3 × 200 mL). The combined organic layers are washed with brine (50 mL), dried over anhydrous Na₂SO₄, filtered, and concentrated under vacuum. The residue was then purified by flash chromatography (eluting with PE/EtOAc from 100/0 to 70/30 in 30 mins) to give methyl 4-methoxy-1-methyl-6-oxo-5-(prop-1-en-2-yl)-1,6-dihydropyridine-2-carboxylate. LCMS (ESI) calcd for C₁₂H₁₆NO₄ [M+H]⁺ m/z 238.1, found 238.2.

To a solution of methyl 4-methoxy-1-methyl-6-oxo-5-(prop-1-en-2-yl)-1,6-dihydropyridine-2-carboxylate (420.0 mg, 1.76 mmol) in MeOH (50 mL) was added Pd/C (10%, 187.8 mg, 0.18 mmol) stirred for 1 h at room temperature under H₂ (1 atm). The mixture was filtered and concentrated under vacuum. The crude product (about 390.0 mg) is used without further purification in the next step. LCMS (ESI): calcd for C₁₂H₁₈NO₄ [M+H]⁺ m/z 240.1, found 240.2.

To a solution of methyl 5-isopropyl-4-methoxy-1-methyl-6-oxo-1,6-dihydropyridine-2-carboxylate (crude, about 390.0 mg) in THF (50 mL) was added LiAlH₄ (74.4 mg, 1.96 mmol) slowly stirred for 0.5 h at 0 °C. Water (20 mL) and 10% aq. NaOH (10 mL) were added to the mixture and the mixture is extracted with EtOAc (3 × 50 mL). The combined organic layers were washed with brine (30 mL), dried over anhydrous Na₂SO₄, filtered, and concentrated under vacuum. The crude product was used without further purification in the next step. LCMS (ESI): calcd for C₁₁H₁₈NO₃ [M+H]⁺ m/z 212.1, found 212.2.

To a solution of 6-(hydroxymethyl)-3-isopropyl-4-methoxy-1-methylpyridin-2(1H)-one (crude, about 120.0 mg) in MeCN (15 mL) was added IBX (191.1 mg, 0.68 mmol) stirred for 6 h at 80°C. The mixture was cooled to room temperature. The solvent is removed. Water (10 mL) is added to the residue and the mixture is extracted with EtOAc (3 × 30 mL). The combined organic layers are washed with brine (50 mL), dried over anhydrous Na₂SO₄, filtered, and concentrated under vacuum. The residue was then purified by flash chromatography (eluting with PE/EtOAc from 100/0 to 50/50 in 30 mins) to give 5-isopropyl-4-methoxy-1-methyl-6-oxo-1,6-dihydropyridine-2-. LCMS (ESI): calcd for C₁₁H₁₆NO₃ [M+H]⁺ m/z 210.1, found 210.2.

To a solution of 5-isopropyl-4-methoxy-1-methyl-6-oxo-1,6-dihydropyridine-2-carbaldehyde (51.0 mg, 0.24 mmol) in DMF (10 mL) was added dimethyl benzylphosphonate (73.2 mg, 0.37 mmol), NaOMe (30 wt% in methanol, 88.7 mg, 0.49 mmol) and 18-crown-6 (12.9 mg, 0.05 mmol) stirred for 3 h at 50 °C. The mixture was cooled to room temperature. The solvent is removed. Water (5 mL) is added to the residue and the mixture is extracted with EtOAc (3 × 15 mL). The combined organic layers are washed with brine (50 mL), dried over anhydrous Na₂SO₄, filtered, and concentrated under vacuum. The residue was then purified by flash chromatography (eluting with PE/EtOAc from 100/0 to 40/60 in 30 mins) to give 5-isopropyl-4-methoxy-1-methyl-6-oxo-1,6-dihydropyridine-2-carbaldehyde. LCMS (ESI): calcd for C₁₈H₂₂NO₂ [M+H]⁺ m/z 284.2, found 284.2.

To a solution of 5-isopropyl-4-methoxy-1-methyl-6-oxo-1,6-dihydropyridine-2-carbaldehyde (42.0 mg, 0.1574. mmol) in DCM (5 mL) was added BBr₃ (74.2 mg, 0.30 mmol), then the mixture was stirred at 25 °C for 12 h. The solution was added NaHCO₃ (aq.) and extracted with DCM (10 mL × 3). The organic phases were combined and washed with brine, dried over Na₂SO₄, filtered out and concentrated in vacuo. The residue was purified by prep-HPLC (columns: Gemini 5 um C18 150 × 21.2 mm, mobile phase: ACN - H₂O (0.1% FA), gradient: 30 - 70, 9.0 min) to afford compound **128**.

### Example 28 - Preparation of 2-isopropyl-5-(phenylthio) benzene-1,3-diol (Compound 129)

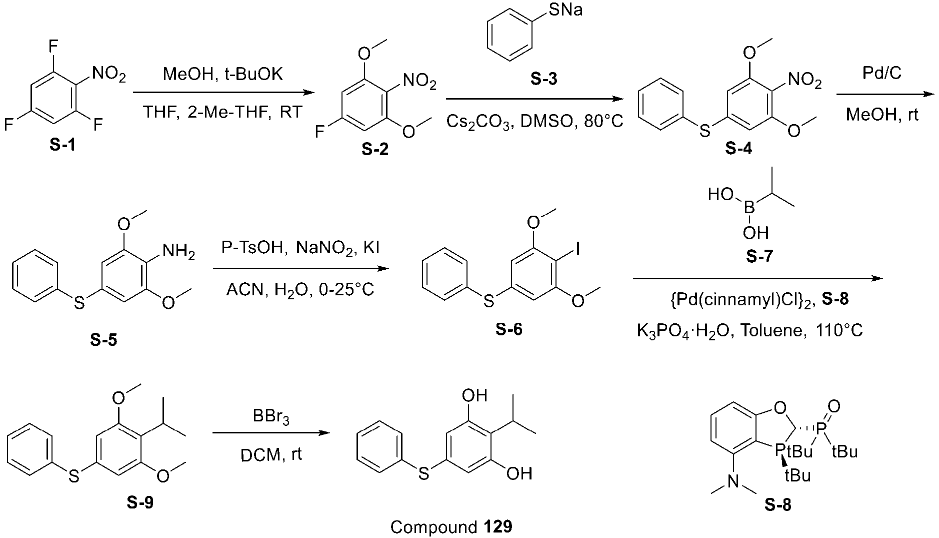

To a solution of MeOH (1.81 g, 0.0564 mol) in THF (50 mL) and 2-Methyltetrahydrofuran (50 mL) was added t-BuOK (6.3 g, 0.0564 mol) at 25°C. After for 30 min. Then 1,3,5-trifluoro-2-nitrobenzene (5.0 g, 0.0282 mol) was added at 0°C. The reaction mixture was stirred at 25°C for 30 min. The residue was quenched by ice water, extracted with EtOAc (100 mL × 3). The organic phases were combined, washed with brine, dried over Na₂SO₄, filtered out and concentrated in vacuo. The solvent was removed under reduced pressure to afford 5-fluoro-1,3-dimethoxy-2-nitrobenzene, which was used directly for the next step without purification. ¹H NMR (400 MHz, DMSO-*d₆*, ppm) 6.88 (d, *J =* 12.0 Hz, 2H), 3.87 (s, 6H).

To a solution of 5-fluoro-1,3-dimethoxy-2-nitrobenzene (7.3 g, 0.0361 mol) in DMSO (200 mL) was added sodium benzenethiolate (7.2 g, 0.0542 mol) and Cs₂CO₃ (23.5 g, 0.0722 mol), then the reaction mixture was stirred at 80°C for 12 h. and then cooled to ambient temperature. The mixture was extracted with EtOAc (100 mL × 3). The combined organic phase was washed with sat. NaCl (300 mL), dried over anhydrous Na₂SO₄, filtered and concentrated. The residue was purified by silica gel chromatography (eluting with PE/EtOAc from 100/0 to 80/20 in 30 mins) to afford (3,5-dimethoxy-4-nitrophenyl) (phenyl) sulfane. LCMS (ESI) calcd for C₁₄H₁₄NO₄S [M + H] ⁺ ms/z = 292.3, found 292.0.

To a solution of (3,5-dimethoxy-4-nitrophenyl) (phenyl) sulfane (4.0 g, 0.0138 mol) in MeOH (300 mL) was added Pd/C (400.0 mg, 10% Pd), then the mixture was stirred at 25°C for 12 h under 1 atm of H₂. The solution was filtered, the cake was washed with MeOH (10 mL × 3), and the filtrate was combined. The solution was removed under reduced pressure to afford 2,6-dimethoxy-4-(phenylthio) aniline. Without purification and directly for the next step. LCMS (ESI): calcd for C₁₄H₁₆NO₂S [M+H]⁺ m/z 262.3, found 262.1.

To a solution of 2,6-dimethoxy-4-(phenylthio) aniline (2.5 g, 0.0096 mol) in ACN (50 mL) was added P-TsOH (5.5 g, 0.0288 mol) at 0°C, after 30 min at 0°C. NaNO₂ (1.3 g, 0.0192 mol) and KI (4.0 g, 0.024 mol) in water (25 mL) was added dropwise at 0°C. The reaction mixture was stirred at 25°C for 12 h. The residue was quenched by ice water, extracted with EtOAc (100 mL × 3). The organic phases were combined, washed with brine, dried over Na₂SO₄, filtered out and concentrated in vacuo. The residue was purified by silica gel chromatography (eluting with PE/EtOAc from 100/0 to 88/12) to afford 2-iodo-1,3-dimethoxy-5-phenoxybenzene. ¹H NMR (400 MHz, DMSO-*d₆*, ppm) 7.41-7.35 (s, 5H), 6.56 (s, 2H), 3.74 (s, 6H).

To a solution of 2-iodo-1,3-dimethoxy-5-phenoxybenzene (2.3 g, 0.0062 mol) in toluene (100 mL) was added isopropyl boronic acid (0.8 g, 0.0093 mol), {Pd(cinnamyl)Cl}₂ (160.6 mg, 0.3089 mmol), L (49.1 mg, 0.1235 mmol) and K₃PO₄·H₂O (4.2 g, 0.0186 mol). The mixture was stirred at 100°C for 12 h under N₂. Cooled to ambient temperature. The mixture was extracted with EtOAc (100 mL × 3). The combined organic phase was washed with sat. NaCl, dried over anhydrous Na₂SO₄, filtered and concentrated. The residue was purified by silica gel chromatography (eluting with PE/EtOAc from 100/0 to 90/10) to afford (4-isopropyl-3,5-dimethoxyphenyl)(phenyl) sulfane. ¹H NMR (400 MHz, DMSO-*d₆*, ppm) 7.39-7.27 (m, 5H), 6.59 (s, 2H), 3.69 (s, 6H).

To a solution of (4-isopropyl-3,5-dimethoxyphenyl)(phenyl) sulfane (510.0 mg, 1.7683 mmol) in DCM (3 mL) was added BBr₃ (10 mL, 1M in DCM) at 0 °C. The reaction mixture was stirred at 25°C for 36 h. The reaction was quenched with MeOH and concentrated in vacuo. Then the mixture was adjusted to 7-8 with NaHCO₃(aq.), extracted with EtOAc, washed with brine, dried over NaSO₄ and concentrated. The residue was purified by Prep-HPLC (columns: Gemini 5 um C18 150 × 21.2 mm, mobile phase: ACN - H₂O (0.1% FA), gradient: 70 - 95, 6.0 min) to afford compound **129** (132.3 mg, yield: 28.69%).

### Example 29 - Preparation of 2-isopropyl-5-(phenylsulfinyl) benzene-1,3-diol (Compound 130)

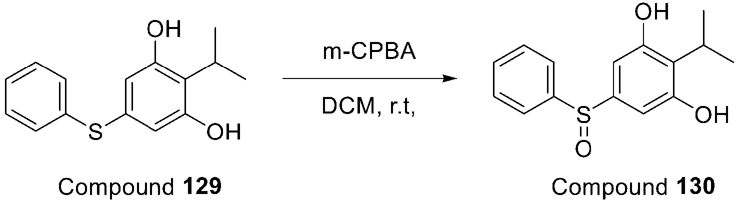

To a solution of compound **129** (100.0 mg, 0.3467 mmol) in DCM (3 mL) was added M-CPBA (59.8 mg, 0.3467 mmol). The reaction mixture was stirred at 25°C for 2 h. The reaction was quenched with NaHCO₃ (aq.), extracted with EtOAc (100 mL × 3). The organic phases were combined, washed with brine, dried over Na₂SO₄, filtered out and concentrated in vacuo. The residue was purified by Prep-HPLC (columns: Gemini 5 um C18 150 × 21.2 mm, mobile phase: ACN - H₂O (0.1% FA), gradient: 70 - 95, 6.0 min) to afford compound **130.**

### Example 30 - Preparation of 2-isopropyl-5-(phenylsulfonyl)benzene-1,3-diol (Compound 131)

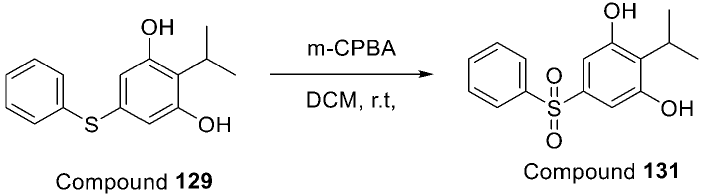

To a solution of compound **129** (100.0 mg, 0.3467 mmol) in DCM (3 mL) was added M-CPBA (239.2 mg, 1.3868 mmol). The reaction mixture was stirred at 25°C for 2 h. The reaction was quenched with NaHCO₃, extracted with EtOAc (100 mL × 3). The organic phases were combined, washed with brine, dried over Na₂SO₄, filtered out and concentrated in vacuo. The residue was purified by Prep-HPLC (columns: Gemini 5 um C18 150 × 21.2 mm, mobile phase: ACN - H₂O (0.1% FA), gradient: 70 - 95, 6.0 min) to afford compound **131.**

### Example 31 - Preparation of 2-isopropyl-5-phenoxybenzene-1,3-diol (Compound 132)

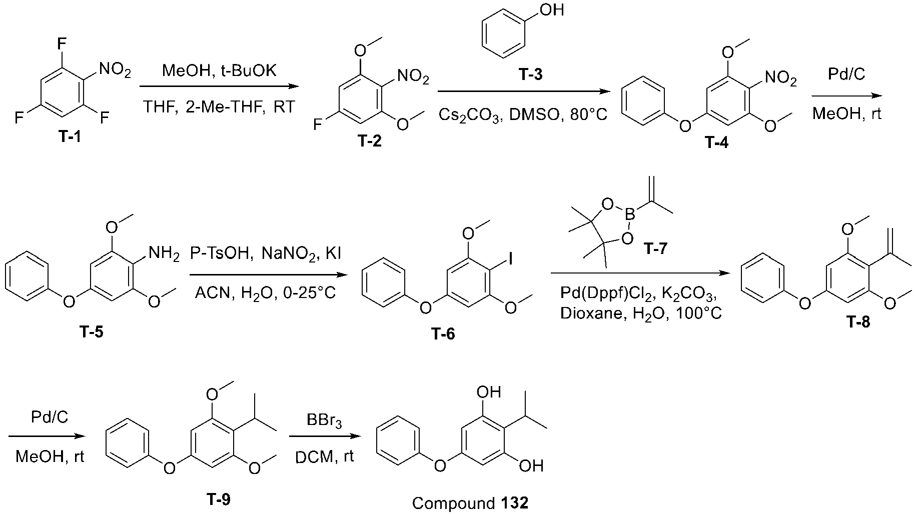

To a solution of MeOH (1.8 g, 0.0564 mol) in THF (50 mL) and 2-Methyltetrahydrofuran (50 mL) was added t-BuOK (6.3 g, 0.0564 mol) at 25°C. After 30 min. Then 1,3,5-trifluoro-2-nitrobenzene (5.0 g, 0.0282 mol) was added at 0°C. The reaction mixture was stirred at 25°C for 30 min. The residue was quenched by ice water, extracted with EtOAc (100 mL × 3). The organic phases were combined, washed with brine, dried over Na₂SO₄, filtered and concentrated in vacuo. The solvent was removed under reduced pressure to afford 5-fluoro-1,3-dimethoxy-2-nitrobenzene, which was used directly for the next step, without purification. ¹H NMR (400 MHz, DMSO-*d₆*. ppm): 6.88 (d, *J* = 12.0 Hz, 2H), 3.87 (s, 6H).

To a solution of 5-fluoro-1,3-dimethoxy-2-nitrobenzene (7.3 g, 0.0361 mol) in DMSO (200 mL) was added phenol (5.1 g, 0.0542 mol) and Cs₂CO₃ (23.5 g, 0.0722 mol), then the reaction mixture was stirred at 80°C for 12 h. and then cooled to ambient temperature. The mixture was extracted with EtOAc (100 mL × 3). The combined organic phase were washed with sat. NaCl (300 mL), dried over anhydrous Na₂SO₄, filtered and concentrated. The residue was purified by silica gel chromatography (eluting with PE/EtOAc from 50/1 to 4/1 in 30 mins) to afford 1,3-dimethoxy-2-nitro-5-phenoxybenzene. ¹H NMR (400 MHz, DMSO-*d₆*, ppm): 7.45 (t, *J =* 8.0 Hz, 2H), 7.23 (t, *J* = 7.6 Hz, 1H), 7.14 (d, *J* = 8.0 Hz, 2H), 6.48 (s, 2H), 3.78 (s, 6H).

To a solution of 1,3-dimethoxy-2-nitro-5-phenoxybenzene (4.1 g, 0.0161 mol) in MeOH (300 mL) was added Pd/C (400.0 mg, 10% Pd), then the mixture was stirred at 25°C for 12 h under 1 atm of H₂. The solution was filtered, the cake was washed with MeOH (10 mL × 3), and the filtrate was combined. The solution was removed under reduced pressure to afford 2,6-dimethoxy-4-phenoxyaniline. Without purification and directly for the next step. ¹H NMR (400 MHz, DMSO-*d₆*, ppm): 7.31 (t, *J* = 8.0 Hz, 2H), 7.01 (t, *J =* 7.2 Hz, 1H), 6.89 (d, *J* = 8.0 Hz, 2H), 6.35 (s, 2H), 4.04 (s, 2H), 3.72 (s, 6H).

To a solution of 2,6-dimethoxy-4-phenoxyaniline (2.5 g, 0.0103 mol) in ACN (50 mL) was added P-TsOH (5.9 g, 0.0308 mol) at 0°C. After 30 min at 0°C. NaNO₂ (1.4 g, 0.0205 mol) and potassium iodide (4.3 g, 0.0257 mol) in water (25 mL) was added dropwise at 0°C. The reaction mixture was stirred at 25°C for 12 h. The residue was quenched by ice water, extracted with EtOAc (100 mL × 3). The organic phases were combined, washed with brine, dried over Na₂SO₄, filtered and concentrated in vacuo. The residue was purified by silica gel chromatography (eluting with PE/EtOAc from 100/0 to 90/10 in 30 mins) to afford 2-iodo-1,3-dimethoxy-5-phenoxybenzene. LCMS (ESI): calcd for C1₄H₁₄IO₃ [M+H]⁺ m/z 357.0, found 356.9.

To a solution of 2-iodo-1,3-dimethoxy-5-phenoxybenzene (2.3 g, 0.0065 mol) in 1,4-dioxane (100 mL) and H₂O (20 mL) was added 4,4,5,5-tetramethyl-2-(prop-1-en-2-yl)-1,3,2-dioxaborolane (1.3 g, 0.0078 mol), Pd(dppf)Cl2 (480.0 mg, 0.0006 mmol) and K₂CO₃ (1.8 g, 0.013 mol). The mixture was stirred at 100°C for 12 h under N₂. Cooled to ambient temperature. The mixture was extracted with EtOAc (100 mL × 3). The combined organic phase was washed with sat. NaCl, dried over anhydrous Na₂SO₄, filtered and concentrated. The residue was purified by silica gel chromatography (eluting with PE/EtOAc from 100/0 to 90/10 in 30 mins) to afford 1,3-dimethoxy-5-phenoxy-2-(prop-1-en-2-yl)benzene. LCMS (ESI): calcd for C₁₇H₁₉O₃ [M+H]⁺ m/z 271.1, found 271.2.

To a solution of 1,3-dimethoxy-5-phenoxy-2-(prop-1-en-2-yl)benzene (610.0 mg, 2.1299 mmol) in MeOH (200 mL) was added Pd/C (300.0 mg), then the mixture was stirred at 25°C for 12 h under 1 atm of H₂. The solution was filtered, the cake was washed with MeOH (20 mL × 3), and the filtrate was combined. The solution was removed under reduced pressure to afford 2-isopropyl-1,3-dimethoxy-5-phenoxybenzene. Without purification and directly for the next step. ¹H NMR (400 MHz, DMSO-*d₆*, ppm): 7.42-7.34 (m, 2H), 7.11 (t, *J* = 7.6 Hz, 1H), 7.03-6.97 (m, 2H), 6.28 (s, 2H), 3.68 (s, 6H), 3.56-3.41 (m, 1H), 1.20 (d, *J* = 7.2 Hz, 6H).

To a solution of 2-isopropyl-1,3-dimethoxy-5-phenoxybenzene (100.0 mg, 0.3672 mmol) in DCM (3 mL) was added BBr₃ (3 mL, 1M in DCM) at 0 °C). The reaction mixture was stirred at 25°C for 36 h. After completion. The reaction was quenched with MeOH and concentrated in vacuo. Then the mixture was adjusted with NaHCO₃ (aq), extracted with EtOAc (100 mL × 3). The combined organic phase was washed with sat. NaCl (300 mL), dried over anhydrous Na₂SO₄, filtered and concentrated. The residue was purified by Prep-HPLC (columns: Gemini 5 um C18 150 × 21.2 mm, mobile phase: ACN - H₂O (0.1% FA), gradient: 60 - 95, 9.0 min) to afford compound **132.**

### Example 32 - Preparation of 2-isopropyl-3-methoxy-5-phenoxyphenol (Compound 133)

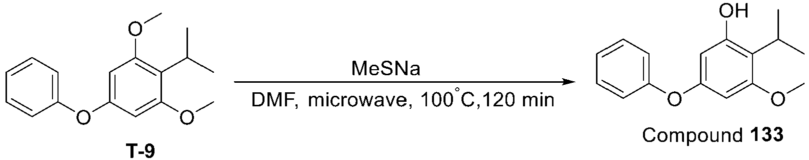

To a solution of 2-isopropyl-1,3-dimethoxy-5-phenoxybenzene (100.0 mg, 0.3672 mmol) in DMF (3 mL) was added MeSNa (264.5 mg, 3.6718 mmol). Then the reaction mixture was heated under microwave irradiation at 100°C for 120 min. The reaction was quenched with H₂O and concentrated in vacuo. The residue was purified by Prep-HPLC ((columns: Gemini 5 um C18 150 × 21.2 mm, mobile phase: ACN - H₂O (0.1% FA), gradient: 50 - 95, 7.0 min) to afford compound **133.**

| Compound No. | Structure | LCMS (ESI): [M + H] ⁺ | 1H NMR |
|---|---|---|---|
| 103 | 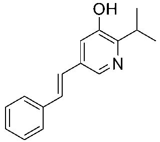 | 240.1 | ¹H NMR (400 MHz, CDCl₃, ppm): 8.26 (s, 1H), 7.49 (d, *J* = 7.6 Hz, 2H), 7.36 (t, *J =* 7.2 Hz, 2H), 7.28 (d, *J =* 5.6 Hz, 2H), 7.11-6.97 (m, 2H), 3.61-3.15 (m, 1H), 1.33 (d, *J* = 6.8 Hz, 6H). |
| 104 | 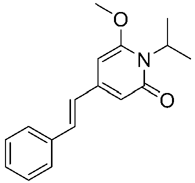 | 270.1 | ¹H NMR (400 MHz, MeOD-*d₄*, ppm): 7.61-7.59 (m, 2H), 7.40-7.30 (m, 4H), 7.01-6.96 (m, 1H), 6.22 (s, 1H), 6.12 (t, *J* = 2.0 Hz, 1H), 5.62-5.36 (m, 1H), 4.02 (d, *J* = 1.6 Hz 3H), 1.48 (d, *J* = 6.8 Hz, 6H). |
| 105 | 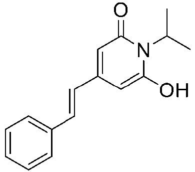 | 256.2 | 1H NMR (400 MHz, CDCl₃, ppm) 7.54-7.46 (m, 2H), 7.43-7.31 (m, 3H), 6.91-6.81 (m, 2H), 6.16 (t, *J* = 1.2 Hz, 1H), 5.06 (hept, *J* = 7.2 Hz, 1H), 3.61 (d, *J* = 1.2 Hz, 2H), 1.44 (d, *J* = 7.2 Hz, 6H). |
| 106 | 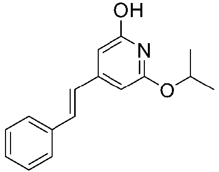 | 256.1 | ¹H NMR (400 MHz, DMSO-*d₆*, ppm) 7.61 (d, *J =* 7.2 Hz, 2H), 7.41-7.35 (m, 3H), 7.32-7.29 (m, 1H), 7.08 (d, *J =* 16.4 Hz, 1H), 6.34 (s, 1H), 6.29 (s, 1H), 5.12 (hept, *J =* 6.0 Hz, 1H), 1.27 (d, *J* = 6.0 Hz, 6H). HPLC purity: 254 nm (99.1%), and 214 nm (98.9%). |
| 107 | 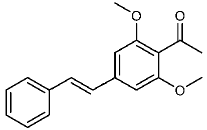 | 283.0 | ¹H NMR (400 MHz, DMSO-d6, ppm): 7.51 (d, *J =* 7.6 Hz, 2H), 7.38 (t, *J* = 7.6 Hz, 2H), 7.33-7.18 (m, 2H), 7.03-6.90 (m, 2H), 6.60 (d, *J* = 1.8 Hz, 1H), 3.87 (s, 3H), 3.83 (s, 3H), 2.45 (s, 3H). |
| 108 | 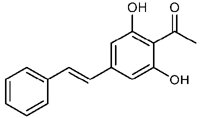 | 255.0 | ¹H NMR (400 MHz, DMSO-d*₆*, ppm) 7.50 (d, *J =* 7.2 Hz, 2H), 7.36 (t, *J* = 7.6 Hz, 2H), 7.26 (t, *J* = 7.2 Hz, 1H), 7.17 (d, *J =* 16.2 Hz, 1H), 6.93 (d, *J* = 16.2 Hz, 1H), 6.56 (d, *J* = 2.0 Hz, 1H), 6.28 (d, *J* = 2.0 Hz, 1H), 1.22 (s, 3H). |
| 109 | 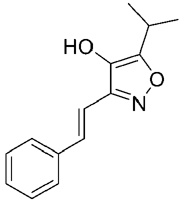 | 230.2 | ¹H NMR (400 MHz, DMSO-*d₆*, ppm) 8.83 (s, 1H), 7.63-7.60 (m, 2H), 7.56 (s, 1H), 7.42-7.39 (m, 2H), 7.36-7.32 (m, 1H), 7.12 (d, *J* = 16.4 Hz, 1H), 3.27-3.20 (m, 1H), 1.24 (d, *J* = 7.2 Hz, 6H). |
| 110 | 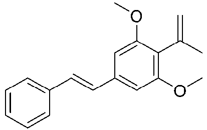 | 281.2 | ¹H NMR (400 MHz, DMSO-*d₆*, ppm) 7.61 (d, *J =* 7.6 Hz, 2H), 7.39 (t, *J* = 7.6 Hz, 2H), 7.34-7.19 (m, 3H), 6.90 (s, 2H), 5.18 (s, 1H), 4.73 (s, 1H), 3.78 (s, 6H), 1.89 (s, 3H). |
| 111 | 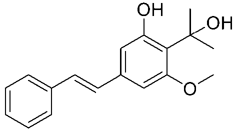 | 267.1 | ¹H NMR (400 MHz, DMSO-*d₆*, ppm) 11.40 (s, 1H), 7.58 (d, *J* = 8.0 Hz, 2H), 7.37 (t, *J* = 7.2 Hz, 2H), 7.28-7.07 (m, 3H), 6.89 (s, 1H), 6.75-6.64 (m, 1H), 6.55 (s, 1H), 3.80 (s, 3H), 1.61 (s, 6H) |
| 112 | 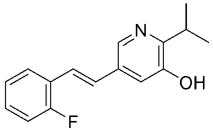 | 258.0 | ¹H NMR (400 MHz, DMSO-*d₆*, ppm) 9.83 (s, 1H), 8.18 (d, *J* = 1.8 Hz, 1H), 7.85-7.78 (m, 1H), 7.36-7.29 (m, 2H), 7.29-7.12 (m, 4H), 3.41-3.35 (m, 1H), 1.18 (d, *J =* 6.8 Hz, 6H). |
| 113 | 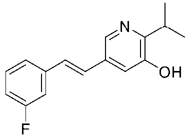 | 258.1 | ¹H NMR (400 MHz, DMSO-*d₆*, ppm) 9.83 (s, 1H), 8.18 (d, *J* = 1.8 Hz, 1H), 7.50 (d, *J* = 11.4 Hz, 1H), 7.46- 7.37 (m, 2H), 7.29 (m, 2H), 7.18-7.06 (m, 2H), 3.42-3.33 (m, 1H), 1.17 (d, *J* = 6.8 Hz, 6H). |
| 114 | 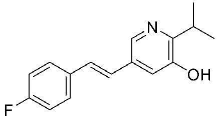 | 258.1 | ¹H NMR (400 MHz, DMSO-*d₆*, ppm) 9.78 (s, 1H), 8.16 (d, *J* = 1.8 Hz, 1H), 7.66 (m, 2H), 7.27 (d, *J* = 1.8 Hz, 1H), 7.21 (t, *J* = 8.8 Hz, 2H), 7.14 (s, 2H), 3.36 (m, 1H), 1.17 (d, *J =* 6.8 Hz, 6H). |
| 115 | 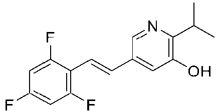 | 294.2 | ¹H NMR (400 MHz, DMSO-*d₆*, ppm) 9.91 (s, 1H), 8.15 (d, *J* = 1.6 Hz, 1H), 7.34-7.23 (m, 4H), 6.95 (d, *J* = 16.8 Hz, 1H), 3.40-3.32 (m, 1H), 1.17 (d, *J* = 6.8 Hz, 6H). |
| 116 | 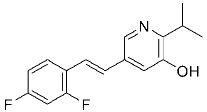 | 276.1 | ¹H NMR (400 MHz, DMSO-*d₆*, ppm) 9.81 (s, 1H), 8.17 (d, *J* = 2.0 Hz, 1H), 7.87 (dd, *J* = 15.6, 8.8 Hz, 1H), 7.32-7.23 (m, 3H), 7.17-7.11 (m, 2H), 3.39-3.34 (m, 1H), 1.17 (d, *J* = 6.8 Hz, 6H). |
| 117 | 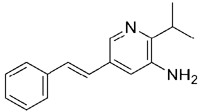 | 239.2 | ¹H NMR (400 MHz, DMSO-*d₆*, ppm) 8.16 (d, *J =* 2.0 Hz, 1H), 7.51-7.49 (m, 2H), 7.41-7.32 (m, 2H), 7.30-7.24 (m, 1H), 7.11 (d, *J* = 2.0 Hz, 1H), 7.09-6.97 (m, 2H), 3.69 (s, 2H), 3.05-2.98 (m, 1H), 1.33 (d, *J* = 6.8 Hz, 6H). |
| 118 | 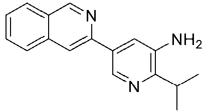 | 264.1 | ¹H NMR (400 MHz, DMSO-*d₆*, ppm) 9.39 (s, 1H), 8.54 (d, *J* = 2.0 Hz, 1H), 8.30 (s, 1H), 8.13 (d, *J* = 8.0 Hz, 1H), 8.02 (d, *J* = 7.6 Hz, 1H), 7.82-7.77 (m, 1H), 7.76 (d, *J* = 2.0 Hz, 1H), 7.69-7.65 (m, 1H), 5.22 (s, 2H), 3.26-3.16 (m, 1H), 1.21 (d, *J* = 6.8 Hz, 6H). |
| 119 | 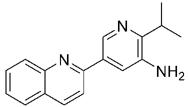 | 264.1 | ¹H NMR (400 MHz, DMSO-*d₆*, ppm) 8.56 (d, *J =* 2.0 Hz, 1H), 8.44 (d, *J* = 8.8 Hz, 1H), 8.05 (d, *J* = 8.4 Hz, 1H), 8.03-7.98 (m, 2H), 7.82 (d, *J* = 2.4 Hz, 1H), 7.80-7.76 (m, 1H), 7.63-7.57 (m, 1H), 5.31(s, 2H), 3.27-3.19 (m, 1H), 1.21 (d, *J* = 6.8 Hz, 6H). |
| 120 | 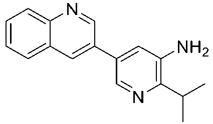 | 264.1 | ¹H NMR (400 MHz, DMSO-*d₆*, ppm) 9.16 (d, *J =* 2.4 Hz, 1H), 8.55 (d, *J* = 2.0 Hz, 1H), 8.23 (d, *J* = 2.0 Hz, 1H), 8.05 (dd, *J* = 8.4, 0.8 Hz, 2H), 7.80-7.76 (m, 1H), 7.67-7.63 (m, 1H), 7.35 (d, *J* = 2.0 Hz, 1H), 5.28 (s, 2H), 3.25-3.17 (m, 1H), 1.21 (d, *J* = 6.8 Hz, 6H). |
| 121 | 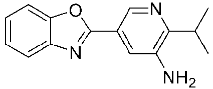 | 254.1 | ¹H NMR (400 MHz, CDCl₃, ppm) 8.86 (d, *J =* 1.8 Hz, 1H), 7.81-7.73 (m, 2H), 7.63-7.57 (m, 1H), 7.40-7.34 (m, 2H), 3.86 (s, 2H), 3.18-3.00 (m, 1H), 1.37 (d, *J =* 6.8 Hz, 3H). |
| 122 | 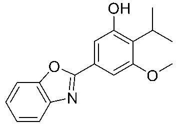 | 284.1 | ¹H NMR (400 MHz, DMSO-*d₆*, ppm) 9.83 (s, 1H), 7.81-7.74 (m, 2H), 7.44-7.37 (m, 2H), 7.34 (d, *J* = 1.6 Hz, 1H), 7.22 (d, *J* = 1.6 Hz, 1H), 3.87 (s, 3H), 3.56 (hept, *J* = 6.8 Hz, 1H), 1.27 (d, *J* = 7.2 Hz, 6H). |
| 123 | 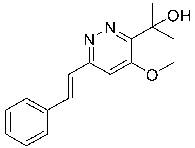 | 271.2 | ¹H NMR (400 MHz, DMSO-*d₆*, ppm) 7.82 (d, *J =* 16.4 Hz, 1H), 7.73-7.67 (m, 2H), 7.58 (s, 1H), 7.48-7.41 (m, 3H), 7.40-7.33 (m. 1H), 5.39 (s, 1H), 4.0 (s, 3H), 1.55 (s, 6H). |
| 124 | 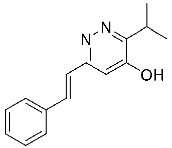 | 241.3 | ¹H NMR (400 MHz, DMSO-*d₆*, ppm) 13.04 (s, 1H), 7.61-7.55 (m, 3H), 7.46-7.37 (m, 3H), 6.96 (d, *J* = 16.8 Hz, 1H), 6.50 (s, 1H), 3.30 (d, *J* = 6.8 Hz, 2H), 1.15 (d, *J* = 6.8 Hz, 6H). |
| 125 | 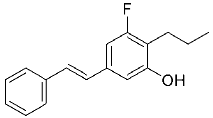 | 257.2 | ¹H NMR (400 MHz, DMSO-*d₆*, ppm) 9.79 (s, 1H), 7.59 (d, *J =* 7.6 Hz, 2H), 7.37 (t, *J* = 7.6 Hz, 2H), 7.27 (t, *J =* 7.2 Hz, 1H), 7.13-7.09 (m, 2H), 6.91-6.79 (m, 2H), 1.59-1.42 (m, 2H), 1.24 (t, *J =* 6.4 Hz, 2H), 0.89 (t, *J* = 8.0 Hz, 3H). |
| 127 | 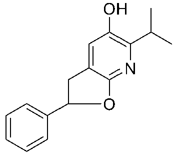 | 256.0 | ¹H NMR (400 MHz, CDCl₃, ppm) 7.42-7.29 (m, 5H), 7.15 (s, 1H), 5.79 (t, *J =* 8.8 Hz, 1H), 3.58 (dd, *J =* 16.4, 9.6 Hz, 1H), 3.33-3.23 (m, 1H), 3.16 (dd, *J* = 15.6, 7.6 Hz, 1H), 1.29 (d, *J* = 6.8 Hz, 6H). |
| 128 | 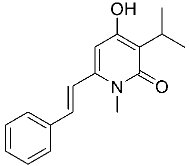 | 270.3 | ¹H NMR (400 MHz, DMSO-*d₆*, ppm) 10.00 (s, 0.6H), 9.89 (s, , 0.4 H), 7.67-7.65 (m, 1.2H), 7.60-7.58 (m, 0.7H), 7.48-7.39(m, 2H), 7.36-7.33(m, 1H), 7.27 (s, 0.3H), 7.23 (s, 0.4H), 7.03(s, 0.4H), 6.99(s, 0.3H), 6.16 (s, 0.6H), 5.78 (s, 0.3H), 3.58-3.49 (m, 0.6H), 3.42(s, 2H), 3.38(s, 1H), 3.29-3.21 (m, 0.4H), 1.19 (d, *J* = 7.2 Hz, 4H), 1.14(d, *J =* 7.2 Hz, 2H). |
| 129 | 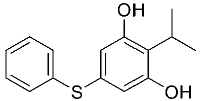 | 260.9 | ¹H NMR (400 MHz, DMSO-*d₆*, ppm) 9.27 (s, 2H), 7.37-7.33 (m, 2H), 7.29-7.24 (m, 3H), 6.28 (s, 2H), 3.43-3.39 (m, 1H), 1.21 (d, *J=* 7.2 Hz, 6H). |
| 130 | 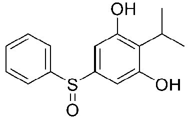 | 277.0 | ¹H NMR (400 MHz, DMSO-*d₆*, ppm) 9.62 (s, 2H), 7.63-7.49 (m, 2H), 7.57-7.51(m, 3H), 6.54 (s, 2H), 3.43-3.48 (m, 1H), 1.18 (d, *J* = 7.2 Hz, 6H). |
| 131 | 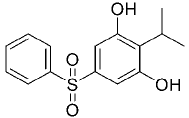 | 356.1 | ¹H NMR (400 MHz, DMSO-*d₆*, ppm) 9.89 (s, 2H), 7.84-7.82 (m, 2H), 7.71-7.61 (m, 3H), 6.79 (s, 2H), 3.46-3.49 (m, 1H), 1.18 (d, *J* = 7.2 Hz, 6H). |
| 132 | 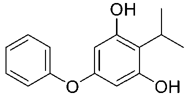 | 245.0 | ¹H NMR (400 MHz, DMSO-*d₆*, ppm): 9.13 (s, 2H), 7.41-7.29 (m, 2H), 7.14-7.07 (m, 1H), 7.02-6.95 (m, 2H), 5.91 (s, 2H), 3.42-3.35 (m, 1H), 1.21 (d, *J* = 7.2 Hz, 6H). |
| 133 | 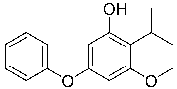 | 259.0 | ¹H NMR (400 MHz, DMSO-*d₆*, ppm): 9.29 (brs, 1H), 7.40-7.36 (m, 2H), 7.14-7.10 (m, 1H), 7.02-6.99 (m, 2H), 6.14 (d, *J* = 2.4 Hz, 1H), 6.00 (d, *J* = 2.4 Hz, 1H), 3.68 (s, 3H), 3.45-3.38 (m, 1H), 1.20 (d, *J* = 7.2 Hz, 6H). |

Compounds 139-166 were made in a manner similar to syntheses as described above.

| Compound | | LCMS (ESI): [M + H]⁺ | NMR |
|---|---|---|---|
| 139 | 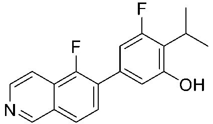 | 300.1 | 10.09 (s, 1H), 9.43 (s, 1H), 8.65 (d, *J* = 6.0 Hz, 1H), 8.07 (d, *J* = 8.4 Hz, 1H), 7.97 (d, *J* = 6.0 Hz, 1H), 7.80 (t, *J =* 8.0 Hz, 1H), 7.00 (s, 1H), 6.93 (d, *J =* 12.0 Hz, 1H), 3.55 - 3.38 (m, 1H), 1.30 (d, *J* = 7.2 Hz, 6H). |
| 140 | 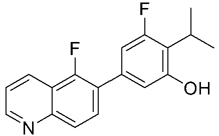 | 300.1 | ¹H NMR (400 MHz, DMSO-*d₆*, ppm) 10.04 (s, 1H), 9.00 (d, *J* = 2.8Hz, 1H), 8.56 (d, *J* = 8.4 Hz, 1H), 7.96 (d, *J* = 8.8 Hz, 1H), 7.88 (t, *J* = 8.4 Hz, 1H), 7.68 (dd, *J =* 8.4, 4.0 Hz, 1H), 6.99 (s, 1H), 6.91 (d, *J* = 12.0 Hz, 1H), 3.55 - 3.38 (m, 1H), 1.31 (d, *J* = 7.2 Hz, 6H). |
| 141 | 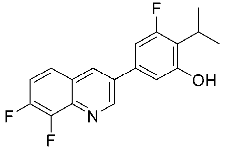 | 318.1 | ¹H NMR (400 MHz, DMSO-*d₆*, ppm) 10.12 (s, 1H), 9.23 (d, *J =* 2.0 Hz, 1H), 8.68 (s, 1H), 8.15 - 7.91 (m, 1H), 7.85 - 7.75 (m, 1H), 7.13 (d, *J* = 12.0 Hz, 1H), 7.08 (s, 1H), 3.55 - 3.38 (m, 1H), 1.30 (d, *J =* 7.2 Hz, 6H). |
| 142 | 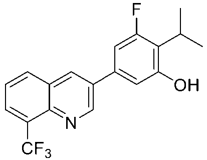 | 350.1 | ¹H NMR (400 MHz, DMSO-*d₆*, ppm) 10.12 (s, 1H), 9.30 (d, *J* = 2.0 Hz, 1H), 8.71 (d, *J* = 2.0 Hz, 1H), 8.37 (d, *J* = 8.0 Hz, 1H), 8.20 (d, *J* = 7.2 Hz, 1H), 7.79 (s, 1H), 7.16 (d, *J =* 12.0 Hz, 1H), 7.09 (s, 1H), 3.48 - 3.36 (m, 1H), 1.30 (d, *J* = 7.2 Hz, 6H). |
| 143 | 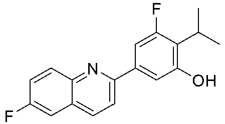 | 300.1 | ¹H NMR (400 MHz, DMSO-*d₆*, ppm) 10.07 (s, 1H), 8.44 (d, *J* = 8.8 Hz, 1H), 8.14 - 8.04 (m, 2H), 7.82 (dd, *J* = 9.2, 2.8 Hz, 1H), 7.75 - 7.64 (m, 1H), 7.61 (s, 1H), 7.43 (dd, *J* = 12.4, 1.2 Hz, 1H), 3.51 - 3.35 (m, 1H), 1.30 (d, *J* = 7.2 Hz, 6H). |
| 144 | 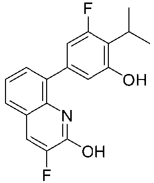 | 316.1 | ¹H NMR (400 MHz, DMSO-*d₆*, ppm) 10.85 (s, 1H), 9.95 (s, 1H), 7.96 (d, *J =* 10.8 Hz, 1H), 7.68 (d, *J* = 7.6 Hz, 1H), 7.37 (d, *J =* 7.2 Hz, 1H), 7.30 (t, *J* = 7.6 Hz, 1H), 6.66 (s, 1H), 6.62 (s, 1H), 3.51 - 3.37 (m, 1H), 1.31 (d, *J* = 7.2 Hz, 6H). |
| 145 | 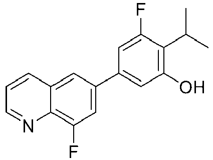 | 300.1 | ¹H NMR (400 MHz, DMSO-*d₆*, ppm) 10.02 (s, 1H), 8.99 - 8.89 (m, 1H), 8.50 (d, *J* = 8.4 Hz, 1H), 8.05 (s, 1H), 7.90 - 7.78 (m, 1H), 7.72 - 7.56 (m, 1H), 7.13 - 6.95 (m, 2H), 3.47 - 3.36 (m, 1H), 1.29 (d, *J* = 7.2 Hz, 6H). |
| 146 | 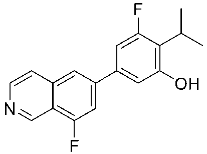 | 300.1 | ¹H NMR (400 MHz, DMSO-*d₆*, ppm) 10.06 (s, 1H), 9.46 (s, 1H), 8.64 (d, *J* = 6.0 Hz, 1H), 8.05 (s, 1H), 7.96 (d, *J* = 6.0 Hz, 1H), 7.75 (d, *J* = 12.0 Hz, 1H), 7.16 - 7.02 (m, 2H), 3.50 - 3.36 (m, 1H), 1.29 (d, *J* = 7.2 Hz, 6H). |
| 147 | 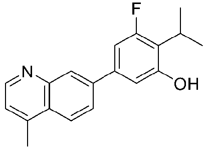 | 296.1 | ¹H NMR (400 MHz, DMSO-*d₆*, ppm) 10.00 (s, 1H), 8.78 (d, *J* = 4.4 Hz, 1H), 8.21 - 8.11 (m, 2H), 7.92 - 7.80 (m, 1H), 7.39 (d, *J* = 4.4 Hz, 1H), 7.07 (d, *J =* 11.6 Hz, 2H), 3.47 - 3.37 (m, 1H), 2.71 (s, 3H), 1.30 (d, *J* = 7.2 Hz, 6H). |
| 148 | 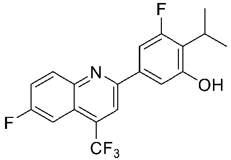 | 368.1 | ¹H NMR (400 MHz, DMSO-*d₆*, ppm) 10.13 (s, 1H), 8.40 (s, 1H), 8.34 - 8.25 (m, 1H), 7.97 - 7.85 (m, 1H), 7.77 (d, *J* = 9.2 Hz, 1H), 7.65 (s, 1H), 7.59 (d, *J = 12.0* Hz, 1H), 3.48 - 3.39 (m, 1H), 1.31 (d, *J =* 7.2 Hz, 6H). |
| 149 | 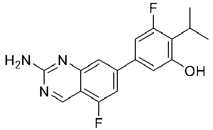 | 316.1 | ¹H NMR (400 MHz, MeOD, ppm) 9.25 (s, 1H), 7.45 (s, 1H), 7.21 - 7.12 (m, 1H), 6.96 - 6.74 (m, 2H), 3.54 - 3.41 (m, 1H), 1.33 (d, *J* = 7.2 Hz, 6H). |
| 150 | 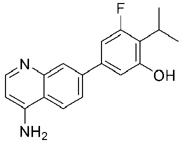 | 296.1 | ¹H NMR (400 MHz, DMSO-*d₆*, ppm) 8.32 (s, 1H), 8.23 (d, *J* = 8.4 Hz, 1H), 8.18 (s, 1H), 7.90 (s, 1H), 7.64 (d, *J* = 8.4 Hz, 1H), 7.04 (m, 4H), 6.55 (s, 1H), 3.44 - 3.38 (m, 1H), 1.29 (d, *J* = 6.8 Hz, 6H). |
| 151 | 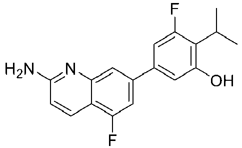 | 315.1 | ¹H NMR (400 MHz, DMSO-*d₆*, ppm) 9.95 (s, 1H), 8.01 (d, *J* = 9.2 Hz, 1H), 7.41 (s, 1H), 7.22 - 7.07 (m, 1H), 7.02 - 6.91 (m, 2H), 6.82 (d, *J* = 9.2 Hz, 1H), 6.73 (s, 2H), 3.47 - 3.36 (m, 1H), 1.28 (d, *J =* 6.8 Hz, 6H). |
| 152 | 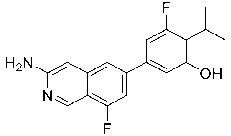 | 315.1 | ¹H NMR (400 MHz, DMSO-*d₆*, ppm) 10.08 (s, 1H), 8.93 (s, 1H), 7.53 (s, 1H), 7.09 (d, *J* = 12.0 Hz, 1H), 6.99 - 6.97 (m, 2H), 6.69 (s, 1H), 6.24 (s, 2H), 3.44 - 3.37 (m, 1H), 1.28 (d, *J* = 7.2 Hz, 6H). |
| 153 | 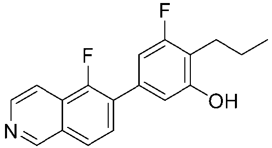 | 300.1 | ¹H NMR (400 MHz, DMSO-*d₆*, ppm) 10.07 (s, 1H), 9.43 (s, 1H), 8.65 (d, *J* = 6.0 Hz, 1H), 8.07 (d, *J =* 8.4 Hz, 1H), 7.97 (d, *J* = 5.6 Hz, 1H), 7.81 (t, *J* = 8.0 Hz, 1H), 7.07 - 6.88 (m, 2H), 2.59 (t, *J* = 7.2, 2H), 1.65 - 1.49 (m, 2H), 0.92 (t, *J* = 7.2, 3H). |
| 154 | 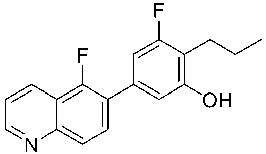 | 300.1 | ¹H NMR (400 MHz, DMSO-*d₆*, ppm) 10.04 (s, 1H), 9.00 (d, *J* = 4.0 Hz, 1H), 8.56 (d, *J* = 8.8 Hz, 1H), 7.96 (d, *J* = 8.8 Hz, 1H), 7.88 (t, *J* = 8.4 Hz, 1H), 7.68 (dd, *J* = 4.4 Hz, 1H), 7.00 (s, 1H), 6.95 (d, *J* = 10.4 Hz, 1H), 2.59 (t, *J* = 7.2 Hz, 2H), 1.65 - 1.48 (m, 2H), 0.93 (t, *J* = 7.6 Hz, 3H). |
| 155 | 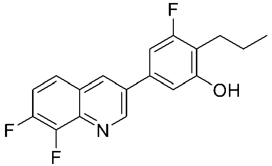 | 318.1 | ¹H NMR (400 MHz, DMSO-*d₆*, ppm) 10.12 (s, 1H), 9.25 (d, *J* = 2.0 Hz, 1H), 8.70 (s, 1H), 8.05 - 7.93 (m, 1H), 7.88 - 7.72 (m, 1H), 7.17 (d, *J* = 10.8 Hz, 1H), 7.10 (s, 1H), 2.59 (t, *J* = 7.2 Hz, 2H), 1.67 - 1.47 (m, 2H), 0.93 (t, *J* = 7.6 Hz, 3H). |
| 156 | 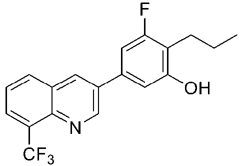 | 350.1 | ¹H NMR (400 MHz, DMSO-*d₆*, ppm) 10.11 (s, 1H), 9.32 (d, *J* = 2.0 Hz, 1H), 8.73 (d, *J* = 2.0 Hz, 1H), 8.38 (d, *J* = 8.4 Hz, 1H), 8.20 (d, *J* = 7.2 Hz, 1H), 7.79 (t, *J* = 7.6 Hz, 1H), 7.20 (d, *J* = 10.8 Hz, 1H), 7.12 (s, 1H), 2.60 (t, *J* = 8.0 Hz, 2H), 1.64 - 1.48 (m, 2H), 0.93 (t, *J* = 7.2 Hz, 3H). |
| 157 | 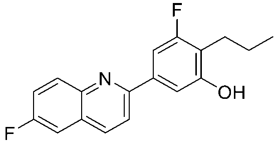 | 300.1 | ¹H NMR (400 MHz, DMSO-*d₆*, ppm) 10.06 (s, 1H), 8.44 (d, *J* = 8.8 Hz, 1H), 8.15 - 8.05 (m, 2H), 7.82 (dd, *J* = 9.6, 2.8 Hz, 1H), 7.75 - 7.66 (m, 1H), 7.63 (s, 1H), 7.47 (d, *J =* 10.8 Hz, 1H), 2.59 (t, *J =* 7.2 Hz, 2H), 1.71 - 1.44 (m, 2H), 0.92 (t, *J* = 7.2 Hz, 3H). |
| 158 | 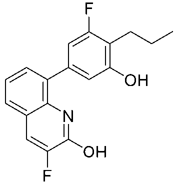 | 316.1 | ¹H NMR (400 MHz, DMSO-*d₆*, ppm) 10.83 (s, 1H), 9.96 (s, 1H), 7.96 (d, *J =* 10.8 Hz, 1H), 7.68 (d, *J* = 8.0 Hz, 1H), 7.37 (d, *J* = 7.2 Hz, 1H), 7.29 (t, *J =* 7.6 Hz, 1H), 6.79 - 6.62 (m, 2H), 2.58 (t, *J* = 7.6 Hz, 2H), 1.70 - 1.47 (m, 2H), 0.96 (t, *J* = 7.2 Hz, 3H). |
| 159 | 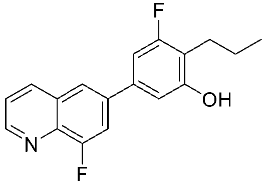 | 300.1 | ¹H NMR (400 MHz, DMSO-*d₆*, ppm) 10.02 (s, 1H), 8.95 (dd, *J* = 4.0, 1.6 Hz, 1H), 8.50 (d, *J* = 8.4 Hz, 1H), 8.07 (s, 1H), 7.87 (dd, *J* = 12.4, 1.6 Hz, 1H), 7.67 (dd, *J* = 8.4, 4.0 Hz, 1H), 7.16 - 7.04 (m, 2H), 2.58 (t, *J* = 7.6 Hz, 2H), 1.62 - 1.45 (m, 2H), 0.93 (t, *J* = 7.2 Hz, 3H). |
| 160 | 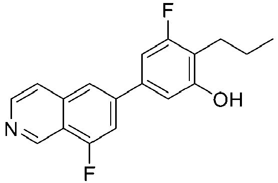 | 300.1 | ¹H NMR (400 MHz, DMSO-*d₆*, ppm) 10.07 (s, 1H), 9.46 (s, 1H), 8.64 (d, *J* = 6.0 Hz, 1H), 8.07 (s, 1H), 7.97 (d, *J* = 5.6 Hz, 1H), 7.77 (d, *J* = 12.0 Hz, 1H), 7.15 (d, *J* = 10.8 Hz, 1H), 7.09 (s, 1H), 2.58 (t, *J =* 7.6 Hz, 2H), 1.63 - 1.40 (m, 2H), 0.92 (t, *J* = 7.2 Hz, 3H). |
| 161 | 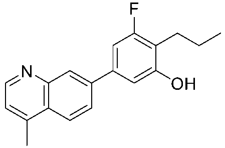 | 296.1 | ¹H NMR (400 MHz, DMSO-*d₆*, ppm) 10.00 (s, 1H), 8.78 (d, *J* = 4.4 Hz, 1H), 8.31 - 8.10 (m, 2H), 7.91 - 7.72 (m, 1H), 7.39 (d, *J* = 4.4 Hz, 1H), 7.21 - 7.00 (m, 2H), 2.71 (s, 3H), 2.58 (t, *J* = 7.6 Hz, 2H), 1.63 - 1.49 (m, 2H), 0.93 (t, *J* = 7.2 Hz, 3H). |
| 162 | 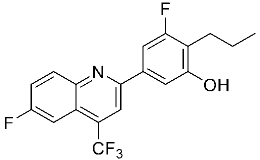 | 368.1 | ¹H NMR (400 MHz, DMSO-*d₆*, ppm) 10.13 (s, 1H), 8.42 (s, 1H), 8.36 - 8.22 (m, 1H), 7.91 (t, *J* = 7.2 Hz, 1H), 7.77 (d, *J* = 9.6 Hz, 1H), 7.68 (s, 1H), 7.63 (d, *J* = 11.2 Hz, 1H), 2.61 (t, *J =* 7.2 Hz, 2H), 1.72 - 1.49 (m, 2H), 0.93 (t, *J* = 7.2 Hz, 3H). |
| 163 | 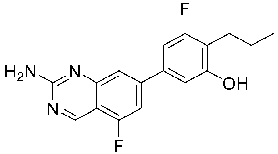 | 316.1 | ¹H NMR (400 MHz, DMSO-*d₆*, ppm) 10.03 (s, 1H), 9.24 (s, 1H), 7.38 (s, 1H), 7.24 (d, *J* = 11.2 Hz, 1H), 7.15 (s, 2H), 7.09 - 7.02 (m, 1H), 7.01 (s, 1H), 2.57 (t, *J* = 8.0 Hz, 3H), 1.62 - 1.46 (m, 2H), 0.92 (t, *J =* 7.2 Hz, 3H). |
| 164 | 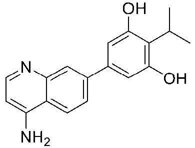 | 295.1 | ¹H NMR (400 MHz, DMSO-*d₆*, ppm) 9.24 (brs, 2H), 8.31 (d, *J* = 5.6 Hz, 1H), 8.23 - 8.10 (m, 2H), 7.79 (s, 1H), 7.51 (d, *J* = 8.8 Hz, 1H), 6.97 (s, 2H), 6.65 (s, 2H), 6.53 (d, *J* = 5.6 Hz, 1H), 3.50 - 3.46 (m, 1H), 1.27 (d, *J* = 6.8 Hz, 3H). |
| 165 | 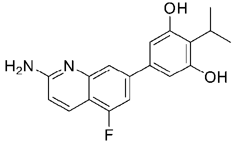 | 313.1 | ¹H NMR (400 MHz, DMSO-*d₆*, ppm) 9.22 (s, 2H), 8.00 (d, *J =* 9.2 Hz, 1H), 7.32 (s, 1H), 6.99 (d, *J =* 11.2 Hz, 1H), 6.79 (d, *J* = 9.0 Hz, 1H), 6.70 (s, 2H), 6.58 (s, 2H), 3.51 - 3.43 (m, 1H), 1.26 (d, *J =* 7.2 Hz, 6H). |
| 166 | 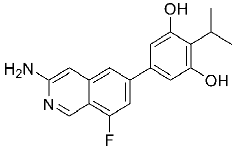 | 313.1 | ¹H NMR (400 MHz, DMSO-*d₆*, ppm) 9.24 (s, 2H), 8.91 (s, 1H), 7.38 (s, 1H), 6.95 (d, *J* = 12.2 Hz, 1H), 6.69 (s, 1H), 6.59 (s, 2H), 6.21 (d, *J* = 8.0 Hz, 2H), 3.50 - 3.43 (m, 1H), 1.26 (d, *J* = 7.2 Hz, 6H). |

### Example 33: AHR Agonist Reporter Assay in Human Cell Line

The AHR reporter assay was performed following the protocol of the Human Aryl Hydrocarbon Receptor (AHR) Reporter Assay System (INDIGO Bioscience, #IB06001-32). AHR Reporter Cell was thawed and pre-incubated in Cell Recovery Medium (CRM) for 6 hours. The media was then removed and the cells were incubated with Compound Screening Medium (CSM) in the absence (negative control) or presence of increasing concentrations of test compounds (typical dilution: 128pM, 640pM, 3.2nM, 16nM, 80nM, 400nM, 2µM, 10µM in duplicates) for 24 hours. For positive inhibition control, cells were activated with literature AHR agonists. Following 24 hours incubation, the media was removed and Luciferase Detection Reagent (LDR) added. The firefly luciferase activity was quantified using a plate reader. Results were normalized by positive and negative controls. The compounds of the disclosure were found to act at the AHR, as shown in Tables A', B', C', and D', below.

### Example 34 - AHR-regulated CYP1A1 Expression in Human Cell Line

To evaluate the AHR agonist activity of the test compounds, quantitative PCR analysis was used to determine expression level of the AHR-regulated gene CYP1A1 in a human monocytic U937 cell line. U937 cells were seeded at a concentration of 5X10⁵ cells/well in 200 µL of growth medium in 96-well cell culture microplates. CYP1A1 expression was induced with AHR agonists described in literature as a positive control or the test compounds for 16 hours. Human U937 cells were typically incubated with eight different concentrations of test compounds (typical dilution: 12.8 pM, 64 pM, 320 pM, 1.6 nM, 8 nM, 40 nM, 200 nM, 1 µM) and analyzed in duplicate on the same cell culture microplate. After stimulation, the media was removed, the cell RNA was isolated using RNeasy Mini Kit (Qiagen, 74104) and reverse-transcribed to cDNA using PrimeScript RT reagent Kit (Perfect Real Time) (TaKaRa, RR037A). Unstimulated cells were used as the negative control. Taqman probes for human CYP1A1 (Hs01054797_g1) and human GAPDH (Hs02786624_g1) were used to analyze fold expression of CYP1A1 of GAPDH. The compounds of the disclosure were found to initiate expression of CYP1A1, as shown in Tables A', B', C', and D', below.

### Example 35: Nrf2 Transcription Factor Activity Assay in Human Cell Line

The Nrf2 reporter assay was performed following the protocol of the Human Nuclear Factor (erythroid-derived2)-like 2 (Nrf2) Reporter Assay System (INDIGO Bioscience, #IB10001). Nrf2 Reporter Cell was thawed and pre-incubated in Cell Recovery Medium (CRM) for 6 hours. The media was then removed and the cells were incubated with Compound Screening Medium (CSM) in the absence (negative control) or presence of increasing concentrations of test compounds (typical dilution: 128pM, 640pM, 3.2nM, 16nM, 80nM, 400nM, 2µM, 10µM in duplicates) for 24 hours. For positive control, cells activated with CDDO or Nrf2 agonists described in literature. Following 24 hours incubation, the media was removed and Luciferase Detection Reagent (LDR) added. The firefly luciferase activity was quantified using a plate reader. Results were normalized by positive and negative controls. The compounds of the disclosure were found act at Nrf2, as shown in Tables A', B', C', and D', below.

### Example 36: AHR-regulated IL-6 inhibition in Human primary M1 macrophage

To analyze the inflammation cytokine IL-6 inhibition activity of the test compounds, quantitative PCR analysis was used to determine expression level of the IL-6 gene expression in human primary M1 macrophage cells. Human M1 macrophage was differentiated from PBMC. Briefly, at day 1, monocytes were isolated from human PBMC using EasySe Human Monocyte Enrichment Kit without CD16 Depletion (Stemcell, 19058). The isolated monocytes were seeded at 3 x 10⁷ cells in 15cm dish using 30ml medium containing 50ng/ml GM-CSF (R&D, 215-GM-050/CF). At day 5, 30 ml fresh medium containing 50ng/ml GM-CSF was added. At day 8, 1.5 x 10⁵ cells/well were re-seeded into 24 wells plate, and 500ul medium containing 10ng/ml LPS (Sigma, L7770), 20ng/ml IFN-gamma (PeproTech, 300-02), 50ng/ml GM-CSF and the test compounds was added. Cells were typically incubated with eight different concentrations of test compounds (typical dilution: 12.8 pM, 64 pM, 320 pM, 1.6 nM, 8 nM, 40 nM, 200 nM, 1 µM in duplicates). The IL-6 expression was inhibited with AHR agonists described in literature as the positive inhibition control, alternatively the un-stimulated cells were used as the negative inhibition control. After 24 hours stimulation at day 9, the media was removed, the cell RNA was isolated using RNeasy^{®} Plus 96 kit (Qiagen, 74192) and reverse-transcribed to cDNA using PrimeScript RT reagent Kit (Perfect Real Time) (TaKaRa, RR037A). The primers for human GAPDH (F: GCACCGTCAAGGCTGAGAAC; R: TGGTGAAGACGCCAGTGGA) and human IL-6 (F: GGTACATCCTCGACGGCATCT; R: GTGCCTCTTTGCTGCTTTCAC) were used to analyze fold expression of IL-6 of GAPDH by quantitative PCR. Data for IL-6 inhibition activity is shown in Table F', below.

The data provided herein demonstrate that the compounds of the disclosure are advantageously potent agonists of the AHR. See the data presented in Tables A', B', C', D', and E', below. Table E' includes data for two reference compounds.

**TABLE A'**

| **Cmpd No.** | **AHR reEC₅₀ (% activation to Tapinarof at 1µM)** | **Nrf2 reEC₅₀ (nM)** | **CYP1A1** |
|---|---|---|---|
| | | **% activation to Tapinarof at 9µM** | **(%activation to Tapinarof at 1µM)** |
| | **AHR abEC₅₀ (nM)** | | |
| 1 | ---- | | |
| | 25.2 nM | | |
| 2 | ---- | | |
| | >100 nM | | |
| 3 | ---- | | |
| | >100 nM | | |
| 4 | ---- | | |
| | >100 nM | | |
| 5 | ---- | | |
| | 78.7 nM | | |
| 6 | ---- | | |
| | 109 nM | | |
| 7 | ---- | | |
| | >100 nM | | |
| 8 | ---- | | |
| | >100 nM | | |
| 9 | ---- | | |
| | 20.9 nM | | |
| 10 | ---- | | |
| | 33.4 nM | | |
| 11 | ---- | | |
| | >100 nM | | |
| 12 | --- | | |
| | >100 nM | | |
| 13 | ---- | | |
| | >100 nM | | |
| 15 | ---- | | |
| | >100 nM | | |
| 16 | ---- | | |
| | >100 nM | | |
| 17 | ---- | | |
| | >100 nM | | |
| 18 | ---- | | |
| | >100 nM | | |
| 19 | ---- | | |
| | 90 nM | | |
| 20 | ---- | | |
| | >100 nM | | |
| 21 | ---- | | |
| | >100 nM | | |
| 22 | ---- | | |
| | 2.12 nM | | |
| 23 | ---- | | |
| | >100 nM | | |
| 24 | ---- | | |
| | >100 nM | | |
| 25 | ---- | | |
| | >100 nM | | |
| 26 | ---- | | |
| | >100 nM | | |
| 27 | ---- | | |
| | >100 nM | | |
| 28 | ---- | | |
| | >100 nM | | |
| 30 | ---- | | |
| | >100 nM | | |
| 31 | ---- | | |
| | 2.47 nM | | |
| 32 | ---- | | |
| | >100 nM | | |
| 33 | ---- | | |
| | 1.29 nM | | |
| 34 | ---- | | |
| | >100 nM | | |
| 35 | ---- | | |
| | >100 nM | | |
| 36 | ---- | | |
| | >100 nM | | |
| 38 | ---- | | |
| | >100 nM | | |
| 39 | ---- | | |
| | 3.58 nM | | |
| 40 | ---- | | |
| | >100 nM | | |
| 42 | ---- | | |
| | >100 nM | | |
| 43 | ---- | | |
| | >100 nM | | |
| 44 | ----- | | |
| | >100 nM | | |
| 45 | ---- | | |
| | 38.9 nM | | |
| 46 | 72.7 nM (95.8%) | 16.3 nM (69.3%) | 95.3% |
| | 39.0 nM | 166.2 nM | |
| 47 | ---- | ---- | |
| | 4.21 nm | 530 nM | |
| 48 | 64.3 nM (69.5%) | 14.5 nM (84.4%) | 78.4% |
| | 57.5 nM | 29.6 nM | |
| 49 | 38130 nM (104.6%) | 34.9 nM (69.7%) | 95.6% |
| | 53.4 nM | 73.7 nM | |
| 50 | ---- | ---- | |
| | 17.4 nM | 20 nM | |
| 51 | ---- | | |
| | 90 nM | | |
| 52 | ---- | | |
| | >100 nM | | |
| 53 | ---- | | |
| | >100 nM | | |
| 54 | ---- | | |
| | >100 nM | | |
| 55 | ---- | | |
| | >100 nM | | |
| 57 | ---- | | |
| | >100 nM | | |
| 58 | ---- | | |
| | >100 nM | | |
| 59 | ---- | | |
| | >100 nM | | |
| 60 | ---- | | |
| | >100 nM | | |
| 61 | ---- | | |
| | >100 nM | | |
| 63 | ---- | | |
| | >100 nM | | |
| 64 | ---- | | |
| | >100 nM | | |
| 68 | ---- | | |
| | >100 nM | | |
| 69 | ---- | | |
| | >100 nM | | |
| 71 | ---- | | |
| | >100 nM | | |
| 72 | ---- | | |
| | >100 nM | | |
| 73 | ---- | | |
| | >100 nM | | |
| 76 | ---- | | |
| | >100 nM | | |
| 77 | ---- | | |
| | >100 nM | | |
| 78 | ---- | | |
| | >100 nM | | |
| 79 | ---- | | |
| | >100 nM | | |
| 81 | ---- | | |
| | >100 nM | | |
| 83 | ---- | | |
| | >100 nM | | |
| 85 | ---- | | |
| | >100 nM | | |
| 89 | ---- | | |
| | >100 nM | | |
| 90 | ---- | | |
| | >100 nM | | |
| 91 | ---- | | |
| | >100 nM | | |
| 92 | ---- | | |
| | >100 nM | | |
| 94 | ---- | | |
| | >100 nM | | |
| 95 | ---- | | |
| | >100 nM | | |
| 96 | ---- | | |
| | >100 nM | | |
| 97 | 1622 nM (62.6%) | 248.6 nM (67.2%) | 78.9% |
| | 569.0 nM | 501.1 nM | |
| 98 | 176173 nM (71.2%) | 40 nM (83.6%) | 83.6% |
| | 402.8 nM | 138.6 nM | |
| 99 | ---- | ---- | |
| | >100 nM | 100 nM | |
| 100 | 21.9 nM (49.7%) | 200.1 (62.3%) | 90.5% |
| | > 1000 nM | 889.7 | |
| 101 | ---- | | |
| | 94.4 nM | | |
| 118 | ---- | | |
| | >100 nM | | |
| 119 | 66 nM (16.4%) | 124.7 nM (97.8%) | 0.4% |
| | ---- | 129.2 nM | |
| 120 | ---- | | |
| | > 100 nM | | |
| 121 | ---- | ---- | |
| | >100 nM | 120 nM | |
| 122 | 254.3 nM (64.4%) | 2641 nM (25.7%) | 39.3% |
| | 394.6 nM | --- | |
| 134 | ---- | | |
| | 101 nM | | |

**TABLE B'**

| Cmpd No. | **AHR reEC₅₀ (% activation to Tapinarof at 1µM)** | **Nrf2 reEC₅₀ (nM)** | **CYP1A1** |
|---|---|---|---|
| | **AHR abEC₅₀ (nM)** | **% activation to Tapinarof at 9µM** | **(%activation to Tapinarof at 1µM)** |
| 103 | 166.1 nM (69.5%) | 70.8 nM (78.8%) | 34.0% |
| | 241.5 nM | 148.7 nM | |
| 107 | ---- | ---- | |
| | >100 nM | >100 nM | |
| 108 | ---- | | |
| | 143 nM | | |
| 110 | ---- | ---- | |
| | >100 nM | >100 nM | |
| 111 | ---- | ---- | |
| | 39 nM | 57 nM | |
| 112 | ---- | ---- | |
| | 99.0 nM | 97 nM | |
| 113 | ---- | | |
| | 298 nM | | |
| 114 | 143 nM (88.5%) | ---- | --- |
| | 120.5 nM | 19 nM | |
| 117 | ---- | | |
| | >100 nM | | |
| 125 | ---- | | |
| | 11.5 nM | | |
| 135 | ---- | | |
| | 38.6 nM | | |
| 136 | ---- | | |
| | > 100 nM | | |
| 137 | ---- | | |
| | < 8 nM | | |

**TABLE C'**

| Cmpd No. | **AHR reEC₅₀ (% activation to Tapinarof at 1µM)** | **Nrf2 reEC₅₀ (nM)** | **CYP1A1** |
|---|---|---|---|
| | **AHR abEC₅₀ (nM)** | **% activation to Tapinarof at 9µM** | **(%activation to Tapinarof at 1µM)** |
| 129 | ---- | | |
| | > 400 nM | | |
| 130 | ---- | | |
| | > 400 nM | | |
| 131 | ---- | | |
| | > 400 nM | | |
| 132 | ---- | | |
| | > 400 nM | | |
| 133 | ---- | ---- | |
| | 233 nM | > 400 nM | |

**TABLE D'**

| Cmpd No. | **AHR reEC₅₀ (% activation to Tapinarof at 1µM)** | **Nrf2 reEC₅₀ (nM)** | **CYP1A1** |
|---|---|---|---|
| | **AHR abEC₅₀ (nM)** | **% activation to Tapinarof at 9µM** | **(%activation to Tapinarof at 1µM)** |
| 105 | ---- | | |
| | > 400 nM | | |
| 106 | ---- | ---- | |
| | 343 nM | > 400 nM | |
| 109 | ---- | | |
| | >100 nM | | |
| 127 | ---- | | |
| | >100 nM | | |

**Table E'**

| **Compound** | **Structure** | **AHR reEC₅₀ (% activation to Tapinarof at 1µM)** | **Nrf2 reEC₅₀ (nM)** | **CYP1A1 (%activation to Tapinarof at 1µM)** |
|---|---|---|---|---|
| | | **AHR abEC₅₀ (nM)** | | |
| **Tapinarof** | 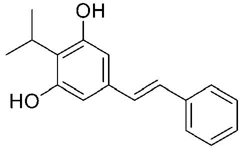 | 60-80 nM (100%) | 55-75 nM | 100% |
| | | 20-30 nM | | |
| **Reference** | 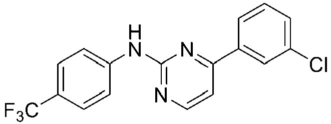 | ---- | | |
| | | 17.1 nM | | |

**Table F'**

| Cmpd No. | M1 assay | M1 assay |
|---|---|---|
| | IL6 -IC50 (nM) | IL6 -max % inhibition (% to no treatment) |
| 22 | 0.05 nM | 77.20% |
| 31 | 1.18 nM | 72.90% |
| 33 | 3.11 nM | 88.30% |
| 39 | 0.2nM | 89.60% |
| 48 | 3.0 nM | 89.60% |
| 49 | 3.14 nM | 90.30% |
| 103 | 92.6 nM | 84.90% |
| 112 | 1.57 nM | 77.90% |
| 113 | 120 nM | 67.80% |
| 125 | 1.35 nM | 91.70% |
| 135 | 1.7 nM | 95.30% |
| 139 | 5.0 nM | 85.80% |
| 140 | 0.13 nM | 92.30% |
| 143 | 2.7 nM | 91.80% |
| 145 | 1.9 nM | 94.10% |
| 147 | 0.03 nM | 91.60% |
| 149 | 0.5 nM | 91.10% |
| 155 | 3.5 nM | 89.10% |
| 161 | 1.19 nM | 91.30% |

It should be appreciated that all combinations of the foregoing concepts and implementations and additional concepts and implementations discussed in greater detail below are contemplated as being part of the inventive subject matter disclosed herein, and may be employed in any suitable combination to achieve the benefits as described herein. In particular, all combinations of claimed subject matter appearing at the end of this disclosure are contemplated as being part of the inventive subject matter disclosed herein

The foregoing description is given for clearness of understanding only, and no unnecessary limitations should be understood therefrom, as modifications within the scope of the invention may be apparent to those having ordinary skill in the art.

The terms "substantially" and "about" used throughout this specification are used to describe and account for small fluctuations. For example, they can refer to less than or equal to ±5%, such as less than or equal to ±2%, such as less than or equal to ±1%, such as less than or equal to ±0.5%, such as less than or equal to ±0.2%, such as less than or equal to ±0.1%, such as less than or equal to ±0.05%.

Throughout this specification and the claims which follow, unless the context requires otherwise, the word "comprise" and variations such as "comprises" and "comprising" will be understood to imply the inclusion of a stated integer or step or group of integers or steps but not the exclusion of any other integer or step or group of integers or steps.

Throughout the specification, where compositions are described as including components or materials, it is contemplated that the compositions can also consist essentially of, or consist of, any combination of the recited components or materials, unless described otherwise. Likewise, where methods are described as including particular steps, it is contemplated that the methods can also consist essentially of, or consist of, any combination of the recited steps, unless described otherwise. The invention illustratively disclosed herein suitably may be practiced in the absence of any element or step which is not specifically disclosed herein.

The practice of a method disclosed herein, and individual steps thereof, can be performed manually and/or with the aid of or automation provided by electronic equipment. Although processes have been described with reference to particular cases, a person of ordinary skill in the art will readily appreciate that other ways of performing the acts associated with the methods may be used. For example, the order of various of the steps may be changed without departing from the scope or spirit of the method, unless described otherwise. In addition, some of the individual steps can be combined, omitted, or further subdivided into additional steps.

All patents, publications and references cited herein are hereby fully incorporated by reference. In case of conflict between the present disclosure and incorporated patents, publications and references, the present disclosure should control.

## Claims

1. A compound having a structure of Formula (I): wherein: 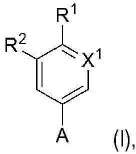 or a pharmaceutically acceptable salt thereof,
R¹ is C₁₋₆alkyl, C₁₋₆haloalkyl, C₁₋₆hydroxyalkyl, OC₁₋₆alkyl, C(=O)C₁₋₆alkyl, or C(=CH)C₁₋₆alkyl;
R² is OH, OC₁₋₆alkyl, N(R^{N1})₂, or halo;
each R^{N1} independently is H or C₁₋₆alkyl;
X¹ is N, C-OH, or C-OC₁₋₆alkyl;
A is an optionally substituted fused bicyclic heteroaryl group having 8-10 total ring atoms and 1, 2, or 3 heteroatoms selected from N, O, or S; or 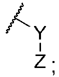
Y is 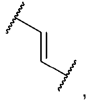 O, S, S=O, or SO₂; and
Z is an optionally substituted phenyl group or an optionally substituted heteroaryl group having 5 or 6 total ring atoms and 1, 2, or 3 heteroatoms selected from N, O, and S;
with the proviso that when R² is OH or OC₁₋₆alkyl and X¹ is C-OH or C-OC₁₋₆alkyl; then Y is not 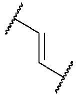 for use in modulating the activity of the aryl hydrocarbon receptor (AHR).

2. The compound or salt for use of claim 1, wherein R¹ is

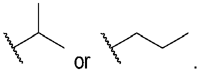

3. The compound or salt for use of claim 1, wherein R¹ is 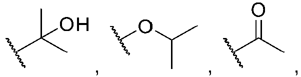 or

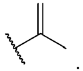

4. The compound or salt for use of any one of claims 1-3, wherein R² is OH, OCH₃, N(R^{N1})₂, or F; and each R^{N1} independently is H or CH₃.

5. The compound or salt for use of claim 6, wherein R² is OH.

6. The compound or salt for use of any one of claims 1-5, wherein X¹ is N.

7. The compound or salt of for use any one of claims 1-3, wherein R² is OH, OCH₃, NH₂, or F and X¹ is N, C-OH, or C-OCH₃.

8. The compound or salt for use of claim 1, wherein R¹ is 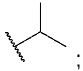 R² is OH, OCH₃, or NH₂; and X¹ is N, C-OH, or C-OCH₃.

9. The compound or salt for use of any one of claims 1-8, wherein A is an optionally substituted fused bicyclic heteroaryl group having 8-10 total ring atoms and 1, 2, or 3 heteroatoms selected from N, O, or S.

10. The compound or salt for use of claim 9, wherein A is 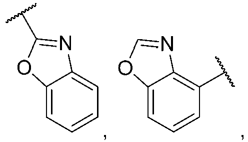 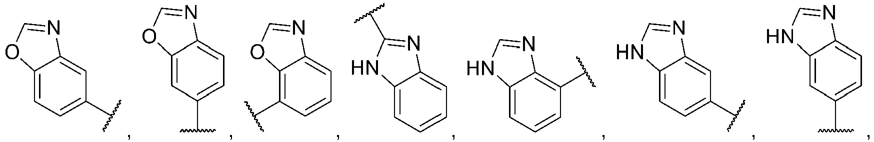 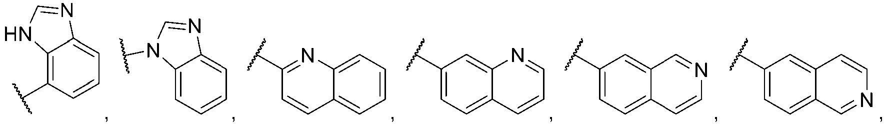 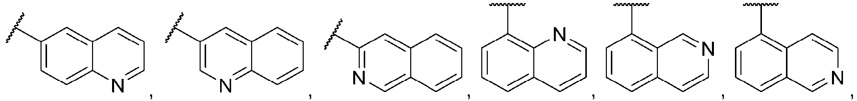 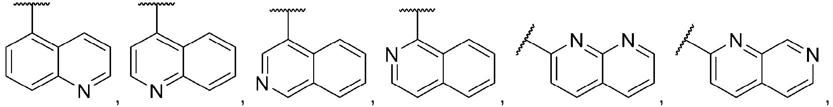 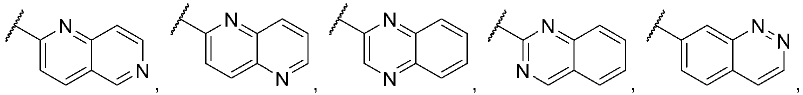 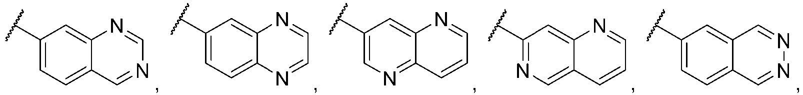 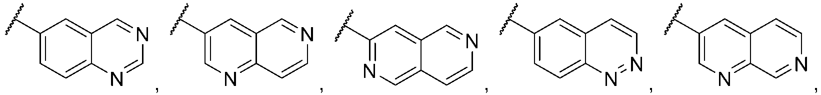 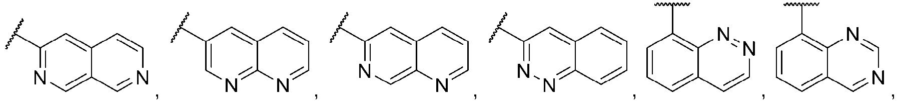 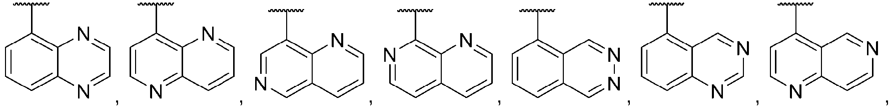 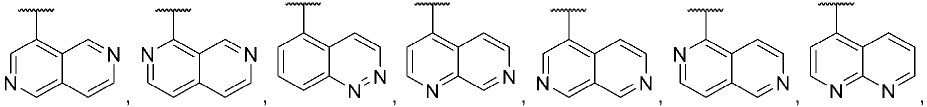 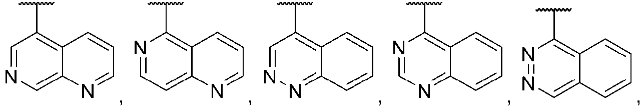 wherein any of the foregoing is unsubstituted or substituted.

11. The compound or salt for use of claim 10, wherein A is

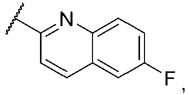

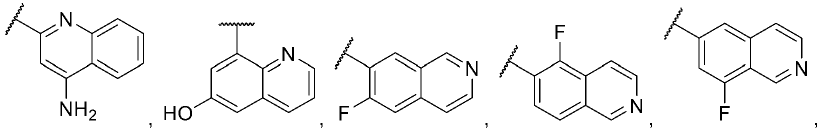

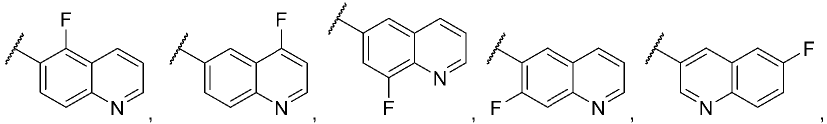

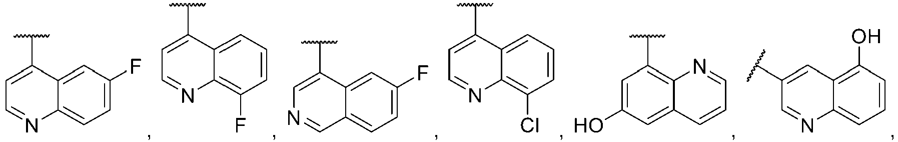

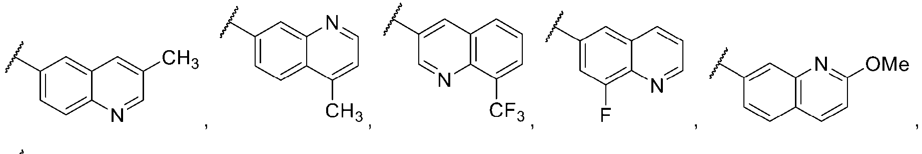

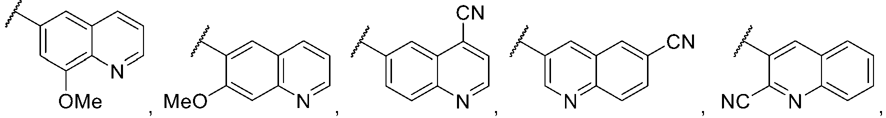

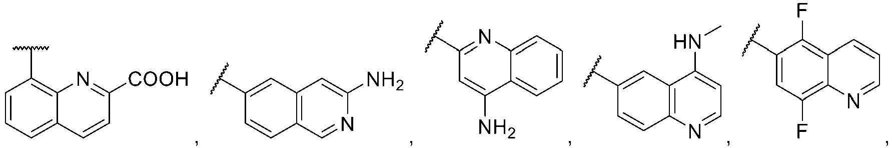

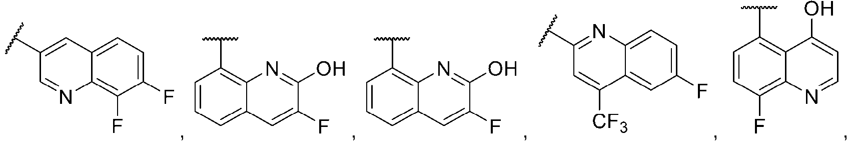

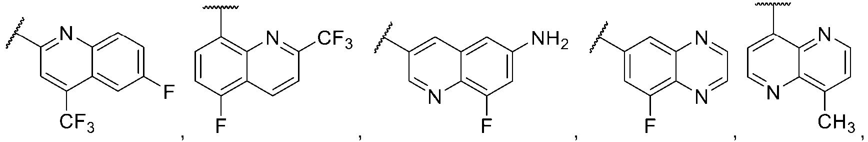

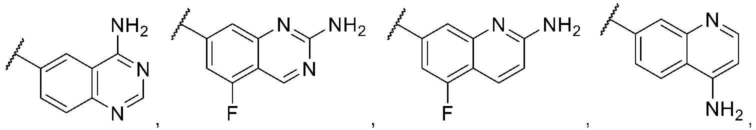

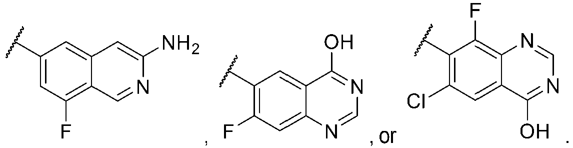

12. The compound or salt of any one of claims 1-8, wherein A is

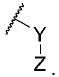

13. The compound or salt of for use claim 12, wherein Y is

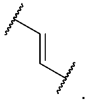

14. The compound or salt for use of claim 13, wherein A is

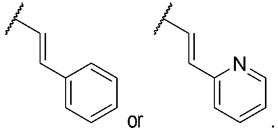

15. The compound or salt for use of any one of claims 1 to 14, wherein the disease or disorder is a disease or disorder of the gastrointestinal tract, skin, lung, central nervous system, pancreas, eye, bones bone joints, a neuroinflammatory disease, or a neurodegenerative disease.
